(19) **European Patent Office**

Europäisches Patentamt
European Patent Office
Office européen des brevets

(11) **EP 4 339 291 A1**

(12) **EUROPEAN PATENT APPLICATION**
published in accordance with Art. 153(4) EPC

(43) Date of publication:
**20.03.2024 Bulletin 2024/12**

(21) Application number: **21734877.0**

(22) Date of filing: **13.05.2021**

(51) International Patent Classification (IPC):
**C12N 15/867** (2006.01)    **C12N 5/10** (2006.01)
**A61K 48/00** (2006.01)    **A61P 7/00** (2006.01)

(52) Cooperative Patent Classification (CPC):
**C12N 15/86; A61K 48/00; A61P 7/00;**
C12N 2740/16043; C12N 2800/22; C12N 2830/48;
C12N 2830/50

(86) International application number:
**PCT/ES2021/070343**

(87) International publication number:
**WO 2022/238595 (17.11.2022 Gazette 2022/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
- **Centro de Investigaciones Energéticas,
  Medioambientales y Tecnológicas
  O.A., M.P. (CIEMAT)
  28040 Madrid (ES)**
- **Consorcio Centro de Investigación Biomédica en
  Red
  28029 Madrid (ES)**
- **Fundación Instituto de Investigación Sanitaria
  Fundación Jiménez Díaz (FIIS-FJD)
  28040 Madrid (ES)**
- **Université Libre de Bruxelles
  1050 Brussels (BE)**

- **Fundación Para La Investigación Biomédica Del
  Hospital Infantil Universitario Niño Jesús
  28009 Madrid (ES)**

(72) Inventors:
- **BUEREN RONCERO, Juan Antonio
  28040 Madrid (ES)**
- **NAVARRO ORDÓÑEZ, Susana
  28040 Madrid (ES)**
- **GIMÉNEZ MARTÍNEZ, Yari
  28040 Madrid (ES)**
- **PALACIOS PÉREZ, Manuel
  28040 Madrid (ES)**
- **LAFONTAINE, Denis
  1050 Bruxelles (BE)**

(74) Representative: **Moreno Nogales, Angeles
Avenida de la Industria, 32
28108 Alcobendas, Madrid (ES)**

Remarks:
The complete document including Reference
Table(s) and the Sequence Listing(s) can be
downloaded from the EPO website

(54) **LENTIVIRAL VECTORS AND USES THEREOF**

(57)    The present invention provides a lentiviral vector, wherein the lentiviral vector comprises a polynucleotide, wherein said polynucleotide is characterized in that it comprises a transcription unit that in turn comprises a human phosphoglycerate kinase (PGK) promoter, a nucleotide sequence encoding for the RPS19 protein and an optimized woodchuck hepatitis virus posttranscriptional regulatory element (Wpre). Compositions and uses of said lentiviral vector are also provided herein, particularly the use for the treatment of Diamond-Blackfan anemia.

EP 4 339 291 A1

**(Cont. next page)**

Figure 1

**Description**

**TECHNICAL FIELD OF THE INVENTION**

The present invention provides compositions and method for rescuing RPS19 expression in cells. In particular, methods and compositions for gene therapy of Diamond Blackfan anemia are provided herein.

**BACKGROUND OF THE INVENTION**

Diamond Blackfan anemia (DBA) is a rare disease with an estimated prevalence of 5-7 cases per million life births showing incidence and distribution of patients across different countries. The hallmark of DBA is macrocytic anemia, which is typically presented at the first year, and frequently evolve to neutropenia and thrombocytopenia, and in some cases to a myelodisplatic syndrome or acute myeloid leukemia (Ruggero & Shimamura, 2014). Thus, although DBA is typically associated with red cell pure aplasia, the generalized hematopoietic defect defines DBA as a bone marrow failure (BMF) syndrome.

Mutations in 20 DBA genes, plus 3 "DBA-like" genes, account for 70-80% of DBA patients. RPS19 - the gene encoding for the ribosomal protein S19 - is the most commonly affected in DBA (25% of patients) (Da Costa, Narla, & Mohandas, 2018). These patients are typically haplo-insufficient for this gene (one gene unaffected, the other inactivated) leading to a substantial reduced production of functional ribosomes.

Corticoesteroids constitute the first therapeutic option for DBA patients. Approximately 80% of patients respond initially to corticosteroids with an improvement in, or complete remission of, their anemia. However, prolonged corticosteroid treatment has showed limited efficacy in many patients, so that only about 40% will remain on corticosteroids for an extended period of time, implying that overall, at least 40% of DBA patients are transfusion dependent. To date, allogenic hematopoietic stem cell transplantation (allo-HSCT) is the only available curative treatment for DBA patients. The consensus is to use sibling donors or fully-matched (10/10) unrelated donors, however, only a minority of patients has access to a suitable donor. Furthermore, the clinical outcome of DBA patients transplanted with alternative donors after the age of 10 is very poor. Additionally, DBA is now identified as a cancer-prone disease, and the cancer incidence in these patients could be even increased after myeloablative conditioning and transplantation, as already demonstrated in patients with Fanconi anaemia (FA).

Gene therapy aimed at correcting gene mutations in hematopoietic stem cells represents a potential therapeutic strategy for this genetic disorder. Gene therapy via gammaretroviruses, lentiviruses, adenoviruses, and adeno-associated viruses is attractive because of the natural ability of viruses to enter into and deliver genetic material to cells. In particular, lentiviral vectors (LVs) are suitable vehicles for gene delivery since they considered relatively safe and are capable of stably integrating into the genomic DNA of a broad range of dividing and nondividing mammalian cell types. The ability to stably integrate in the genome make LVs a unique and ideal tool for gene therapy, especially for correcting and treating genetic disorders such as DBA.

Accordingly, there remains a critical need for an effective treatment regimen for DBA, and the present invention provides LVs that are not only capable of restoring the expression of RPS19 protein, but also capable of correcting the alteration in the ribosomal biogenesis process.

**DESCRIPTION OF THE FIGURES**

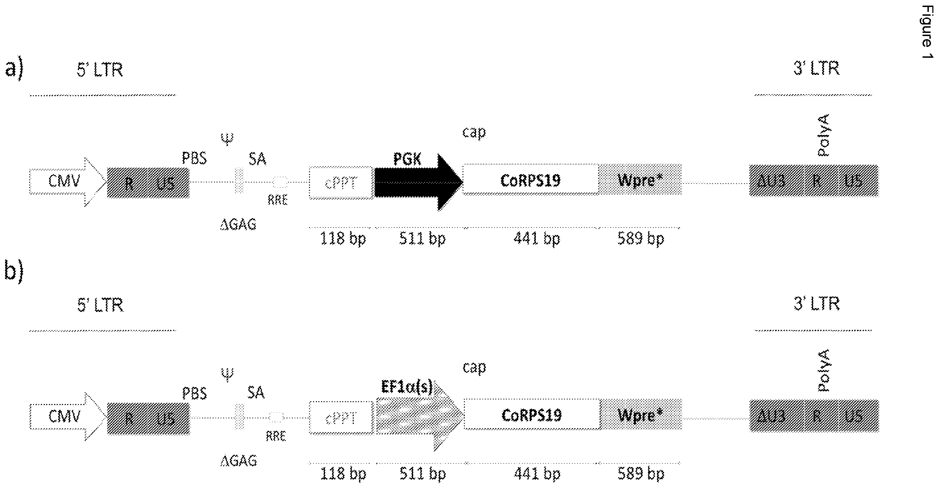

**Figure 1. Schematic diagram of the therapeutic vectors generated: a) *PGK.CoRPS19.Wpre*\* and b) *EF1α(s).CoRPS19.Wpre*\*.** 5' LTR: 5' long terminal repeat; PBS: primer binding site; Ψ: psi packaging signal; ΔGAG: truncated Gag sequence; SA: splicing acceptor; RRE: Rev responsive element (RRE); cPPT: DNA flap central polypurine tract; PGK: phosphoglycerate kinase promoter; EF1α(s): elongation factor 1α in its short version; CoRPS19: codon-optimized nucleotide sequence encoding for the RPS19 protein; Wpre*: mutated optimized woodchuck hepatitis virus posttranscriptional regulatory element; 3' LTR :3' long terminal repeat; PolyA: polyadenylation signal sequence.

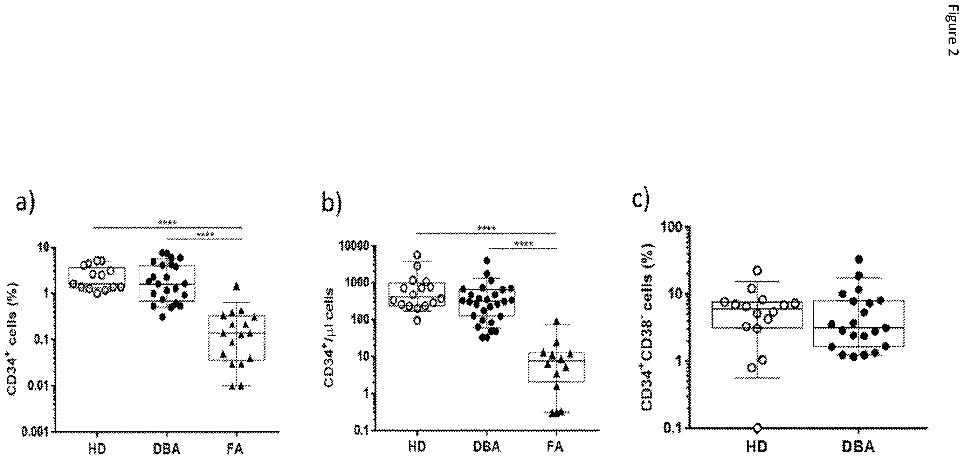

**Figure 2. Determination of the number of CD34+cells in Diamond Blackfan anemia (DBA) patients' bone marrow: a)** Percentage of CD34+ cells in total bone marrow cells extracted from DBA patients (DBA), Fanconi anemia patients (FA) and healthly donors (HD), as control. **b)** Cellularity of the bone marrow. c) Percentage of CD34+/CD38- cells in bone marrow. The degree of significance was determined using the Mann Whitney test (P-value; * ≤ 0.05; ** ≤ 0.01; *** ≤ 0.001; **** ≤ 0.0001). The graphs show the median and interquartile range along with the 90:10 percentiles.

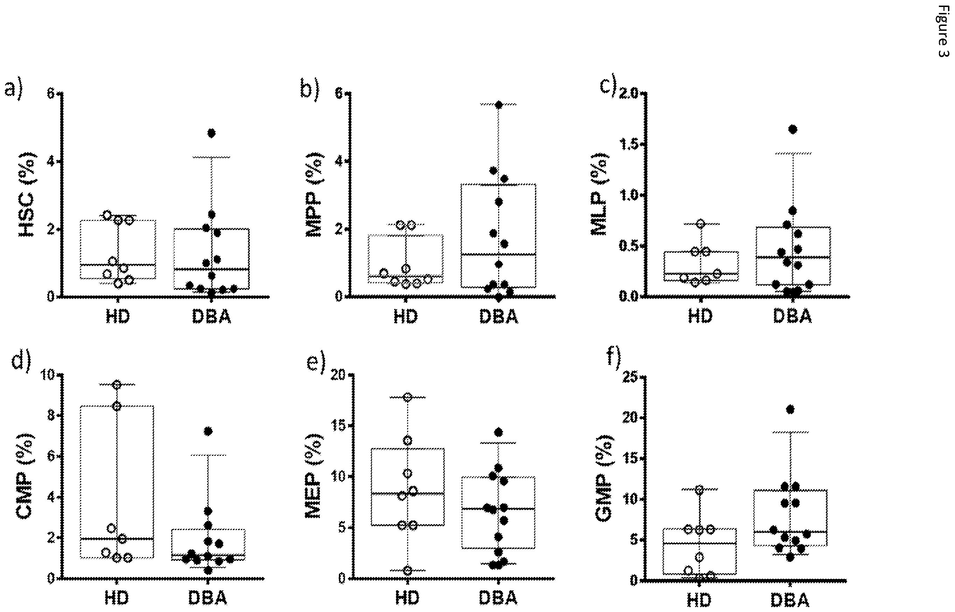

**Figure 3. Quantification of the percentage of different subpopulations of hematopoietic progenitors in bone marrow of DBA patients and healthy donors, a)** HSC (hematopoietic stem cell: Lin⁻CD34⁺CD38⁻CD90⁺CD45RA⁻). **b)** MPP (multipotent progenitors: CD34⁺CD38⁻Thy-1⁻CD45RA⁻Flt3⁺CD7⁻CD10⁻). **c)** MLP (multi-lymphoid progenitors: CD34⁺CD38⁻Thy-1^low^CD45RA⁻Flt3⁺CD7⁻/⁺CD10⁻). **d)** CMP (common myeloid progenitors: CD34⁺CD38⁺Thy-1⁻CD45RA⁻,Flt3⁺CD7⁻CD10⁻). **e)** MEP (megakaryocytic and erythroid progenitors: CD34⁺CD38⁺Thy⁻1⁻CD45RA⁻Flt3⁻CD7⁻CD10⁻) and f) GMP (granulocytic-monocytic progenitors: CD34⁺CD38⁺Thy-1⁻CD45RA⁺Flt3⁺CD7⁻CD10⁻). The degree of significance was determined using the Mann Whitney test. The graphs show the median and interquartile range along with the 90:10 percentiles. HD: healthy donors; DBA: DBA patients.

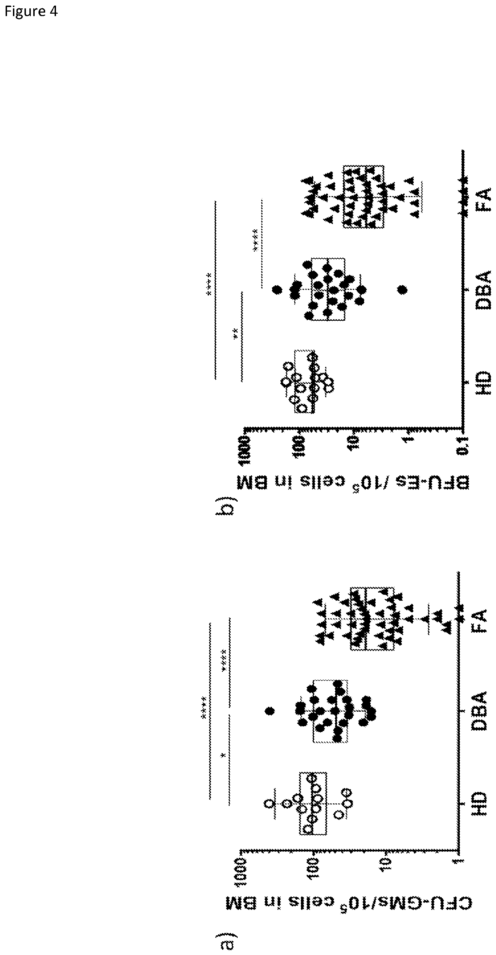

**Figure 4. Determination of the number of hematopoietic progenitor colony forming cells (CFCs) in bone marrow: a)** Number of granulocyte-macrophage progenitor colony forming units (CFU-GMs) for each $10^5$ of seeded mononuclear cells (MNC). **b)** Number of burst-forming unit-erythroid progenitors (BFU-Es) for each $10^5$ seeded MNC. The degree of significance was determined using the Mann Whitney test (P-value; * ≤ 0.05; ** ≤ 0.01; *** ≤ 0.001; **** ≤ 0.0001). The graphs show the median and interquartile range along with the 90:10 percentiles. BM: bone marrow; HD: healthy donors; DBA: DBA patients; FA: Fanconi anemia patients.

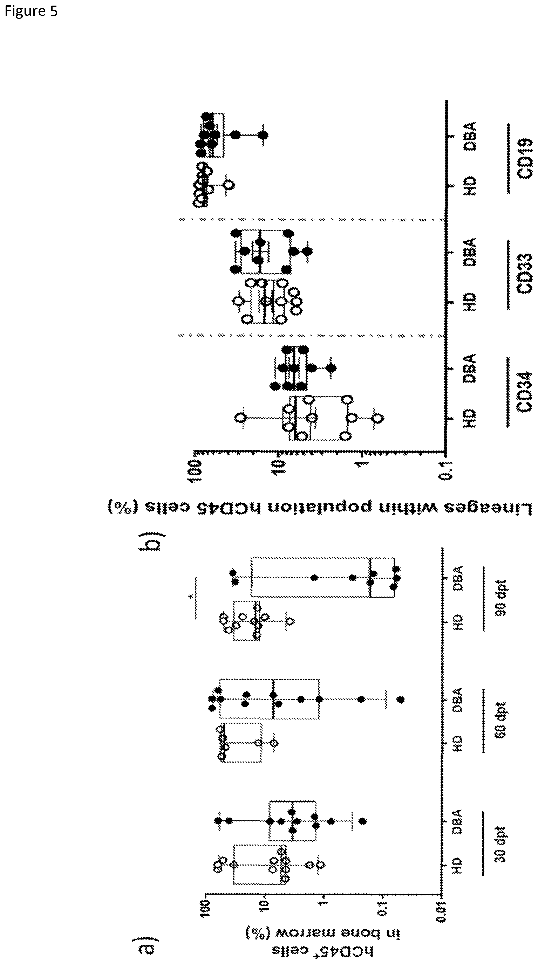

**Figure 5. Determination of the potential for reconstitution of CD34⁺ cells from healthy donors and DBA patients in immunodeficient NSG mice:** a) Percentage of human CD45⁺ (hCD45⁺) cells grafted into mouse bone marrow at three different time points (30, 60 and 90 days post-transplant (dpt)). **b)** Differentiation to the different lineages. CD33⁺: myeloid, CD19⁺: lymphoid and CD34⁺: hematopoietic stem cells (HSC). The degree of significance was determined by the test Mann Whitney (P-value; * ≤ 0.05; ** ≤ 0.01; *** ≤ 0.001; **** ≤ 0.0001). The graphs show the median and interquartile range along with the 90:10 percentiles. HD: healthy donors DBA: DBA patients.

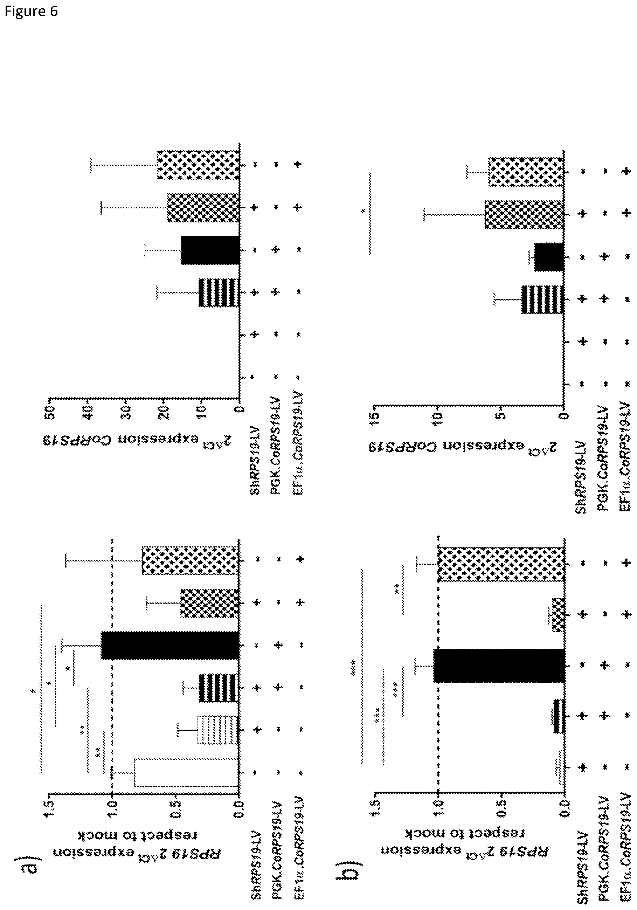

**Figure 6. Study of endogenous expression of *the RPS19* gene in a disease model cell line (*K562 interfered with in the RPS19* gene by vectors *LV-THM.shRPS19* and *MISSION® pLKO.1-pure TurboGFP™ shRPS19)* subsequently corrected by the *PGK.CoRPS19.Wpre\** or *EF1α(s).CoRPS19.Wpre\** therapeutic vectors: a)** Left panel: Reduction of endogenous *expression of RPS19* by means of the vector *LV-THM.shRPS19* (ShRPS19-LV); right panel: subsequent detection *of the CoRPS19* sequence present in therapeutic vectors *PGK.CoRPS19.Wpre\**(PGK.CoRPS19-LV) and *EF1α(s).CoRPS19.Wpre\** (EF1α.CoRPS19-LV). **b)** Left panel: reduction of the endogenous *expression of RPS19* by means of the vector LV-*MISSION® pLKO.1-pure TurboGFP™ shRPS19 (*ShRPS19-LV*);* right panel: the subsequent detection of the CoRPS19 sequence present in therapeutic vectors *PGK.CoRPS19.Wpre\** (PGK.CoRPS19-LV) *and EF1α(s).CoRPS19.Wpre\** (PGK.CoRPS19-LV). The degree of significance was determined by the t test (P-value; * ≤ 0.05; ** ≤ 0.01; *** ≤ 0.001; **** ≤ 0.0001). The graphs show the mean and standard deviation. The dashed line in the left panels indicates *RPS19* basal endogenous expression in not-interfered K562 control cells (mock).

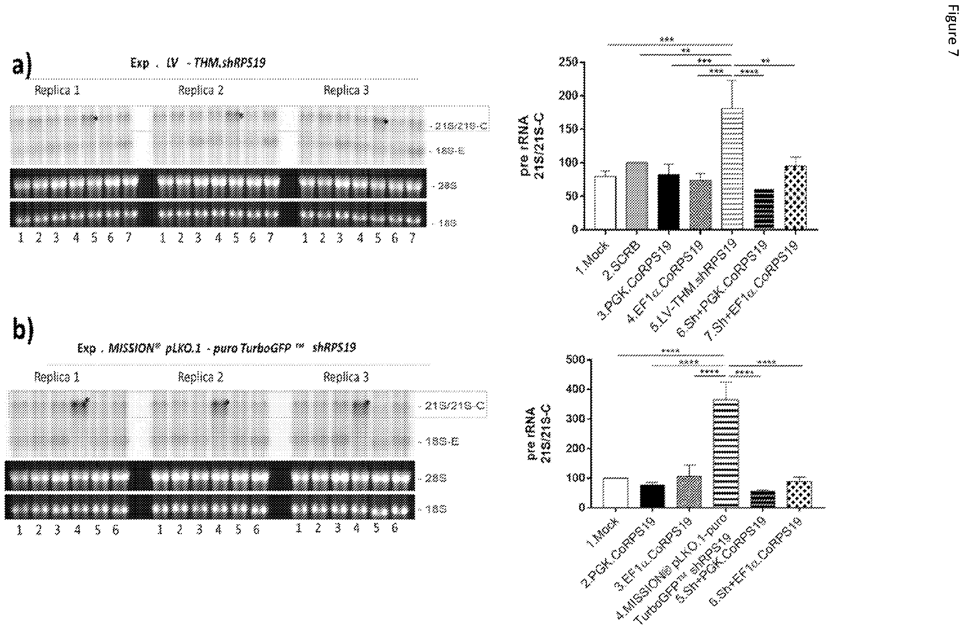

**Figure 7. Analysis of ribosomal biogenesis in the K562 cell line interfered for the *RPS19* gene and subsequently corrected by the *PGK.CoRPS19.Wpre\** or *EF1α(s).CoRPS19.Wpre\** therapeutic vectors: a)** Left panel: detection (of the level of pre-21S / 21C rRNA (asterisk, 21S/21S-C) by northern blot in cells interfered with the interference vector *LV-THM.shRPS19* and subsequently transduced with the therapeutic vectors *PGK.CoRPS19.Wpre\** (PGK.CoRPS19-LV) or *EF1a(s).CoRPS19.Wpre\** (EF1α.CoRPS19-LV); right panel: graph showing the quantification of the ratio between pre-21S and 21C rRNA (pre rRNA 21S/21S-C) detected by northern blot in cells interfered with the interference vector *LV-THM.shRPS19* and subsequently transduced with the therapeutic vectors *PGK.CoRPS19.Wpre\** or *EF1α(s).CoRPS19.Wpre\**. **b)** Left panel: detection of the 21S and 21C pre-rRNA level (asterisk) by northern blot in cells interfered with the interference vector *LV-MISSION® pLKO.1-pure TurboGFP™ shRPS19* and subsequently transduced with the therapeutic vectors *PGK.CoRPS19.Wpre\** and *EF1α(s).CoRPS19.Wpre \*;* right panel: graph showing the quantification of the ratio between pre-21S and 21C rRNA (pre rRNA 21S/21S-C) detected by northern blot in cells interfered with the interference vector *LV-MISSION® pLKO.1-pure TurboGFP™ shRPS19* and subsequently transduced with the therapeutic vectors *PGK.CoRPS19.Wpre\** or *EF1α(s).CoRPS19.Wpre\**. The degree of significance was determined by the ANOVA test and its group by group analysis (P-value; * ≤ 0.05; ** ≤ 0.01; *** ≤ 0.001; **** ≤ 0.0001). The graphs show the mean and standard deviation of three experiments. 1.Mock: non transduced cells; 2.SCRB: cells transduced with a *LV-THM.shscramble.* 1.Mock and 2.SCRB are included as negative controls. PGK.CoRPS19: condition transduced with *PGK.CoRPS19.Wpre\** vecto*r*, EF1α.CoRPS19: condition transduced with *EF1α(s).CoRPS19.Wpre\**vector; Sh+PGK.CoRPS19: condition co-transduced with the interference vectors *in a) LV-THM.shRPS19* and in b) *MISSION® pLKO.1-pure TurboGFP™ shRPS19 and the PGK.CoRPS19.Wpre\** vector and Sh+EF1α.CoRPS19: condition co-transduced with the interference vectors *in a) LV-THM.shRPS19* and in b) *MISSION® pLKO.1-pure TurboGFP™ shRPS19 and the EF1α(s).CoRPS19.Wpre\** LV.

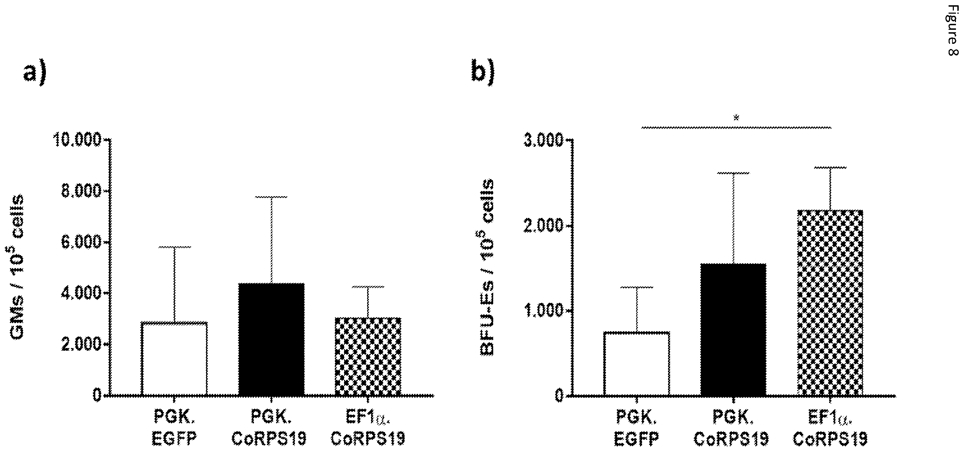

**Figure 8. Determination of the number of hematopoietic progenitor colonies generated by CD34$^+$ cells of DBA patients transduced with the *PGK.CoRPS19.Wpre\** or *EF1α(s).CoRPS19.Wpre\** therapeutic vectors or with the *PGK.EGFP.Wpre\** control vector: a)** Number of CFU-GMs colonies for every 10$^5$ mononuclear (MNC) cells seeded. b) Number of BFU-Es colonies for every 10$^5$ MNC cells seeded. The number of experiments performed with cells transduced with the *PGK.EGFP.Wpre\** vector (PGK EGFP) was N=3, with *PGK.CoRPS19.Wpre\* vector* (PGK.CoRPS19) was N=5 and with *EF1α(s).CoRPS19.Wpre\*vector* (EF1α.CoRPS19) was N=5.

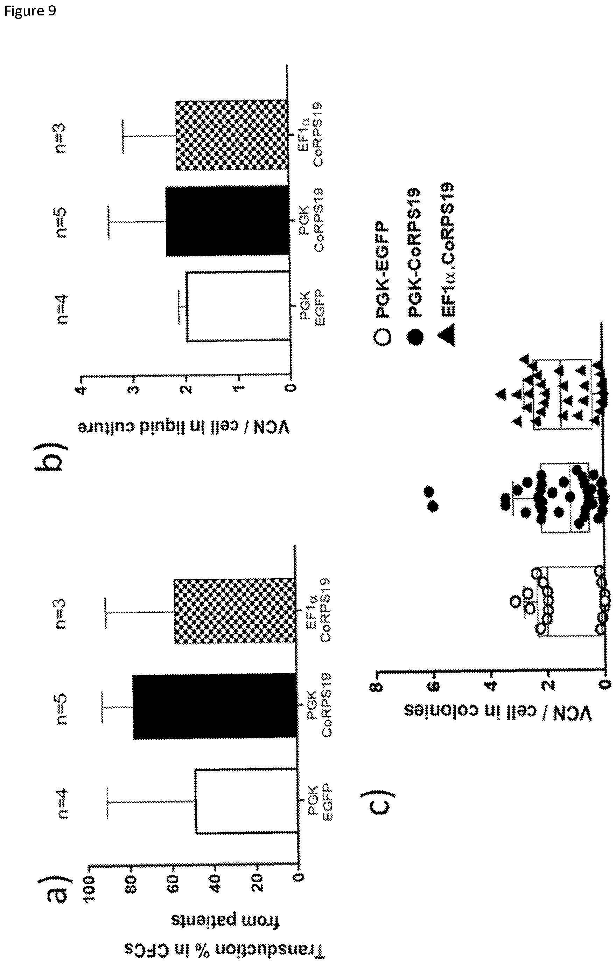

**Figure 9. Efficient correction and null toxicity with the *PGK.CoRPS19.Wpre\** or *EF1α(s).CoRPS19.Wpre\** therapeutic vectors in CD34$^+$ cells of DBA patients: a)** Percentage of transduction in hematopoietic progenitors of DBA patients with *PGK.CoRPS19.Wpre\** (PGK.CoRPS19) or *EF1α(s).CoRPS19.Wpre\** (EF1α.CoRPS19) therapeutic vectors or the *PGKEGFP.Wpre\* control vector* (PGK EGFP). **b)** Number of vector copies (VCN) integrated into cells maintained in liquid culture and **c)** in CFCs. The degree of significance was determined by the Mann Whitney test. The number of experiments performed for condition: *PGK.EGFP.Wpre\* vector* :N= 4, *PGK.CoRPS19.Wpre vector* N=5, and for *EF1α(s).CoRPS19.Wpre\**: N= 3. The graph represents the mean value corresponding to the transduction of CD34$^+$ cells from 5 different patients with the corresponding standard deviation.

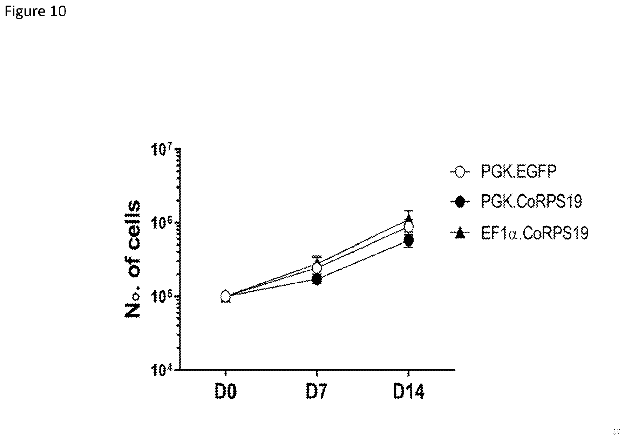

**Figure 10. Analysis of the effect of therapeutic vector transduction on the ex vivo expansion in liquid culture of DBA patients' hematopoietic progenitors.** Number of bone marrow hematopoietic progenitor cells from DBA patients successfully transduced with *PGK.CoRPS19.Wpre\** (PGK.CoRPS19) or *EF1α(s).CoRPS19.Wpre\** (EF1α.CoRPS19) therapeutic vectors. The *PGK.EGFP.Wpre\* vector* (PGK EGFP) was used as control. The cell number was measured at day 0 before transduction (D0) and at two different time points after transduction (D7 and D14, after 7 and 14 days).

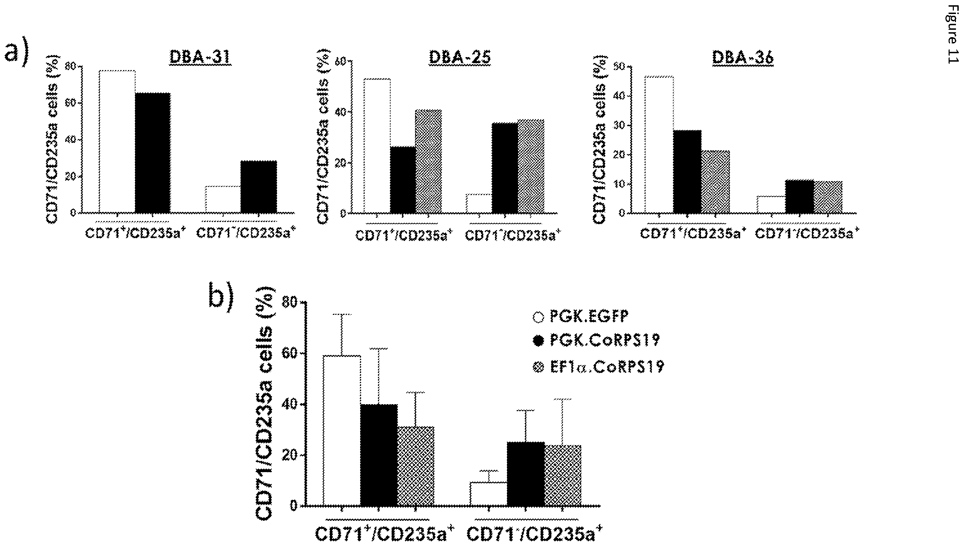

**Figure 11. Analysis of the erythroid differentiation process of cells from DBA patients corrected with therapeutic vectors:** Quantification of the percentage of CD71$^+$/CD235a$^+$ and CD71$^-$/CD235a$^+$ (CD71/CD235a) cell populations in BM CD34$^+$ DBA cells transduced with the *PGK.CoRPS19.Wpre\** (PGK.CoRPS19) or the *EF1α(s).CoRPS19.Wpre\** (EF1α.CoRPS19) therapeutic vectors or the *PGK.EGFP.Wpre\** control vector (PGK.EGFP). The figure shows **a)** the individual analyses of 3 patients (DBA-31, DBA-25, and DBA-36) and b) the bulk analysis. The degree of significance was determined by the Mann Whitney test. The graphs show the mean and standard deviation.

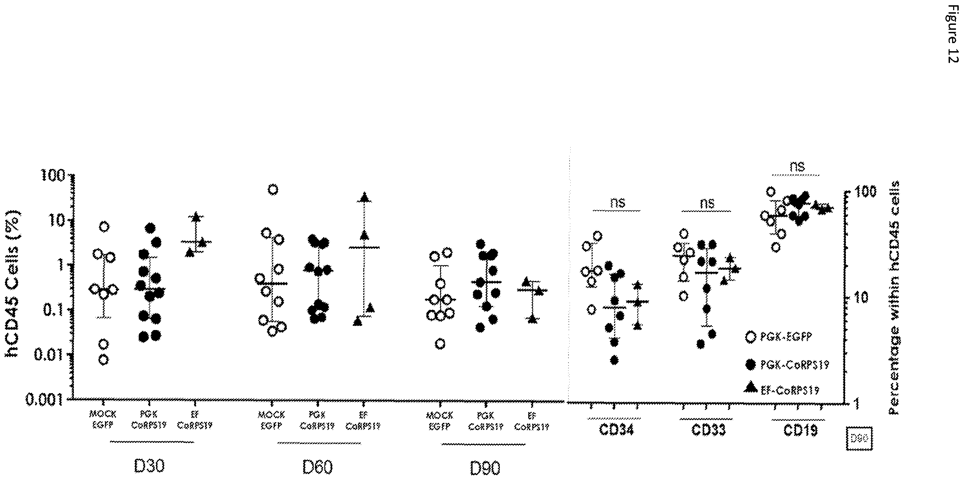

**Figure 12. Analysis of the reconstitution potential of DBA patients' cells transduced with the *PGK.CoRPS19.Wpre\** or *EF1α(s).CoRPS19.Wpre\** therapeutic vectors, or the *PGK.EGFP.Wpre\* control vector transplanted* in NSG immunodeficient mice:** Engraftment level (percentage of hCD45+ cells) of BM CD34+ cells from DBA patients corrected with the therapeutic vectors at three different time points after cells transplantation (30 days (D30),60 days (D60) and 90 days (D90)) and determination of multilineage potential of differentiation in myeloid cells (CD33$^+$), lymphoid cells (CD19$^+$) and HSC (CD34$^+$).The degree of significance was determined using the Mann Whitney test (P-value; * ≤ 0.05; ** ≤ 0.01; *** ≤ 0.001; **** ≤ 0.0001; ns: no significant). The graphs show the mean and standard deviation. MOCK EGFP: *PGK.EGFP.Wpre\** control vector; PGK CoRPS19: *PGK.CoRPS19.Wpre\** vector; EF.CoRPS19, *EF1α(s).CoRPS19.Wpre\** vector.

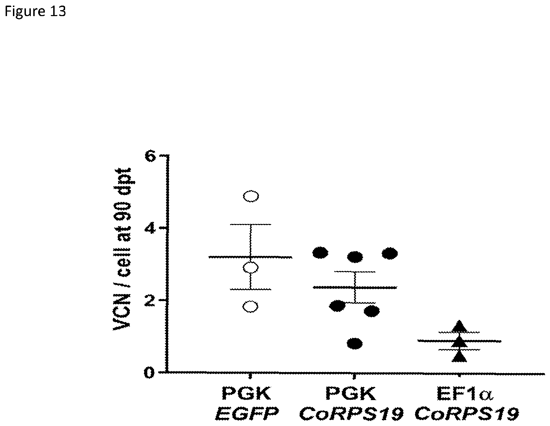

**Figure 13. Quantification of vector copy number (VCN) on day 90 post-transplant in DBA patient cells transduced with the therapeutic vectors or an EGFP vector control and engrafted in NSG recipient mice.** Statistical significance was determined using the Mann Whitney test (P value <0.05).

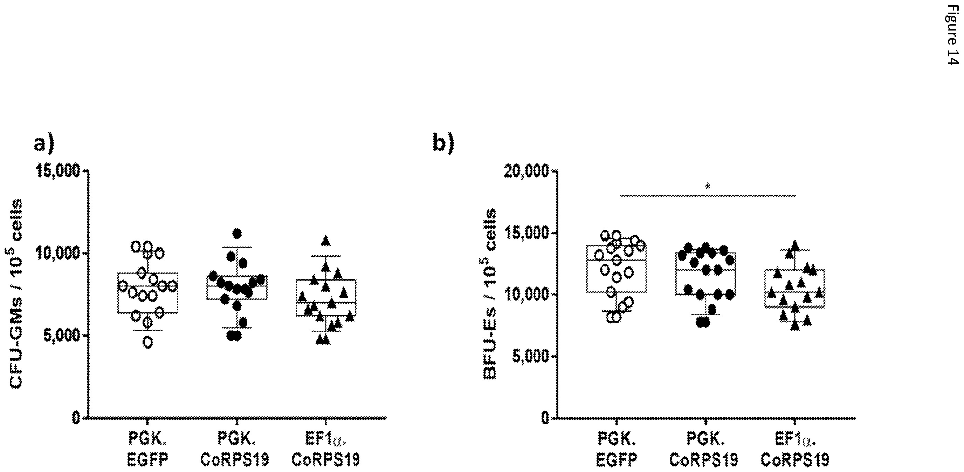

**Figure 14. Quantification of the number of colonies generated from healthy donors umbilical cord CD34$^+$ cells transduced with the *PGK.CoRPS19.Wpre\** or the *EF1α(s).CoRPS19.Wpre\** therapeutic vectors or the *PGK.EGFP.Wpre\** control vector: a)** Number of granulocyte-macrophage progenitor colony forming units_(CFU-GMs) for every 10$^5$ mononuclear cells (MNC) seeded. **b)** Number of BFU-Es for every 10$^5$ MNC seeded. The degree of significance was determined using the Mann Whitney test (P-value; * ≤ 0.05; ** ≤ 0.01; *** ≤ 0.001; **** ≤ 0.0001). The graph shows the mean and standard deviation of 5 experiments. PGK.EGFP: PGK.EGFP.Wpre* control vector; PGK.CoRPS19: *PGK.CoRPS19.Wpre\** vector; EF1α.CoRPS19: *EF1α(s).CoRPS19.Wpre\** vector.

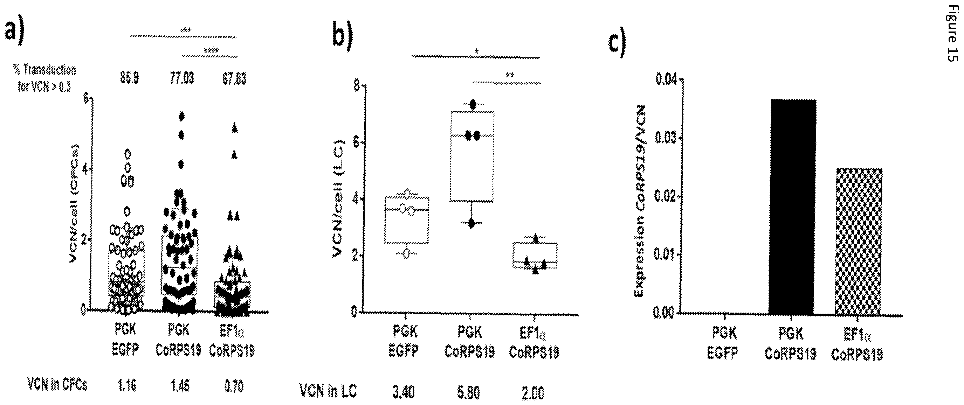

**Figure 15. Determination of the VCN per cell, percentage of transduction and level of expression of the *CoRPS19* transgene in colonies and liquid culture of healthy donors CD34$^+$ cells transduced with the *PGK.CoRPS19.Wpre\** or *EF1α(s).CoRPS19.Wpre\** therapeutic vectors, or the *PGK.EGFP.Wpre\** control vector: a)** Determination of the vector copy number (VCN) per cell in colonies and percentage of transduction in CD34$^+$ cells. **b)** Determination of the VCN per cell in liquid culture (LC). **c)** Expression of *CoRPS19* normalized for the VCN per cell. The statistical significance was determined using the Mann Whitney test, the Mann Whitney test (P-value; $* \leq 0.05$; $** \leq 0.01$; $*** \leq 0.001$; $**** \leq 0.000105$). Graph in panel a) shows the mean and standard deviation of 5 experiments, graph in b) of 4 experiments, and graph in c) of 3 experiments. PGK EGFP: *PGK.EGFP.Wpre** control vector; PGK CoRPS19*: PGK.CoRPS19.Wpre** vector; EF1$\alpha$ CoRPS19: *EF1$\alpha$(s).CoRPS19.Wpre** vector.

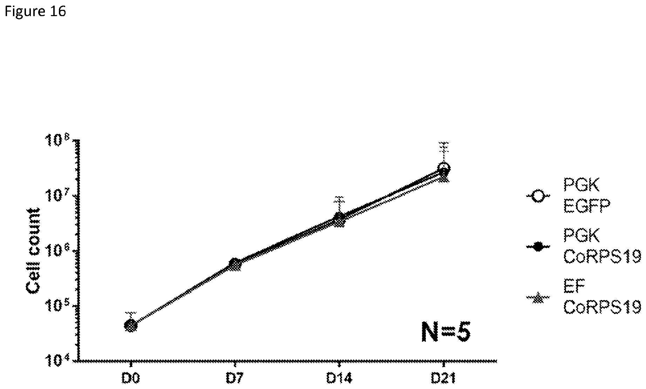

**Figure 16. Growth curve of healthy donors umbilical cord CD34$^+$ cells transduced with the *PGK.CoRPS19.Wpre*** or *EF1$\alpha$(s).CoRPS19.Wpre*** therapeutic vectors and the *PGK.EGFP.Wpre***control vector.** The degree of significance was determined by the Mann Whitney test (P value <0.05). The graph shows the mean and standard deviation of 5 experiments (N=5). D, days; PGK EGFP: *PGK.EGFP.Wpre** control vector; PGK CoRPS19: *PGK.CoRPS19.Wpre** vector; EF CoRPS19: *EF1$\alpha$(s).CoRPS19.Wpre** vector.

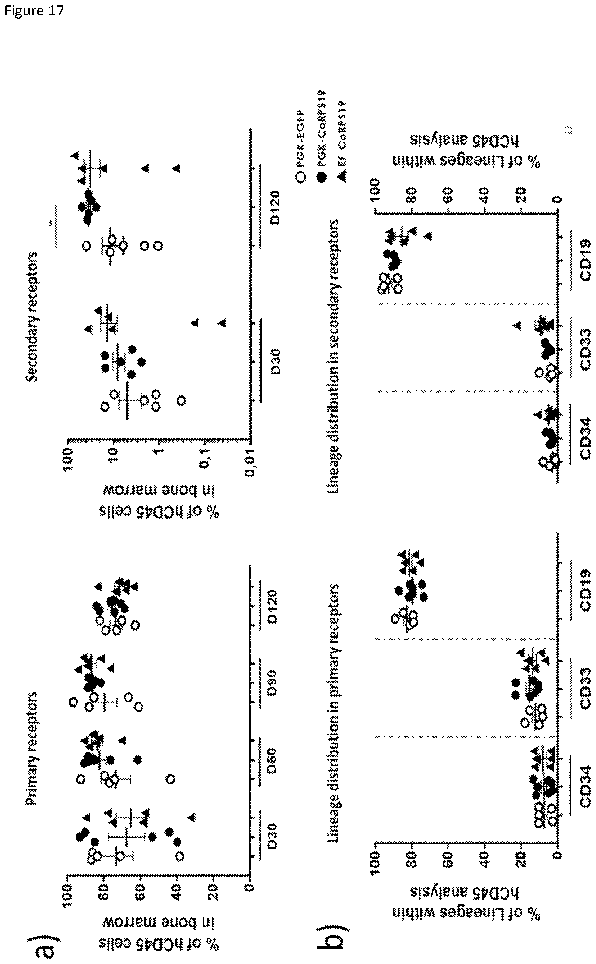

**Figure 17. Analysis of the repopulation and differentiation potential of healthy donors CD34$^+$ cells transduced with the *PGK.CoRPS19.Wpre*** or the *EF1$\alpha$(s).CoRPS19.Wpre*** therapeutic vectors or the *PGK.EGFP.Wpre*** control vector:** a) The repopulation potential was determined by quantifying the percentage of hCD45$^+$ cells in primary and secondary recepients. b) Distribution of myeloid (CD33$^+$), lymphoid (CD19$^+$) and HSC (CD34$^+$) cells in primary and secondary recipients. The statistical significance was determined using the Mann Whitney test (P-value; $* \leq 0.05$; $** \leq 0.01$; $*** \leq 0.001$; $**** \leq 0.000105$). The graph shows the mean and standard error of the mean of two safety experiments conducted after transducing CD34$^+$from cord blood of healthy donors and repopulating in vivo hematopoiesis of NSG mice. D, days; PGK EGFP: *PGK.EGFP.Wpre** control vector; PGK CoRPS19: *PGK.CoRPS19.Wpre** vector; EF CoRPS19: *EF1$\alpha$(s).CoRPS19.Wpre** vector.

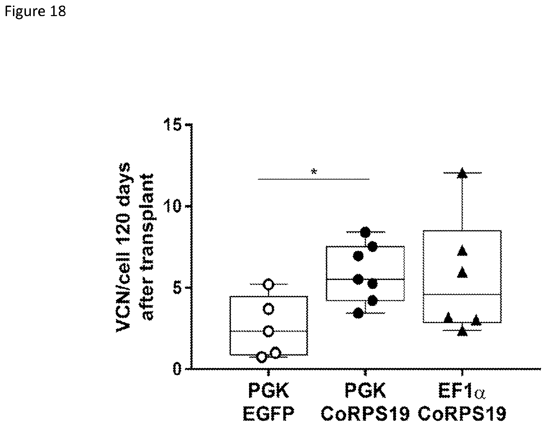

**Figure 18. VCN quantification in healthy donors CD34+ cells transduced with the *PGK.CoRPS19.Wpre*** or the *EF1$\alpha$(s).CoRPS19.Wpre*** therapeutic vectors or the *PGK.EGFP.Wpre*** control vector and transplanted in NSG mice as primary recipients.** The statistical significance was determined using the Mann Whitney test (P-value; $* \leq 0.05$; $** \leq 0.01$; $*** \leq 0.001$; $**** \leq 0.0001$). The graph shows the mean and standard error of the mean of two different experiments conducted to analyze safety and toxicity effect in vivo of the therapeutic vectors after transducing healthy donors CD34$^+$from cord blood and transplanting them in NSG mice. PGK EGFP: *PGK.EGFP.Wpre** control vector; PGK CoRPS19: *PGK.CoRPS19.Wpre** vector; EF CoRPS19: *EF1$\alpha$(s).CoRPS19.Wpre** vector.

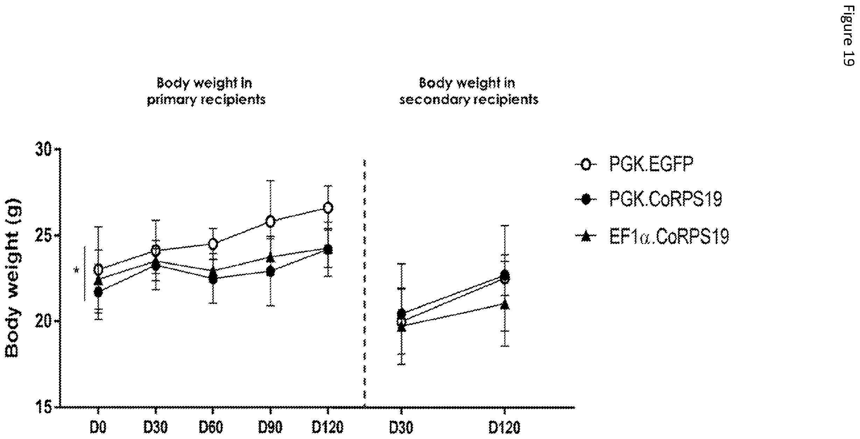

**Figure 19. Determination of the body weight of NSG mice transplanted with healthy donors CD34$^+$ cells transduced with the *PGK.CoRPS19.Wpre*** or the *EF1$\alpha$(s).CoRPS19.Wpre*** therapeutic vectors and the *PGK.EGFP.Wpre*** control vector.** The statistical significance was determined using the Mann Whitney test (P-value; $* \leq 0.05$; $** \leq 0.01$; $*** \leq 0.001$; $**** \leq 0.000105$). The graph shows the mean and standard error of the mean of two different experiments conducted to analyze safety and toxicity effect in vivo of the therapeutic vectors after transducing healthy donors CD34$^+$from cord blood and transplanting them in NSG mice. D, days;_PGK EGFP: *PGKEGFP.Wpre** control vector; PGK CoRPS19: *PGK.CoRPS19.Wpre** vector; EF CoRPS19: *EF1$\alpha$(s).CoRPS19.Wpre** vector.

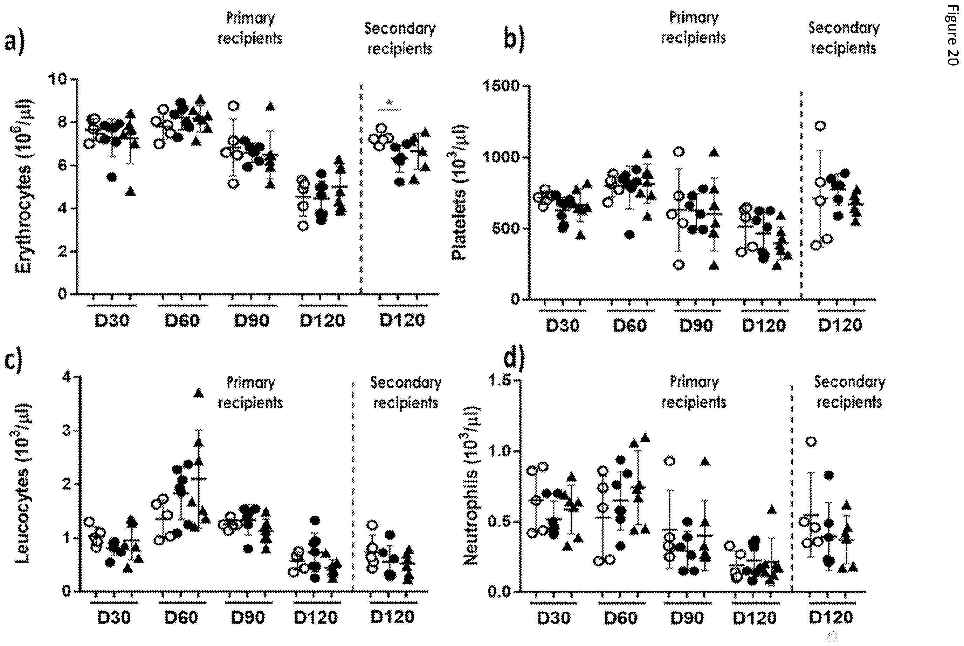

**Figure 20. Determination of hematological parameters in NSG mice transplanted with healthy donors CD34$^+$ cells transduced with the *PGK.CoRPS19.Wpre*** or the *EF1$\alpha$(s).CoRPS19.Wpre*** therapeutic vectors or the *PGK.EGFP.Wpre*** control vector:** a) erythrocytes, b) leukocytes, c) platelets and d) neutrophils. The statistical significance was determined using the Mann Whitney test (P-value; $* \leq 0.05$; $** \leq 0.01$; $*** \leq 0.001$; $**** \leq 0.000105$). The graph shows the mean and standard error of the mean of two different experiments conducted to analyze safety and toxicity effect in vivo of the therapeutic vectors after transducing healthy donors CD34$^+$from cord blood and transplanting them in NSG mice. D, days; PGK EGFP: *PGK.EGFP.Wpre** control vector; PGK CoRPS19: *PGK.CoRPS19.Wpre** vector; EF CoRPS19: *EF1$\alpha$(s).CoRPS19.Wpre** vector.

## DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" is used herein to mean approximately, roughly, around, or in the regions of. When the term "about" is used in conjunction with a numerical range, it modifies that range by extending the boundaries above and below the numerical values set forth. In general, the term "about" is used herein to modify a numerical value above and below the stated value by a variance of 10 percent, up or down (higher or lower).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element.

A protein or nucleotide that "consists essentially of" a protein or nucleotide is a protein or nucleotide that has considerably the same amino acid or nucleotide sequence as the specified protein or nucleotide.

A protein or nucleotide that has "essentially the same amino acid or nucleotide sequence" as a protein or nucleotide, respectively, typically has more than 90% amino acid or nucleotide identity with this protein or nucleotide. Included in this definition are conservative amino acid substitutions.

The term "isolated" for the purposes of the present invention designates a biological material (cell, polypeptide, polynucleotide, or a fragment, variant, or derivative thereof) that has been removed from its original environment (the environment in which it is naturally present).

By "enriched" as used herein is meant that the purity or percentage of a specific population of cells, such as $CD34^+$ cell, is increased in at least 20%, 30%, 40%, 50%, 60%, 70%, 75%, 80%, 85%, 90%, 91%, 92%, 93%, 94%, 95%, 96%, 97%, 98%, 99%, 100% with respect to the total cells comprised in the composition. Methods to enrich a specific population of cells are known in the art, e.g., by using specific kits that use negative or positive selection.

"Nucleic acids," "nucleic acid molecules," "oligonucleotide," and "polynucleotide" are used interchangeably and refer to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single strandedform, or a double-stranded helix. The term nucleic acid molecule, and in particular DNA or RNA molecule, refers only to the primary and secondary structure of the molecule, and does not limit it to any particular tertiary forms. Thus, this term includes double-stranded DNA found, inter alia, in linear or circular DNA molecules (e.g., restriction fragments), plasmids, supercoiled DNA and chromosomes.

By "codon optimized" is referred herein as to enhance expression by replacing the wild type represented codons with more traditionally stable codons to maximize the chance of obtaining a functional and active protein.

A "coding region" or "coding sequence" is a portion of polynucleotide which consists of codons translatable into amino acids.

A "promoter" as used herein encompasses a DNA sequence that directs the binding of RNA polymerase and thereby promotes RNA synthesis, i.e., a minimal sequence sufficient to direct transcription. Promoters and corresponding protein or polypeptide expression may be ubiquitous meaning active in a wide range of cells, tissues and species or cell-type specific, tissue-specific, or species specific. The promoter sequence may be composed of different promoter fragments (either different or the same fragments) that are localized closely in the DNA sequence and may be separated by linkers or spacers. Such promoters are referred to as chimeric promoters.

An "enhancer" as used herein encompasses a cis-acting element that stimulates or inhibits transcription of adjacent genes. An enhancer that inhibits transcription also is termed a "silencer". Enhancers can function (i.e., can be associated with a coding sequence) in either orientation, over distances of up to several kilobase pairs (kb) from the coding sequence and from a position downstream of a transcribed region.

The term "downstream" refers to a nucleotide sequence that is located 3' to a reference nucleotide sequence or sequences. In certain embodiments, downstream nucleotide sequences relate to sequences that follow the starting point of transcription.

The term "upstream" refers to a nucleotide sequence that is located 5' to a reference nucleotide sequence. In certain embodiments, upstream nucleotide sequences relate to sequences that are located on the 5' region a nucleotide region.

As used herein, the term "gene regulatory region" or "regulatory region" refers to nucleotide sequences located upstream (5' sequences), within, or downstream (3' sequences) of a coding region, and which influence the transcription, RNA processing, stability, or translation of the associated coding region. Regulatory regions can include promoters, translation leader sequences, introns, polyadenylation recognition sequences, RNA processing sites, effector binding sites and stem-loop structures. If a coding region is intended for expression in a eukaryotic cell, a polyadenylation signal and transcription termination sequence will usually be located 3' to the coding sequence.

The term "posttranscriptional regulatory element" refers to a DNA sequence that when transcribed creates a tertiary structure which enhances or inhibits the expression of a protein.

"Transcriptional control sequences" refer to DNA regulatory sequences, such as promoters, enhancers, terminators, and the like, that provide for the expression of a coding sequence in a host cell.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The term "therapeutically effective amount" refers to an amount of matter which has a therapeutic effect, and which is able to treat DBA.

The terms "individual", "patient" or "subject" are used interchangeably in the present application and are not meant to be limiting in any way. The "individual", "patient" or "subject" can be of any age, sex and physical condition.

The term "host cell", as used herein refers to a cell which has been transduced, infected, transfected or transformed with a vector. The vector may be a plasmid, a viral particle, a phage, etc. The culture conditions, such as temperature, pH and the like, are those previously used with the host cell selected for expression, and will be apparent to those skilled in the art. It will be appreciated that the term "host cell" refers to the original transduced, infected, transfected or transformed cell and progeny thereof.

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulphur containing reducing agents, such as urea, glutathione, thioctic acid, sodium thioglycolate, thioglycerol, [alpha]-monothioglycerol, and sodium thio sulphate; low molecular weight proteins, such as human serum albumin, bovine serum albumin, gelatin, or other immunoglobulins; and hydrophilic polymers, such as polyvinylpyrrolidone.

As used herein, the phrase "subject in need thereof includes subjects, such as mammalian subjects, that would benefit from administration of a polynucleotide molecule, a polypeptide, or vector provided herein, e.g., to correct a genetic defect, to correct a defective function of a protein or to improve homeostasis.

As used herein, the term "optimized," with regard to nucleotide sequences, refers to a polynucleotide sequence that encodes a polypeptide, wherein the polynucleotide sequence has been mutated to enhance a property of that polynucleotide sequence.

The term "lentivirus" refers to a genus of retroviruses that can infect and replicate in human cells, causing diseases in the human and other mammalian species. A "vector" is any vehicle which can be used to artificially carry foreign genetic material into a cell. Thus, a "lentiviral vector" refers to a recombinant lentivirus which carries a polynucleotide into a cell. The term covers all subtypes and both naturally occurring and recombinant forms, except where required otherwise. The term "LV" is an abbreviation for lentivirus and may be used to refer to the virus itself or derivatives thereof.

As used herein, the term "gene" or "coding sequence" refers to a nucleotide sequence in vitro or in vivo that encodes a gene product. In some instances, the gene consists or consists essentially of coding sequence, that is, sequence that encodes the gene product.

By "transcriptional unit" as used herein is meant the nucleotide sequence comprised in the LV vector of the present invention that comprises from 5' to 3' a phosphoglycerate kinase (PGK) promoter or an Elongation factor 1 alpha (EF1$\alpha$) in its short version (EF1$\alpha$(s)), a codon-optimized nucleotide sequence encoding for the RPS19 protein (CoRPS19), a woodchuck hepatitis virus posttranscriptional regulatory element (Wpre), preferably a mutated Wpre, and at least a polyadenylation (polyA) signal sequence; wherein a phosphoglycerate kinase (PGK) promoter or an Elongation factor 1 alpha (EF1$\alpha$), the Wpre and the polyadenylation (polyA) signal sequence are operably linked to and regulate the expression of CoRPS19.

## DESCRIPTION

As stated above, DBA patients are typically haplo-insufficient for RPS19 gene. This means that, although one of the alleles of RPS19 gene is unaffected and functional, there is not enough expression of RPS19 protein, which eventually leads to dysfunctional ribosomes and the development of the disease.

Previous studies have used gene therapy (GT) to try to correct the expression of RPS19 protein by using lentiviral vectors (LV) where the *RPS19* gene is placed under the control of strong viral promoters with the aim to obtain higher expression levels of RPS19 protein. However, although human promoters such as PGK or EF1$\alpha$ are known to be safer than the strong viral promoters, it may be that PGK or EF1$\alpha$ are not sufficiently strong to drive the expression of a protein (RPS19) in the context of a specific disease (such as autosomal-dominant disorders, i.e. DBA) where high amounts of said protein are required.

The authors of the present invention show herein that lentiviral vectors comprising human promoters are able to express sufficient amount of RPS19 protein to correct the alteration of ribosomal biogenesis. In particular, two self-inactivating lentiviral vectors (SIN-LV) have been developed with two different human promoters: the phosphoglycerate kinase (PGK) and elongation factor 1$\alpha$ in its short version (EF1$\alpha$(s)) promoters. As shown in Fig. 7, both LVs were able to express a codon-optimized RPS19 protein (named CoRPS19) and correct the alteration of ribosomal biogenesis of the K562 cell line, whose expression of the RPS19 gene had been silenced by interference RNA. It is also shown herein that in CD34$^+$ cells from the bone marrow of patients deficient in the RPS19, these LV vectors increased 1.5 and 3.9 times respectively, compared to the control group transduced with a non-therapeutic vector carrying the gene of green fluorescent protein (EGFP), the number of granulomacrophagic (CFU-GM) and erythroid (BFU-Es) colonies. Thus, it was unexpectedly found that, even though if the human PGK promoter was only able to increase the expression of RPS19 in 1.5 times, this increase turned out to be sufficient to achieve a correction of ribosomal biogenesis in a severe model of DBA; K562 cell line where expression of RPS19 was nearly completely abrogated.

In addition, the therapeutic vectors reversed the characteristic erythroid differentiation defect of erythroid progenitors from DBA patients, increasing the production of mature CD71$^-$/CD235a$^+$ erythroid cells 2.5 times. Likewise, it is also shown herein that the transduction of these progenitors preserved the repopulation capacity of these cells in immunodeficient NSG mice, suggesting that this capacity of the hematopoietic stem cells of DBA patients is not severely affected. Toxicity studies showed that the overexpression of the optimized version of the RPS19 gene in cells from healthy donors was not toxic.

In conclusion, these results open the path to the use of these LV vectors for DBA therapy since they demonstrate the feasibility of correcting RPS19 protein expression deficiency in patients suffering from this disease. Particularly, these results show the potential use of the lentiviral vector expressing RPS19 protein under the control of PGK promoter in the treatment of DBA.

Thus, in a **first aspect,** the present invention provides a lentivirus vector (LV) comprising a polynucleotide, wherein the polynucleotide is characterized in that it comprises a transcriptional unit, wherein the transcriptional unit is defined herein as comprising, from 5' to 3', a promoter selected from the group consisting of a phosphoglycerate kinase (hereinafter referred to as PGK) promoter or an Elongation factor 1 alpha (hereinafter referred to as EF1$\alpha$) promoter or functional variants thereof, a nucleotide sequence encoding for the human RPS19 protein, preferably a codon-optimized nucleotide sequence encoding for human RPS19 protein (hereinafter referred to as CoRPS19) or a functional variant thereof, and a woodchuck hepatitis virus posttranscriptional regulatory element (hereinafter referred to as Wpre, preferably a mutated Wpre, or a functional variant thereof; wherein the PGK or the EF1$\alpha$ promoters and the Wpre are operably linked to and regulate the expression of RPS19 or CoRPS19. A coding sequence and a gene expression control sequence are said to be operably linked when they are linked in such a way as to place the expression or transcription and/or translation of the coding sequence under the influence or control of the gene expression control sequence For example, the PGK or EF1$\alpha$ promoter and the Wpre are operably linked to the nucleotide sequence encoding CoRPS19 protein so that the expression levels of CoRPS19 are regulated by one of the promoters and the Wpre.

In an embodiment, the polynucleotide comprised in the LV vector according to the first aspect further comprises one or more lentiviral backbone elements, which are defined as polynucleotides encoding for the necessary proteins for the LV to carry out its function (infect the cell and integrate into the viral genome to persist in the cell) and polynucleotides encoding for the necessary regulatory sequences for the LV to carry out its functions.

Thus, the LV vector of the first aspect comprises a polynucleotide, wherein the polynucleotide is characterized in that it comprises 1) a transcriptional unit and 2) one or more LV vector backbone elements, or a combination thereof. Each of these two components and their polynucleotides are characterized in detail below:

1) The transcriptional unit

As defined above, by transcriptional unit is referred herein as to the polynucleotide sequence comprised in the polynucleotide of the LV vector of the present invention that comprises from 5' to 3' a promoter selected from the group consisting of PGK or E1Fα, a nucleotide sequence encoding for the RPS19 protein, preferably CoRPS19, and a Wpre element, preferably a mutated Wpre, wherein the PGK or E1Fα promoter and the Wpre are operably linked to and regulate the expression of RPS19 or CoRPS19.

• *The promoter*

As stated above, the promoter comprised in the transcriptional unit is selected from the group consisting of PGK or E1Fα promoters. Said promoters lead the expression of RPS19, preferably the CoRPS19, after lentiviral vector genome integration into the target cells.

In some embodiments, the promoter in the lentiviral vector selectively enhances the expression of RPS19, preferably CoRPS19, protein in a target cell. In some embodiments, the polynucleotide molecule comprised in the lentiviral vector is stably integrated into the genome of the target cell or target tissue, for example, in the genome of a hematopoietic stem and progenitor cell.

In a preferred embodiment, the promoter comprised in the polynucleotide of the lentiviral vector provided herein is the PGK promoter. In an embodiment, the PGK promoter is human PGK promoter. Preferably, said PGK promoter comprises, consists, or consists essentially of SEQ ID NO: 10 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 10. In some embodiments, the PGK promoter comprises, consists, or consists essentially of SEQ ID NO: 10.

In an embodiment, the promoter comprised in the polynucleotide of the lentiviral vector provided herein is the E1Fα promoter. In a preferred embodiment, the E1Fα promoter is the short version of the E1Fα promoter as described in Shubhranshu et al. 2017 (Shubhranshu et al., Lentiviral Vectors with Cellular Promoters Correct Anemia and Lethal Bone Marrow Failure in a Mouse Model for Diamond-Blackfan Anemia, Molecular Therapy, Volume 25, Issue 8, 2017, Pages 1805-1814, ISSN 1525-0016, https://doi.org/10.1016/j.ymthe.2017.04.002). In an embodiment, the E1Fα promoter is human E1Fα promoter. Preferably, said short E1Fα promoter comprises, consists, or consists essentially of SEQ ID NO: 11 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 11. In some embodiments, the short E1Fα promoter comprises, consists, or consists essentially of SEQ ID NO: 11.

• *The nucleotide sequence encoding for the RPS19 protein, preferably a codon-optimized RPS 19 protein (CoRPS19).*

The lentiviral vectors provided herein comprise in the transcriptional unit a nucleotide sequence encoding for the human RPS19 protein or a functional fragment thereof. Preferably, the transcriptional unit comprises a codon optimized version of the human RPS19 gene (herein after referred to as CoRPS19). In one embodiment, the *RPS19* or CoRPS19 is operably linked to at least one, preferably two, expression control sequences, including a promoter and a posttranscriptional element. In some embodiments, the present invention provides a lentiviral vector comprising an isolated polynucleotide molecule comprising a nucleotide sequence encoding a polypeptide with similar activity to RPS19.

Preferably, said codon-optimized nucleotide sequence encoding for CoRPS19 protein comprises, consists, or consists essentially of SEQ ID NO: 12 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 12. In some embodiments, the codon-optimized nucleotide sequence encoding for CoRPS19 protein comprises, consists, or consists essentially of SEQ ID NO: 12.

• *WHV (woodchuck hepatitis virus) post-transcriptional regulatory element (Wpre)*

Lentiviral vectors provided herein also comprise in the transcriptional unit at least one posttranscriptional regulatory element operably linked to the nucleotide encoding for the RPS19, preferably CoRPS19, protein. In an embodiment, said posttranscriptional regulatory element is the WHV (woodchuck hepatitis virus) post-transcriptional regulatory element (Wpre). In an embodiment, the posttranscriptional regulatory element is mutated (also called Wpre*) to increase the safety of the vector. In a preferred embodiment, the mutated Wpre contains a mutation in gene X frame.

In a preferred embodiment, the nucleotide sequence comprising the WHV (woodchuck hepatitis virus) post-transcriptional regulatory element (Wpre) is the mutated Wpre element. Preferably, said nucleotide comprising the mutated Wpre comprises, consists, or consists essentially of SEQ ID NO: 13 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 13. In some embodiments, the nucleotide sequence comprising the mutated Wpre comprises, consists, or consists essentially of SEQ ID NO: 13.

It is to be understood that all the embodiments described above for the different elements of the transcription unit (the PGK or E1Fα promoters, the nucleotide sequence encoding for the RPS19, preferably CoRPS19 protein, and the Wpre element, preferably Wpre*) can be combined with each other.

In a preferred embodiment, the transcription unit comprised in the polynucleotide of the lentiviral vector consists of a promoter selected from the group consisting of phosphoglycerate kinase (PGK) promoter or Elongation factor 1 alpha (EF1α) promoter, a codon optimized nucleotide sequence encoding for the RPS19 protein, and a mutated a woodchuck hepatitis virus posttranscriptional regulatory element (Wpre), wherein the promoters and the Wpre element are operably linked to and regulate the expression of a nucleotide sequence encoding for the RPS19 protein.

In a preferred embodiment, the transcription unit comprised in the polynucleotide of the lentiviral vector consists of a phosphoglycerate kinase (PGK) promoter with at least 95% sequence identity over the full length with SEQ ID NO: 10, a codon optimized nucleotide sequence encoding for the CoRPS19 protein with at least 95%, preferably 98%, sequence identity over the full length of SEQ ID NO: 12, and a mutated a woodchuck hepatitis virus posttranscriptional regulatory element (Wpre) with at least 95%, preferably 98%, sequence identity over the full length of SEQ ID NO: 13, wherein the PGK promoter and the mutated Wpre element are operably linked to and regulate the expression of a nucleotide sequence encoding for the CoRPS19 protein.

In a preferred embodiment, the transcription unit comprised in the polynucleotide of the lentiviral vector consists of a Elongation factor 1 alpha (EF1α) promoter with at least 95% sequence identity over the full length with SEQ ID NO: 11, a codon optimized nucleotide sequence encoding for the CoRPS19 protein with at least 95%, preferably 98%, sequence identity over the full length of SEQ ID NO: 12, and a mutated a woodchuck hepatitis virus posttranscriptional regulatory element (Wpre) with at least 95%, preferably 98%, sequence identity over the full length of SEQ ID NO: 13, wherein the EF1α promoter and the mutated Wpre element are operably linked to and regulate the expression of a nucleotide sequence encoding for the CoRPS19 protein

### 2) Lentiviral vector backbone elements

As defined above, the lentiviral vector backbone elements are defined as polynucleotide sequences needed for the LV to carry out its function (infect the cell and integrate into the viral genome to persist in the cell).

Lentiviruses include members of the bovine lentivirus group, equine lentivirus group, feline lentivirus group, ovinecaprine lentivirus group, and primate lentivirus group. In some embodiments, the gene delivery vector is a self-limiting LV.

In some embodiments, the lentiviral vector provided herein is "third-generation" lentiviral vector. As used herein, the term "third-generation" lentiviral vector refers to a lentiviral packaging system that has the characteristics of a second-generation vector system, and that further lacks a functional tat gene, such as one from which the tat gene has been deleted or inactivated. Typically, the gene encoding rev is provided on a separate expression construct. As used herein, a "second-generation" lentiviral vector system refers to a lentiviral packaging system that lacks functional accessory genes, such as one from which the accessory genes vif, vpr, vpu, and nef have been deleted or inactivated. As used herein, "packaging system" refers to a set of viral constructs comprising genes that encode viral proteins involved in packaging a recombinant virus. Typically, the constructs of the packaging system will ultimately be incorporated into a packaging cell.

In some embodiments, the third-generation lentiviral vector provided herein is a self-inactivating lentiviral vector. In some embodiments, the lentiviral vector is a VSV.G pseudo type lentiviral vector.

In certain embodiments, the lentiviral vector is a vector of a recombinant lentivirus capable of infecting dividing and non-dividing cells. In certain embodiments, the lentiviral vector is a vector capable of infecting hematopoietic stem cells, long term hematopoietic stem cells, short term hematopoietic stem cells, multipotent progenitors, hematopoietic CD34+ cells and any cluster differentiation subpopulation within the CD34+ population. The lentiviral genome and the proviral DNA typically have the three genes found in retroviruses: gag, pol and env, which are flanked by two long terminal repeat (LTR) sequences. The gag gene encodes the internal structural (matrix, capsid and nucleocapsid) proteins; the pol gene encodes the RNA-directed DNA polymerase (reverse transcriptase), a protease and an integrase; and the env gene encodes viral envelope glycoproteins. The 5' and 3' LTR's serve to promote transcription and polyadenylation of the virion RNA's. The LTR contains all other cis-acting sequences necessary for viral replication. In some embodiments the lentiviruses have additional genes including vif, vpr, tat, rev, vpu, net and vpx (in HIV-I, HIV-2 and/or SIV).

In certain embodiments, the lentiviral vector has the HIV virulence genes env, vif, vpr, vpu and nef deleted without compromising the ability of the vector to transduce dividing and non-dividing cells.

In certain embodiments, a gene transfer cassette comprises one or more additional elements, e.g., one or more elements selected from the following: 5' LTR, primer binding site, DNA flap central polypurine tract, Rev responsive element, coding sequences, such as truncated gag sequences, packaging signals, 3'LTR, and/or PolyA signal.

As defined above, the lentiviral vector provided herein comprises a polynucleotide, wherein the polynucleotide comprises the transcription unit previously defined. In an embodiment, said polynucleotide comprised in the lentiviral vector further comprises upstream, that is, at the 5' region of the transcriptional unit as defined above, and in order from 5' to 3', at least one or more of the following polynucleotide sequences: a) a 5' long terminal repeat (5' LTR) which in turn comprises a chimeric cytomegalovirus (CMV) promoter, b) a primer binding site (PBS), c) a psi (Ψ) packaging signal, d) a Rev responsive element (RRE), and e) a DNA flap central polypurine tract (cPPT) or any combination thereof.

In another embodiment, said polynucleotide comprised in the lentiviral vector further comprises downstream, that is, at the 3' region of the transcriptional unit as defined above, i) a 3' long terminal repeat (LTR) and, optionally, a j) polyadenylation (polyA) signal sequence located after the 3' LTR. In some embodiments, the lentiviral vector comprises a deletion of the U3 region of the 5' and/or 3' LTR. The deletion of the U3 region of the LTR can be the complete deletion or a partial deletion. The lack of U3 sequence of wild-type LTR involves that the lentiviral vector construct is Tat-independent and, therefore, it will be produced in third generation.

It is to be understood that any combination possible of the nucleotides a) to e) and i) and j) is comprised in the present invention and in combination with the transcription unit described in the section above. Thus, the lentiviral vector may comprise only one, two, three, or more elements selected from a) to e) and i) and j) in combination with the transcription unit defined above. Each of the nucleotides a) to e) and i) and j) and their preferred embodiments are further defined below.

The 5' LTR nucleotide sequence is responsible for provirus transcription during lentiviral vector production. In the lentiviral vector construct provided herein, the 5' LTR comprises or consists of a chimeric form of the CMV promoter. Additionally, in the lentiviral vector construct provided herein, the 5' LTR may also comprise a part of wild-type HIV-1 5' LTR, named RU5. In a preferred embodiment, the 5' LTR comprises or consists of a chimeric form of the CMV promoter and a part of wild-type HIV-1 5' LTR called RU5 element. The chimeric CMV promoter leads the transcription of provirus during the lentiviral vector production. In an embodiment, the chimeric CMV promoter comprises or consists of a CMV enhancer (hereinafter referred to as CMV enhancer) and a CMV promoter (hereinafter referred to as CMV promoter).

In a preferred embodiment, the nucleotide sequence comprising the CMV enhancer comprises, consists, or consists essentially of SEQ ID NO: 3 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 3. In some embodiments, the nucleotide sequence comprising the CMV enhancer comprises, consists, or consists essentially of SEQ ID NO: 3.

In a preferred embodiment, the nucleotide sequence comprising the CMV promoter comprises, consists, or consists essentially of SEQ ID NO: 4 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 4. In some embodiments, the nucleotide sequence comprising the CMV promoter comprises, consists, or consists essentially of SEQ ID NO: 4.

In a preferred embodiment, the nucleotide sequence comprising the chimeric CMV promoter comprises, consists, or consists essentially of SEQ ID NO: 2 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 2. In some embodiments, the nucleotide sequence comprising the chimeric CMV promoter comprises, consists, or consists essentially of SEQ ID NO: 2.

As indicated above, the 5' LTR nucleotide further comprises a nucleotide sequence comprising a RU5 element. This sequence is necessary for retrotranscription and integration of proviral genome into transduced cells. In a preferred embodiment, the nucleotide sequence comprising the RU5 element comprises, consists, or consists essentially of SEQ ID NO: 5 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 5. In some embodiments, the nucleotide sequence comprising the RU5 element, comprises, consists, or consists essentially of SEQ ID NO: 5.

In a preferred embodiment, the nucleotide sequence comprising the 5' LTR comprises, consists, or consists essentially of SEQ ID NO: 1 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 1. In some embodiments, the nucleotide sequence comprising the 5' LTR comprises, consists, or consists essentially of SEQ ID NO: 1.

Adjacent to the 5' LTR there may be sequences necessary for reverse transcription of the genome (the tRNA primer binding site) and for efficient encapsidation of viral RNA into particles (the Psi site). The sequences necessary for encapsidation (or packaging of retroviral RNA into infectious virions) may be missing from the viral genome, the cis defect prevents encapsidation of genomic RNA. However, the resulting mutant remains capable of directing the synthesis of all virion proteins.

In an embodiment, downstream the 5' LTR are sequences necessary for reverse transcription of the genome, which are the tRNA primer binding site (hereinafter referred to as PBS or PBS SL23). Thus, in an embodiment, downstream of the 5' LTR nucleotide is the Primer Binding Site SL123 nucleotide. In this PBS element, the tRNA is merged to PBS element during reverse transcription of proviral genome after target cell transduction and before integration into the cell genome. In a preferred embodiment, the nucleotide sequence comprising the PBS SL123 comprises, consists, or consists essentially of SEQ ID NO: 6 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 6. In some embodiments, the nucleotide sequence comprising the PBS SL123 comprises, consists, or consists essentially of SEQ ID NO: 6.

In some embodiments, the lentiviral vector provided herein comprises at least a psi (Ψ) packaging signal, also called the psi site, for efficient encapsulation of viral RNA into particles. The psi (Ψ) packaging signal can be located downstream the PBS SL123. In a preferred embodiment, the nucleotide sequence comprising the psi (Ψ) packaging signal comprises, consists, or consists essentially of SEQ ID NO: 7 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 7. In some embodiments, the nucleotide sequence comprising the psi (Ψ) packaging signal comprises, consists, or consists essentially of SEQ ID NO: 7.

The lentiviral vector provided herein may contain a packaging signal (Ψ), which is involved in packaging the retroviral genome into the viral capsid, as defined above. LV vectors were thought to require approximately 300 bp of the Gag gene in this region. Currently, this Gag sequence has been reduced to just 40 bp. Thus, in an embodiment, the lentiviral vector provided herein further comprises a nucleotide sequence encoding for a truncated Gag protein, which is located downstream the psi (Ψ) packaging signal.

In an embodiment, the lentiviral vector provided herein further comprises a Rev-response element (hereinafter referred to as RRE), which will merge to this sequence in lentiviral vector transcripts to help nuclear exportation of provirus during lentiviral vector production. In an embodiment, the RRE element is located downstream the psi (Ψ) packaging signal. In a preferred embodiment, the nucleotide sequence comprising RRE element comprises, consists, or consists essentially of SEQ ID NO: 8 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 8. In some embodiments, the nucleotide sequence comprising RRE element comprises, consists, or consists essentially of SEQ ID NO: 8.

In an embodiment, the lentiviral vector provided herein further comprises a central polypurine tract (herein after referred to as cPPT), which acts as a primer for positive DNA chain transcription during reverse transcription of lentiviral vectors after transduction of target cells. cPPT also increases proviral DNA nuclear import and, therefore, lentiviral vector transduction efficacy. Moreover, it increases lentiviral vector titles. In an embodiment, the cPPT element is located downstream the RRE element. In a preferred embodiment, the nucleotide sequence comprising cPPT element comprises, consists, or consists essentially of SEQ ID NO: 9 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 9. In some embodiments, the nucleotide sequence comprising RRE element comprises, consists, or consists essentially of SEQ ID NO: 9.

The backbone elements defined above can be found upstream the transcription unit (i.e., towards the 5' region of the polynucleotide comprised in the lentiviral vector). However, the lentiviral vector provided herein may also have other backbone elements downstream the transcription unit (i.e., towards the 3' region of the polynucleotide comprised in the lentiviral vector). In an embodiment, the polynucleotide comprised in the lentiviral vector provided herein, further comprises a 3' LTR and, optionally, a polyadenylation (polyA) signal sequence. 3' LTR is involved in mRNAs polyadenylation during lentiviral vector production. In an embodiment, the integrated viral genome will contain a deletion in viral promoter region (U3), resulting in the transcriptional inactivation of potentially packable viral genomes in the transduced cells.

In an embodiment, the 3' LTR comprises a truncated U3 element. In an embodiment, the 3' LTR comprises the elements R and U5 from wild-type HIV-1. In an embodiment, the 3' LTR comprises a polyadenylation signal sequence. In a preferred embodiment, the 3' LTR comprises or consists of a truncated U3 element (hereinafter referred to as ΔU3 element), and the elements R and U5 from wild-type HIV-1 (hereinafter referred to as RU5 element).

In a preferred embodiment, the nucleotide sequence comprising the ΔU3 element comprised in the 3' LTR nucleotide comprises, consists, or consists essentially of SEQ ID NO: 15 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 15. In some embodiments, the nucleotide sequence comprising the ΔU3 element comprises, consists, or consists essentially of SEQ ID NO: 15.

In a preferred embodiment, the nucleotide sequence comprising the RU5 element comprised in the 3' LTR nucleotide comprises, consists, or consists essentially of SEQ ID NO: 16 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 16. In some embodiments, the nucleotide sequence comprising the RU5 element comprises, consists, or consists essentially of SEQ ID NO: 16.

In an embodiment, downstream the 3' LTR there is a SV40 polyadenylation signal sequence (hereinafter referred to as PolyA). In an embodiment, the polyA signal sequence is derived from the simian vacuolating virus 40 virus. Preferably, the nucleotide sequence comprising the PolyA located downstream the 3' LTR nucleotide comprises, consists, or consists essentially of SEQ ID NO: 33 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 33. In some embodiments, the nucleotide sequence comprising a polyA comprises, consists, or consists essentially of SEQ ID NO:33.

In a preferred embodiment, the nucleotide sequence comprising 3' LTR comprises, consists, or consists essentially of SEQ ID NO: 14 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 14. In some embodiments, the nucleotide sequence comprising 3' LTR comprises, consists, or consists essentially of SEQ ID NO: 14.

In a further preferred embodiment, the lentiviral vector provided herein comprises a polynucleotide, wherein the polynucleotide is characterized in that it comprises from 5' to 3':

a) a 5' long terminal repeat (5' LTR) which in turn comprises a chimeric cytomegalovirus promoter,
b) a primer binding site (PBS),
c) a psi (Ψ) packaging signal,
d) a Rev responsive element (RRE),
e) a DNA flap central polypurine tract (cPPT),
f) a promoter selected from the group consisting of a *phosphoglycerate kinase promoter (PGK)* or Elongation factor alpha short promoter (EF1α(s)),
g) a nucleotide sequence encoding for the RPS19 protein,
h) a mutated optimized woodchuck hepatitis virus posttranscriptional regulatory element (Wpre)
i) a 3' long terminal repeat (3' LTR),

wherein 5' and 3' LTR regions have been rendered substantially transcriptionally inactive by virtue of total or partial deletion in the U3 region of the LTR, and wherein f) and h) are operably linked to and regulate the expression of g).

In a further preferred embodiment, the lentiviral vector provided herein comprises a polynucleotide, wherein the polynucleotide is characterized in that it comprises from 5' to 3':

a) a 5' long terminal repeat (5' LTR) which in turn comprises a chimeric cytomegalovirus promoter,
b) a primer binding site (PBS),
c) a psi (Ψ) packaging signal,
d) a Rev responsive element (RRE),
e) a DNA flap central polypurine tract (cPPT),
f) a human phosphoglycerate kinase (PGK) promoter,
g) a nucleotide sequence encoding for the RPS19 protein,
h) a mutated optimized woodchuck hepatitis virus posttranscriptional regulatory element (Wpre)
i) a 3' long terminal repeat (3' LTR),

wherein 5' and 3' LTR regions have been rendered substantially transcriptionally inactive by virtue of total or partial deletion in the U3 region of the LTR, and wherein f) and h) are operably linked to and regulate the expression of g).

In a further preferred embodiment, the lentiviral vector provided herein comprises a polynucleotide, wherein the polynucleotide is characterized in that it comprises from 5' to 3'::

a) a 5' long terminal repeat (5' LTR) which in turn comprises a chimeric cytomegalovirus promoter,
b) a primer binding site (PBS),
c) a psi (Ψ) packaging signal,
d) a Rev responsive element (RRE),
e) a DNA flap central polypurine tract (cPPT),
f) a human phosphoglycerate kinase (PGK) promoter,
g) a nucleotide sequence encoding for the RPS19 protein,
h) a mutated optimized woodchuck hepatitis virus posttranscriptional regulatory element (Wpre)
i) a 3' long terminal repeat (3' LTR), and
j) a polyadenylation (PolyA) signal sequence,

wherein 5' and 3' LTR regions have been rendered substantially transcriptionally inactive by virtue of total or partial deletion in the U3 region of the LTR, and wherein f) and h) are operably linked to and regulate the expression of g).

In a further preferred embodiment, the lentiviral vector provided herein comprises a polynucleotide, wherein the polynucleotide is characterized in that it comprises from 5' to 3':

a) a 5' long terminal repeat (5' LTR) which in turn comprises a chimeric cytomegalovirus promoter wherein the 5'LTR has at least 95%, preferably 98%, most preferably 100%, sequence identity over the full length with SEQ ID NO: 1,
b) a primer binding site (PBS) with at least 95%, preferably 98%, most preferably 100%, sequence identity over the full length with SEQ ID NO: 6,
c) a psi (Ψ) packaging signal with at least 95%, preferably 98%, most preferably 100%, sequence identity over the full length with SEQ ID NO: 7,

d) a Rev responsive element (RRE) with at least 95%, preferably 98%, most preferably 100%, sequence identity over the full length with SEQ ID NO: 8,

e) a DNA flap central polypurine tract (cPPT) with at least 95%, preferably 98%, most preferably 100%, sequence identity over the full length with SEQ ID NO: 9,

f) a human phosphoglycerate kinase (PGK) promoter with at least 95%, preferably 98%, most preferably 100% sequence identity over the full length with SEQ ID NO: 10,

g) a nucleotide sequence encoding for the RPS19 protein, preferably a codon-optimized nucleotide sequence encoding for the CoRPS19 protein with at least 95%, preferably 98%, most preferably 100% sequence identity over the full length with SEQ ID NO: 12

h) a mutated optimized woodchuck hepatitis virus posttranscriptional regulatory element (Wpre) with at least 95%, preferably 98%, most preferably 100%, sequence identity over the full length with SEQ ID NO: 13, and

i) a 3' long terminal repeat (3' LTR) with at least 95%, preferably 98%, most preferably 100%, sequence identity over the full length with SEQ ID NO: 14,

wherein 5' and 3' LTR regions have been rendered substantially transcriptionally inactive by virtue of total or partial deletion in the U3 region of the LTR, and wherein f) and h) are operably linked to and regulate the expression of g).

In a further preferred embodiment, the lentiviral vector provided herein comprises a polynucleotide, wherein the polynucleotide is characterized in that it comprises from 5' to 3':

a) a 5' long terminal repeat (5' LTR) which in turn comprises a chimeric cytomegalovirus promoter wherein the 5'LTR has at least 95%, preferably 98%, most preferably 100%, sequence identity over the full length with SEQ ID NO: 1,

b) a primer binding site (PBS) with at least 95%, preferably 98%, most preferably 100%, sequence identity over the full length with SEQ ID NO: 6,

c) a psi packaging signal with at least 95%, preferably 98%, most preferably 100%, sequence identity over the full length with SEQ ID NO: 7,

d) a Rev responsive element (RRE) with at least 95%, preferably 98%, most preferably 100%, sequence identity over the full length with SEQ ID NO: 8,

e) a DNA flap central polypurine tract (cPPT) with at least 95%, preferably 98%, most preferably 100%, sequence identity over the full length with SEQ ID NO: 9,

f) a human phosphoglycerate kinase (PGK) promoter with at least 95%, preferably 98%, most preferably 100% sequence identity over the full length with SEQ ID NO: 10,

g) a nucleotide sequence encoding for the RPS19 protein, preferably a codon-optimized nucleotide sequence encoding for the CoRPS19 protein with at least 95%, preferably 98%, most preferably 100% sequence identity over the full length with SEQ ID NO: 12

h) a mutated optimized woodchuck hepatitis virus posttranscriptional regulatory element (Wpre) with at least 95%, preferably 98%, most preferably 100%, sequence identity over the full length with SEQ ID NO: 13,

i) a 3' long terminal repeat (3' LTR) with at least 95%, preferably 98%, most preferably 100%, sequence identity over the full length with SEQ ID NO: 14, and

j) a polyadenylation (PolyA) signal sequence with at least 95%, preferably 98%, most preferably 100%, sequence identity over the full length with SEQ ID NO: 33,

wherein 5' and 3' LTR regions have been rendered substantially transcriptionally inactive by virtue of total or partial deletion in the U3 region of the LTR, and wherein f) and h) are operably linked to and regulate the expression of g).

Additionally, as will be recognized by one of ordinary skill in the art, the polynucleotide cassettes may optionally contain other elements including, but not limited to restriction sites to facilitate cloning and regulatory elements for a particular gene expression vector.

In a preferred embodiment, the full-length polynucleotide comprised in the lentiviral vector provided herein comprises, consists, or consists essentially of SEQ ID NO: 17 (also referred herein as PGK.CoRPS19. Wpre*- LV sequence) or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 17. In a preferred embodiment, the full-length nucleotide comprised in the lentiviral vector provided herein comprises, consists, or consists essentially of SEQ ID NO: 17.

In a preferred embodiment, the full-length polynucleotide comprised in the lentiviral vector provided herein comprises, consists, or consists essentially of SEQ ID NO: 18 (also referred herein as EF1α(s).CoRPS19. Wpre*- LV sequence) or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 18. In a preferred embodiment, the full-length nucleotide comprised in the lentiviral vector provided herein comprises, consists, or consists essentially of SEQ ID NO: 18.

In an embodiment, the lipid coat of the viral particle can include membrane bound polypeptides that were previously present on the surface of the host cell

In an embodiment, the lentiviral vector according to the present invention or any of its embodiments is used to transduce human hematopoietic stem cells (HSCs), which may be subsequently transplanted into humans with DBA. Thus, in a **second aspect,** the present invention relates to a host cell (e.g., a CD34$^+$ hematopoietic cell) or a population of cells comprising (e.g., transduced) the lentiviral vector as defined in the first aspect or in any of its embodiments.

In other embodiments, the cell is a cell to be delivered to a subject in order to provide to the subject the gene product encoded by the lentivirus vector of the first aspect or any of its embodiments. In an embodiment, the genome of the host cell or cells comprises the polynucleotide comprised in the lentiviral vector of the first aspect or any of its embodiments, completely or partially. In an embodiment, the genome of the host cell or cells comprise at least the transcription unit as defined above. In an embodiment, the host cell or cells comprise in their genome the polynucleotide of the lentiviral vector according to the first aspect or any of its embodiments, either fully or partially. In certain embodiments, the cell is autologous to the subject to be treated or was obtained from the subject to be treated. In other embodiments, the cell is allogeneic to the subject to be treated or was obtained from a donor other than the subject to be treated In particular embodiments, the cell is a mammalian cell, e.g., a human cell. In particular embodiments, the cell is a CD34$^+$ cell obtained from a subject to be treated with the cell after it is transduced by a gene delivery vector disclosed herein. In particular embodiment, the cell is a CD34$^+$ cell obtained from a subject diagnosed with DBA.

In a preferred embodiment, the cells are enriched in CD34$^+$ hematopoietic stem and progenitor cells. In a preferred embodiment, the cells are enriched in CD34$^+$ hematopoietic stem and progenitor cells, wherein enriched is meant that at least 20%, 30%, 40%, 50%, 60%, 70%, 80%, 90%, 92%, 94%, 96%, 98% or 100% of the cells are CD34$^+$. In an embodiment of the second aspect, the host cell or the population of cells comprise or consist of an enriched population of CD34$^+$ hematopoietic cells. In an embodiment, said enriched population of CD34$^+$ hematopoietic stem and progenitor cells comprise in their genome the lentiviral vector according to the first aspect or any of its embodiments.

In another embodiment, the host cells can also be packaging cells in which the LV rep and cap genes are stably maintained in the host cell or producer cells in which the LV vector genome is stably maintained and packaged. Exemplary packaging and producer cells are derived from SF-9, 293, A549 or HeLa cells. LV vectors are purified and formulated using standard techniques known in the art. In another embodiment, the host cell or cells is used to produce the viral gene delivery vector.

In particular embodiments, the cell is a mammalian cell, e.g., a human cell. In some embodiments, the cell is a blood cell. In some embodiments, the cell is an erythroid cell. In some embodiments, the cell is a bone marrow cell, e.g., a lineage depleted bone marrow cell. In particular embodiments, the cell is a hematopoietic stem cell or a CD34$^+$ cell. In some embodiments, the cell is a hematopoietic stem cell. In some embodiments, the preferred cell is a CD34$^+$ hematopoietic stem cell. In some embodiments, the preferred cell is a committed hematopoietic erythroid progenitor cell. In an embodiment of the second aspect, the host cell or the population of cells are CD34$^+$ hematopoietic stem and progenitor cells. In some embodiments, the cells are selected from the group consisting of hematopoietic stem cells, long term hematopoietic stem cells, short term hematopoietic stem cells, multipotent progenitors, hematopoietic CD34$^+$ cells and any cluster differentiation subpopulation within the CD34$^+$ population. In one embodiment, CD34$^+$ cells are obtained, or collected from the bone marrow sample. In some embodiments, the bone marrow sample is depleted of CD16$^+$ white blood cells. In another embodiment, HSCs are obtained from peripheral blood. In one embodiment, the peripheral blood sample is depleted of erythrocytes. In some embodiments, the blood sample is depleted of CD16$^+$ white blood cells. Selection methods for CD34$^+$ cells may be positive selection, negative selection, or any combination thereof. In some embodiments the selection may be based on the expression of any of these markers separately or in combination: CD34$^+$, CD59$^+$, CD90/Thy1$^+$, CD38$^{low/-}$, c-Kit$^{-/low}$, CD16$^+$ and Lin$^-$. In particular embodiments, the preferred cell is a CD34$^+$ cell obtained from a subject to be treated with the cell after it is transduced by a gene delivery vector disclosed herein. In particular embodiment, the cell is a CD34$^+$ DBA cell obtained from a subject diagnosed with DBA. In another embodiment, the host cell or cells may be from established cell lines or they may be primary cells, where "primary cells", "primary cell lines", and "primary cultures" are used interchangeably herein o refer to cells and cells cultures that have been derived from a subject and allowed to grow *in vitro* for a limited or unlimited number of passages, i.e., splitting, of the culture. For example, primary cultures are cultures that may have been passaged 0 times, 1 time, 2 times, 4 times, 5 times, 10 times, or 15 times, but not enough times go through the crisis stage. Typically, the primary cell lines of the present invention are maintained for fewer than 10 passages *in vitro.* Embodiments of the present invention comprise mammalian cells (e.g., CD34$^+$ cells) transduced with a viral delivery vector, e.g., a LV vector containing the human RPS19 gene, preferably CoRPS19, and preferably under the control of the human PGK promoter.

In certain embodiments, when transducing a cell with the vector disclosed herein, the cells are contacted with the vector for about 30 minutes, about 1 hour, about 1.5 hours, about 2 hours, about 2.5 hours, about 3 hours, about 3.5 hours, about 4 hours, about 5 hours, about 6 hours, about 7 hours, about 8 hours, about 2 hours, about 16 hours, about 18 hours, about 20 hours, about 24 hours, about 36 hours, about 8 hours, about 60 hours. In some embodiments, the cells are transduced for less than 60 hours, less than 48 hours, less than 36 hours, or less than 24 hours.

In a **third aspect,** the present invention relates to an isolated nucleic acid comprising the polynucleotide comprised in the lentiviral vector as defined in the first aspect or any of its embodiments.

Pharmaceutical compositions

In a **fourth aspect,** the present invention provides a pharmaceutical composition comprising the lentiviral vector as defined in the first aspect or in any of its embodiments, the host cell or the population of cells according to the second aspect or any of its embodiments, and/or the polynucleotide as defined in the third aspect or any of its embodiments and a pharmaceutically acceptable carrier or diluent. Preferably, the pharmaceutical composition comprises or consists of a population of cells that are enriched in CD34$^+$ hematopoietic stem and progenitor cells transduced with the lentiviral vector according to the first aspect or any of its embodiments, and a pharmaceutically acceptable carrier or diluent.

In some embodiments, the host cell or the population of cells according to the second aspect or any of its embodiments, or the pharmaceutical composition of the fourth aspect or any of its embodiments are contacted or transduced with the lentiviral vector according to the first aspect or any of its embodiments, either *in vivo, in vitro,* or *ex vivo.* Preferably, the administration of the lentiviral vector is *ex vivo.*

A pharmaceutical composition as described herein may also contain other substances. These substances include, but are not limited to, cryoprotectants, lyoprotectants, surfactants, bulking agents, anti-oxidants, and stabilizing agents. In some embodiments, the pharmaceutical composition may be lyophilized. In a preferred embodiment, the pharmaceutical composition comprises a saline solution.

The term "*cryoprotectant*" as used herein, includes agents which provide stability to the lentiviral vector against freezing-induced stresses, by being preferentially excluded from the lentiviral vector's surface. Cryoprotectants may also offer protection during primary and secondary drying and long-term product storage. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In one embodiment, a lyoprotectant is added to a pharmaceutical composition described herein. The term "*lyoprotectant*" as used herein, includes agents that provide stability to the lentiviral vector during the freeze-drying or dehydration process (primary and secondary freeze- drying cycles), by providing an amorphous glassy matrix and by binding with the lentiviral vector's surface through hydrogen bonding, replacing the water molecules that are removed during the drying process. This helps to minimize product degradation during the lyophilization cycle and improve the long-term product stability. Non-limiting examples of lyoprotectants include sugars, such as sucrose or trehalose; an amino acid, such as monosodium glutamate, non-crystalline glycine or histidine; a methylamine, such as betaine; a lyotropic salt, such as magnesium sulphate; a polyol, such as trihydric or higher sugar alcohols, e.g., glycerin, erythritol, glycerol, arabitol, xylitol, sorbitol, and mannitol; propylene glycol; polyethylene glycol; pluronics; and combinations thereof. The amount of lyoprotectant added to a pharmaceutical composition is generally an amount that does not lead to an unacceptable amount of degradation of the strain when the pharmaceutical composition is lyophilized.

In some embodiments, a bulking agent is included in the pharmaceutical composition. The term "*bulking agent*" as used herein, includes agents that provide the structure of the freeze-dried product without interacting directly with the pharmaceutical product. In addition to providing a pharmaceutically elegant cake, bulking agents may also impart useful qualities in regard to modifying the collapse temperature, providing freeze-thaw protection, and enhancing the strain stability over long-term storage. Non-limiting examples of bulking agents include mannitol, glycine, lactose, and sucrose. Bulking agents may be crystalline (such as glycine, mannitol, or sodium chloride) or amorphous (such as dextran, hydroxyethyl starch) and are generally used in formulations in an amount from 0.5% to 10%.

Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition. As used herein, "*pharmaceutically acceptable carrier*" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulphate; low molecular weight proteins, such as human serum albumin, bovine serum albumin, gelatin, or other immunoglobulins; and hydrophilic polymers, such as polyvinylpyrrolidone.

The pharmaceutical composition may be prepared for oral, sublingual, buccal, intravenous, intramuscular, subcutaneous, intraperitoneal, conjunctival, rectal, transdermal, intrathecal, topical and/or inhalation-mediated administration. In a preferred embodiment, the pharmaceutical composition may be a solution which is suitable for intravenous, intramuscular, conjunctival, transdermal, intraperitoneal and/or subcutaneous administration. In another embodiment, the pharmaceutical composition may be a solution which is suitable for sublingual, buccal and/or inhalation-mediated administration routes. In an alternative embodiment, the pharmaceutical composition may be a gel or solution which is suitable for intrathecal administration. In an alternative embodiment, the pharmaceutical composition may be an aerosol which is suitable for inhalation-mediated administration. In a preferred embodiment, the pharmaceutical composition may be prepared for intrathecal administration.

The pharmaceutical composition may further comprise common excipients and carriers which are known in the state of the art. For solid pharmaceutical compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For solution for injection, the pharmaceutical composition may further comprise cryoprotectants, lyoprotectants, surfactants, bulking agents, anti-oxidants, stabilizing agents and pharmaceutically acceptable carriers. For aerosol administration, the pharmaceutical compositions are generally supplied in finely divided form along with a surfactant and propellant. The surfactant must, of course, be nontoxic, and is generally soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. For suppositories, traditional binders and carriers may include, for example, polyalkalene glycols or triglycerides. In a preferred embodiment, the carrier is a nanoparticles. In a more preferred embodiment, the nanoparticles act as a vehicle to introduce the polynucleotide comprised in the lentiviral vector of the present invention inside the cell.

Therefore, a suitable pharmaceutical composition for injection can comprise a buffer (e.g. acetate, phosphate or citrate buffer), a surfactant (e.g. polysorbate), optionally a stabilizer agent (e.g. human albumin), etc. However, in other embodiments compatible with the teachings herein, the lentiviral vector can be delivered directly to the site of the adverse cellular population, that is the hepatocytes, thereby increasing the exposure of the diseased tissue to the therapeutic agent.

The lentiviral vector provided herein can optionally be administered in combination with other agents that are effective in treating the disorder or condition in need of treatment (e.g., prophylactic or therapeutic). The administration of lentiviral vectors provided herein in conjunction or combination with an adjunct therapy means the sequential, simultaneous, coextensive, concurrent, concomitant or contemporaneous administration or application of the therapy and the disclosed polypeptides.

Medical uses and Schedule, route and dose of administration

In a **fifth aspect,** the present invention provides the lentiviral vector according to the first aspect or any of its embodiment, the host cell or the population of cells according to the second aspect or any of its embodiments, the isolated nucleic acid according to the third aspect or any of its embodiments, or the pharmaceutical composition of the fourth aspect or any of its embodiments for use as a medicament. In a **sixth aspect,** the present invention provides the lentiviral vector according to the first aspect or any of its embodiment, the host cell or the population of cells according to the second aspect or any of its embodiments, the isolated nucleic acid according to the third aspect or any of its embodiments, or the pharmaceutical composition of the fourth aspect or any of its embodiments for use in the treatment of Diamond Blackfan anemia (DBA), the use comprising administering to the subject said lentiviral vector, said host cell or population of cells, or said composition. In a preferred embodiment, the lentiviral vector comprising a nucleotide with at least 50%, 60%, 70%, 80%, 85%, 90%, 91%, 92%, 93%, 94%, 95%, 96%, 97%, 98%, 99% or 100% sequence identity over the full length with SEQ ID NO: 17 is used in the treatment of Diamond Blackfan anemia (DBA). Thus, methods and compositions of the present disclosure find use, e.g., in the treatment of Diamond Blackfan anemia.

The present invention also provides methods of treating, preventing, or ameliorating the DBA disorder in a subject in need thereof comprising administering to the subject a therapeutically effective amount of the lentiviral vector according to the first aspect or any of its embodiment, the host cell or the population of cells according to the second aspect or any of its embodiments, the isolated nucleic acid of the third aspect or any of its embodiments, or the pharmaceutical composition of the fourth aspect or any of its embodiments. In particular embodiments, the method is used to treat DBA, and the viral vector is a LV comprising an expression construct disclosed herein comprising a human PGK promoter operably linked to a RPS19 gene cDNA or coding sequence, and a mutated Wpre disclosed herein.

In another embodiment, the administration of a lentiviral vector, the host cell or the population of cells, or the composition provided herein does not induce an immune response in a subject or induces a very low immune response in said subject.

In some embodiments, the lentiviral vector, the host cell or the population of cells, or the composition of the present invention are administered as a single dose or multiple doses. In some embodiments, the lentiviral vector, the host cell or the population of cells, or the composition of the present invention dose are administered at once or divided into multiple sub-dose, e.g., two sub-doses, three sub-doses, four sub-doses, five sub-doses, six sub-doses, or more than six sub-doses. In some embodiments, more than one lentiviral vector is administered. Most preferably, the lentiviral vector, the host cell or the population of cells, or the composition of the present invention are administered as a single dose.

In some embodiments, the dose of lentiviral vector of the present invention, the host cell or the population of cells of the present invention or the pharmaceutical composition is administered repeated at least twice, at least three times, at least four times, at least five times, at least six times, at least seven times, at least eight times, at least nine times, or at least ten times.

The lentiviral vector of the present invention, the host cell or the population of cells of the present invention or the pharmaceutical composition can be administered locally or systemically. In one embodiment, the route of administration of the lentiviral vectors is parenteral. The term parenteral as used herein includes intravenous, intraarterial, intraperitoneal, intramuscular, subcutaneous, rectal or vaginal administration. In an embodiment, said lentiviral vector can be administrated intravenously, subcutaneously, intramuscularly, or via any mucosal surface, e.g., orally, sublingually, buccally, nasally, rectally, vaginally or via pulmonary route. The intravenous form of parenteral administration is preferred. In an embodiment, a form for administration would be a solution for injection, in particular for intravenous or intra arterial injection or drip.

Effective doses of the compositions provided herein, for the treatment of DBA vary depending upon many different factors, including means of administration, target site, physiological state of the subject, whether the subject is a human being or an animal, other medications administered, and whether treatment is prophylactic or therapeutic. Usually, the subject is a human but non-human mammals including transgenic mammals can also be treated. Treatment dosages can be titrated using routine methods known to those of skill in the art to optimize safety and efficacy. In one embodiment, the subjects include, but are not limited to, individuals with DBA. In some embodiments, the subject is a pediatric subject, whereas in other aspects, the subject is an adult subject.

The lentiviral vector, the host cell or the population of cells or the pharmaceutical composition of the present invention can be administered continuously or at specific timed intervals. Intervals between single dosages can be daily, weekly, monthly or yearly. Intervals can also be irregular depending on the development of the disease or the curative effects of said lentiviral vector. Transduced cells may be infused immediately after the transduction process is completed. Effective amounts of dose and/or dose regimen can readily be determined empirically from preclinical assays, from safety and escalation and dose range trials, individual clinician-patient relationships. *In vitro or in vivo assays* can be employed to determine optimal dose ranges and/or schedules for administration. Additionally, effective doses can be extrapolated from dose-response curves obtained from animal models.

In a preferred embodiment, the lentiviral vector of the present invention, the host cell or the population of cells of the present invention or the pharmaceutical composition is administered at a dose of at least $1 \times 10^9$ vector genomes/Kg body weight, preferably $1 \times 10^{10}$, $1 \times 10^{11}$ or $1 \times 10^{12}$ vector genomes/Kg body weight. More preferably, at least or about $4.6 \times 10^{12}$ vector genomes/Kg body weight are administered.

Typically, an effective amount to achieve a change in the DBA patient will be about $1 \times 10^8$ vector genomes or more, in some cases $1 \times 10^9$, $1 \times 10^{10}$, $1 \times 10^{11}$, $1 \times 10^{12}$, or $1 \times 10^{13}$ vector genomes or more, in certain instances, $1 \times 10^{14}$ vector genomes or more. In some cases, the amount of vector genomes that is delivered is at most about $1 \times 10^{15}$ vector genomes, e.g. $1 \times 10^{14}$ vector genomes or less, for example $1 \times 10^{13}$, $1 \times 10^{12}$, $1 \times 10^{11}$, $1 \times 10^{10}$, or $1 \times 10^9$ vector genomes or less, in certain instances $1 \times 10^8$ vector genomes, and typically no less than $1 \times 10^8$ vector genomes. In some cases, the amount of vector genomes that is delivered is $1 \times 10^{10}$ to $1 \times 10^{11}$ vector genomes. In some cases, the amount of vector genomes that is delivered is $1 \times 10^{10}$ to $3 \times 10^{12}$ vector genomes. In some cases, the amount of vector genomes that is delivered is $1 \times 10^9$ to $3 \times 10^{13}$ vector genomes. In some cases, the amount of vector genomes that is delivered is $1 \times 10^8$ to $3 \times 10^{14}$ vector genomes.

In an embodiment, the lentiviral vector may be administered at a concentration of $10^8$ vector genomes per ml or more, for example, $5 \times 10^8$ vector genomes per mL; $10^9$ vector genomes per mL; $5 \times 10^9$ vector genomes per mL, $10^{10}$ vector genomes per mL, $5 \times 10^{10}$ vector genomes per mL; $10^{11}$ vector genomes per mL; $5 \times 10^{11}$ vector genomes per mL; $10^{12}$ vector genomes per mL; $5 \times 10^{12}$ vector genomes per mL; $10^{13}$ vector genomes per mL; $1.5 \times 10^{13}$ vector genomes per mL; $3 \times 10^{13}$ vector genomes per mL; $5 \times 10^{13}$ vector genomes per mL; $7.5 \times 10^{13}$ vector genomes per mL; $9 \times 10^{13}$ vector genomes per mL; $1 \times 10^{14}$ vector genomes per mL, $5 \times 10^{14}$ vector genomes per mL or more, but typically not more than $1 \times 10^{15}$ vector genomes per mL.

In some cases, the lentiviral vector of the present invention, the host cell or the population of cells of the present invention or the pharmaceutical composition to be administered may be measured using multiplicity of infection (MOI). In some cases, MOI may refer to the ratio, or multiple of vector or viral genomes to the cells to which the nucleic acid may be delivered. In some cases, the MOI may be $1 \times 10^6$. In some cases, the MOI may be $1 \times 10^5$ - $1 \times 10^7$. In some

cases, the MOI may be $1 \times 10^4$ $-1 \times 10^{8}$. In some cases, recombinant viruses of the disclosure are at least about $1 \times 10^1$, $1 \times 10^2$, $1 \times 10^3$, $1 \times 10^4$, $1 \times 10^5$, $1 \times 10^6$, $1 \times 10^7$, $1 \times 10^8$, $1 \times 10^9$, $1 \times 10^{10}$, $1 \times 10^{11}$, $1 \times 10^{12}$, $1 \times 10^{13}$, $1 \times 10^{14}$, $1 \times 10^{15}$, $1 \times 10^{16}$, $1 \times 10^{17}$, and $1 \times 10^{18}$ MOI. In some, embodiments the range is from about 20 to about 400 MOI. In some cases, recombinant viruses of this disclosure are $1 \times 10^8$ to $3 \times 10^{14}$ MOI. In some cases, recombinant viruses of the disclosure are at most about $1 \times 10^1$, $1 \times 10^2$, $1 \times 10^3$, $1 \times 10^4$, $1 \times 10^5$, $1 \times 10^6$, $1 \times 10^7$, $1 \times 10^8$, $1 \times 10^9$, $1 \times 10^{10}$, $1 \times 10^{11}$, $1 \times 10^{12}$, $1 \times 10^{13}$. In some embodiments, the dose of cells patients receive by infusion will be that which is obtained from the transduction process. In various preferred embodiments, at least at least about $1 \times 10^1$, $1 \times 10^2$, $1 \times 10^3$, $1 \times 10^4$, $1 \times 10^5$, $1 \times 10^6$, $1 \times 10^7$, $1 \times 10^8$, or more CD34⁺cells/KG of patient weight are infused into the patient. In some embodiments, between $1 \times 10^6$ and $4 \times 10^6$ CD34⁺cells/KG of patient weight are infused into the patient. In other embodiments, $3 \times 10^6$ and $4 \times 10^6$ CD34⁺ cells/Kg of patient weight are infused into the patient.

In some embodiments, the amount of pharmaceutical composition comprises about $1 \times 10^8$ to about $1 \times 10^{15}$ recombinant viruses, about $1 \times 10^9$ to about $1 \times 10^{14}$ recombinant viruses, about $1 \times 10^{10}$ to about $1 \times 10^{11}$ recombinant viruses, or about $1 \times 10^{11}$ to about $3 \times 10^{12}$ recombinant viruses.

Doses intermediate in the above ranges are also intended to be within the scope of the invention.

In order to achieve successful gene therapy in DBA, it is beneficial to collect from a subject a "sufficient" number of hematopoietic stem cells (HSC). In some embodiments of the present invention, the HSCs are obtained from a subject following mobilization. Mobilization may be achieved by treating the subject with drugs or compounds that cause the movement of stem cells from the bone marrow into the blood. The stem cells can be collected and stores. In some embodiments, mobilization is achieved by treating the subject with G-CSF (filgrastin). In other embodiments, mobilization is achieved by treating the subject with plerixafor. In yet other embodiments, mobilization is achieved by treating the subject with a combination of filgrastim and plerixafor.

The dosage and frequency of administration of the lentiviral vector of the present invention, the host cell or the population of cells of the present invention or the pharmaceutical composition may vary depending on whether the treatment is prophylactic or therapeutic. In prophylactic applications, compositions containing the lentiviral vector provided herein are administered to a subject not already in the disease state to enhance the subject's resistance or minimize effects of disease. Such an amount is defined to be a "prophylactic effective dose." A relatively low dosage is administered at relatively infrequent intervals over a long period of time. Some subjects may continue to receive treatment for the rest of their lives.

In some embodiments, methods are provided wherein cells are contacted with the lentivirus, the polynucleotide or the composition according to the present invention. As stated above, the contacting can occur *in vitro*, *in vivo*, or *ex vivo*. In addition, the present invention includes a method of transducing a mammalian cell, e.g. a human hematopoietic stem cell or other cell described herein, comprising contacting the cell with a gene delivery vector, e.g., a LV vector, disclosed herein or comprising a transcription unit described herein. In certain embodiments, the cell was previously obtained from a subject to be treated, or from another donor. In particular embodiments, the subject was diagnosed with DBA, and the cell is transduced with a LV comprising an expression cassette encoding the human RPS19 gene or cDNA. It is understood that the disclosed methods, e.g., those used to deliver the RPS19 gene product, e.g., using a RPS19 cDNA sequence, to a subject may also be used to treat DBA. In particular embodiments, the transduced cells are a population of cells obtained from a subject with DBA, who is to be treated with the cells once they have been transduced. The cells may be obtained from bone marrow or blood. In certain embodiments the subject with DBA is treated with agents to mobilize stem cells, then blood is drawn from the subject, red blood cells are removed, and CD34⁺ cells are selected. Following selection, the cells are then transduced. In particular embodiments, the transduced cells are stored or frozen before use, whereas in certain embodiments, they are provided to the subject immediately or shortly after they are transduced, e.g., within one hour, two hours, or four hours.

Other embodiments of the invention will be apparent to those skilled in the art from consideration of the specification and practice of the invention disclosed herein. It is intended that the specification and examples be considered as exemplary only, with a true scope and spirit of the invention being indicated by the appended claims.

**EXAMPLES**

**EXAMPLE 1- Materials and Methods**

*1. Lentiviral vector construction*

Two therapeutic lentiviral vectors suitable for clinical use in *RPS19* haplo-insufficient patients have been developed. These two vectors carry the codon-optimized version of the human RPS19 gene (mutated in 25% of DBA patients). In these LVs, expression of *RPS19* is driven by the human phosphoglycerate kinase promoter *(PGK); in the PGK.CoRPS19.Wpre* LV*), or by the short version of the human elongation factor-1alpha promoter (EF-1alpha short o EF1α(s)); in the *EF1α(s).CoRPS19.Wpre* LV*). In addition, both vectors harbor a mutated *Wpre* sequence (*Wpre**) that increases the production of the protein of interest by stabilizing its mRNA (See **Figure 1**).

The reason why two different promoters were chosen was the intensity with which they were able to direct the expression of the transgene of interest. On the one hand, we have the PGK promoter which shows stable expression of the gene of interest. Second, the EF1α promoter, was chosen for its greater activity.

These vectors are self-inactivated lentiviral vectors. They were generated from a lentiviral vector developed in our laboratory, carrying a codon optimized version of the PKLR gene, and developed in turn from the vector *pCCL.sin.ppt.hPGK.EGFPY.Wpre**, built and provided by Dr. Naldini's laboratory (HSRTIGET, San Raffaele Telethon Institute or Gene Therapy and Vita Salute San Raffaele University Medical School, Milano, Italy). This vector carried the phosphoglycerate kinase (PGK) promoter. The first step in the development of the *PGK.CoRPS19.Wpre** therapeutic vector consisted of extracting the PKLR gene and introducing an optimized version of the RPS19 gene, using the restriction targets Xbal and Sail, which were introduced by chemical synthesis during design in the sequence of the transgene of interest.

The second therapeutic lentiviral vector (*EF1α(s).CoRPS19.Wpre*LV*) has as its differential element the promoter of the human Elongation Factor Alpha (EF1α of Elongation factor 1 alpha) in its short version (EF1α(s)). In this case, to replace the PGK promoter with EF1α (s), the EF1α(s) sequence was amplified by PCR using *p'HR.EF1αS.eGFP.Wpre* as template vector (provided by the laboratory of Dr. Adrian Trasher; UCL Great Ormond Street Institute Of Child Health). To introduce the EcoRV and Sall restriction enzyme sequence targets into the sequence obtained with the EF1α(s) promoter, their sequence targets were included together with two nucleotide bases (EcoRV-AT and -TC-Sall) at the 3 'and 5' ends of the oligo used. The fragment obtained was isolated and cloned using the Zero Blunt® TOPO® Cloning Kit system. Once the EF1α(s) promoter was cloned, it was isolated using the EcoRV and Sail restriction enzyme targets, and the cloned previously extracting the PGK promoter from the backbone of the first therapeutic vector, thus generating the second therapeutic lentiviral vector *EF1α(s).CoRPS19.Wpre* *.

Thus, in an aspect of the present invention, a gene therapy approach to treat DBA consisting of autologous CD34[+] enriched cells transduced with a self-inactivating lentiviral vector containing the codon-optimized RPS19 gene (CoRPS19) is proposed.

1) Self-inactivating lentiviral vectors (SIN-LV) provide a more robust expression and are less susceptible to transcriptional silencing than gammaretroviral vectors (Pfeifer et al. 2002). They also show a much safer integration profile (Mitchell et al. 2004; Schroder et al. 2002; Wu et al. 2003) and because of the 400 base pairs (bp) deletion that they carry in the 3' LTR sequence (Miyoshi et al. 1998), transgene expression is regulated by internal promoters, increasing the safety of the LV-based genetic modification.

2) Vector sequence also includes several modifications to improve transgene expression and safety in target cells:

- The use of the human phosphoglycerate kinase (PGK) promoter, already characterized by its stable and long term expression in vivo after reinfusion of the gene-corrected HSCs in patients and improved safety properties compared to other promoters used in gene therapy (Biffi et al. 2013; Modlich et al. 2009; Montini et al. 2006). PGK leads to a more physiological expression of the transgene and a lower susceptibility to transcriptional silencing (Garcon et al. 2013; Zychlinski et al. 2008).

- A codon-optimized version of the nucleotide sequence encoding for the RPS19 protein (CoRPS19) to increase mRNA stability upon transcription. For the optimization, the GeneScript® software has been used, increasing the GC content and removing cryptic splice sites in order to avoid transcriptional silencing and therefore increase transgene expression. The CoRPS19 optimized sequence showed 81% homology with the human RPS19 gene, with no changes in the amino acids sequence of the protein.

- A mutated post-transcriptional regulatory element of the woodchuck hepatitis virus (*Wpre**), lacking any residual open reading frame is also included to improve the level of expression and stability of the therapeutic gene.

### 1. 1 Sequences:

### 1.1.1 Element sequences

**5' LTR or Long terminal repeat (SEQ ID NO: 1):** it is responsible for provirus transcription during lentiviral vector production. In this lentiviral vector construct 5' LTR is composed of a chimeric form of the CMV promoter and a part of wild-type HIV-1 5' LTR, RU5. The lack of U3 sequence of wild-type 5' LTR involves that this lentiviral vector construct is Tat-independent and, therefore, it will be produced in third generation.

GACATTGATTATTGACTAGTTATTAATAGTAATCAATTACGGGGTCATTAGTTCATAGCCC
ATATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAGGG
ACTTTCCATTGACGTCAATGGGTGGAGTATTTACGGTAAACTGCCCACTTGGCAGTACA
TCAAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTAC
GTATTAGTCATCGCTATTACCATGGTGATGCGGTTTTGGCAGTACATCAATGGGCGTGG
ATAGCGGTTTGACTCACGGGGATTTCCAAGTCTCCACCCCATTGACGTCAATGGGAGTT
TGTTTTGGCACCAAAATCAACGGGACTTTCCAAAATGTCGTAACAACTCCGCCCCATTGA
CGCAAATGGGCGGTAGGCGTGTACGGTGGGAGGTCTATATAAGCAGAGCTCGTTTAGT
GAACCG**GGGTCTCTCTGGTTAGACCAGATCTGAGCCTGGGAGCTCTCTGGCTAACTAG
GGAACCCACTGCTTAAGCCTCAATAAAGCTTGCCTTGAGTGCTTCAAGTAGTGTGTGC
CCGTCTGTTGTGTGACTCTGGTAACTAGAGATCCCTCAGACCCTTTTAGTCAGTGTGG
AAAATCTCTAGCAG**

- **Chimeric CMV promoter (SEQ ID NO: 2):** This sequence leads the transcription of provirus during the lentiviral vector production. It is composed by two different sequences, one with enhancer activity and the other one with promoter activity.

GACATTGATTATTGACTAGTTATTAATAGTAATCAATTACGGGGTCATTAGTTCATAG
CCCATATATGGAGTTCCGCGTTACATAACTTACGGTAAATGGCCCGCCTGGCTGAC
CGCCCAACGACCCCCGCCCATTGACGTCAATAATGACGTATGTTCCCATAGTAACG
CCAATAGGGACTTTCCATTGACGTCAATGGGTGGAGTATTTACGGTAAACTGCCCAC
TTGGCAGTACATCAAGTGTATCATATGCCAAGTACGCCCCCTATTGACGTCAATGAC
GGTAAATGGCCCGCCTGGCATTATGCCCAGTACATGACCTTATGGGACTTTCCTACT
TGGCAGTACATCTACGTATTAGTCATCGCTATTACCATGGTGATGCGGTTTTGGCAG
TACATCAATGGGCGTGGATAGCGGTTTGACTCACGGGGATTTCCAAGTCTCCACCC
CATTGACGTCAATGGGAGTTTGTTTTGGCACCAAAATCAACGGGACTTTCCAAAATG
TCGTAACAACTCCGCCCCATTGACGCAAATGGGCGGTAGGCGTGTACGGTGGGAG
GTCTATATAAGCAGAGCT

◦ **CMV enhancer (SEQ ID NO: 3):**

GACATTGATTATTGACTAGTTATTAATAGTAATCAATTACGGGGTCATTAGTTCAT
AGCCCATATATGGAGTTCCGCGTTACATAACTTACGGTAAATGGCCCGCCTGGC
TGACCGCCCAACGACCCCCGCCCATTGACGTCAATAATGACGTATGTTCCCATA
GTAACGCCAATAGGGACTTTCCATTGACGTCAATGGGTGGAGTATTTACGGTAA
ACTGCCCACTTGGCAGTACATCAAGTGTATCATATGCCAAGTACGCCCCCTATTG
ACGTCAATGACGGTAAATGGCCCGCCTGGCATTATGCCCAGTACATGACCTTAT
GGGACTTTCCTACTTGGCAGTACATCTACGTATTAGTCATCGCTATTACCATG

◦ **CMV promoter (SEQ ID NO: 4):**

GTGATGCGGTTTTGGCAGTACATCAATGGGCGTGGATAGCGGTTTGACTCACGG
GGATTTCCAAGTCTCCACCCCATTGACGTCAATGGGAGTTTGTTTTGGCACCAAA
ATCAACGGGACTTTCCAAAATGTCGTAACAACTCCGCCCCATTGACGCAAATGG
GCGGTAGGCGTGTACGGTGGGAGGTCTATATAAGCAGAGCT

- **RU5: Truncated 5' LTR without RU3 (SEQ ID NO: 5):** This sequence is necessary for retrotranscription and integration of proviral genome into transduced cells.

**GGGTCTCTCTGGTTAGACCAGATCTGAGCCTGGGAGCTCTCTGGCTAAC
TAGGGAACCCACTGCTTAAGCCTCAATAAAGCTTGCCTTGAGTGCTTCA
AGTAGTGTGTGCCCGTCTGTTGTGTGACTCTGGTAACTAGAGATCCCTC
AGACCCTTTTAGTCAGTGTGGAAAATCTCTAGCAG**

**PBS SL123 (Primer Binding Site) (SEQ ID NO: 6):** tRNA is merged to PBS element during reverse transcription of proviral genome after target cell transduction and before integration into the cell genome.

TGGCGCCCGAACAGGGAC

ψ **(packaging signal) (SEQ ID NO: 7):** This element is responsible for dimerization and packaging of lentiviral vector particles.

CTCTCTCGACGCAGGACTCGGCTTGCTGAAGCGCGCACGGCAAGAGGCGAGGGGCGG
CGACTGGTGAGTACGCCAAAAATTTTGACTAGCGGAGGCTAGAAGGAGAGAGATGGGT
GCGAGAGCGTC

**RRE (Rev-response element) (SEQ ID NO: 8):** Rev will merge to this sequence in lentiviral vector transcripts to help nuclear exportation of provirus during lentiviral vector production.

AGGAGCTTTGTTCCTTGGGTTCTTGGGAGCAGCAGGAAGCACTATGGGCGCAGCGTCA
ATGACGCTGACGGTACAGGCCAGACAATTATTGTCTGGTATAGTGCAGCAGCAGAACAA
TTTGCTGAGGGCTATTGAGGCGCAACAGCATCTGTTGCAACTCACAGTCTGGGGCATCA
AGCAGCTCCAGGCAAGAATCCTGGCTGTGGAAAGATACCTAAAGGATCAACAGCTCCT

**cPPT (central polypurin tract) (SEQ ID NO: 9):** This element act as a primer for positive DNA chain transcription during reverse transcription of lentiviral vectors after transduction of target cells. It also increases proviral DNA nuclear import and, therefore, lentiviral vector transduction efficacy. Moreover, it increases lentiviral vector titles.

TTTTAAAAGAAAAGGGGGGATTGGGGGGTACAGTGCAGGGGAAAGAATAGTAGACATA
ATAGCAACAGACATACAAACTAAAGAATTACAAAAACAAATTACAAAAATTCAAAATTTT

**1.1.2. Eukaryotic Internal Promoters (either one or another):**

- **Phosphoglycerate kinase promoter (PGK) (SEQ ID NO: 10):**

GGGGTTGGGGTTGCGCCTTTTCCAAGGCAGCCCTGGGTTTGCGCAGGGACGCGGCTG
CTCTGGGCGTGGTTCCGGGAAACGCAGCGGCGCCGACCCTGGGTCTCGCACATTCTTC
ACGTCCGTTCGCAGCGTCACCCGGATCTTCGCCGCTACCCTTGTGGGCCCCCCGGCGA
CGCTTCCTGCTCCGCCCCTAAGTCGGGAAGGTTCCTTGCGGTTCGCGGCGTGCCGGAC
GTGACAAACGGAAGCCGCACGTCTCACTAGTACCCTCGCAGACGGACAGCGCCAGGG
AGCAATGGCAGCGCGCCGACCGCGATGGGCTGTGGCCAATAGCGGCTGCTCAGCAGG
GCGCGCCGAGAGCAGCGGCCGGGAAGGGGCGGTGCGGGAGGCGGGGTGTGGGGCG
GTAGTGTGGGCCCTGTTCCTGCCCGCGCGGTGTTCCGCATTCTGCAAGCCTCCGGAGC
GCACGTCGGCAGTCGGCTCCCTCGTTGACCGAATCACCGACCTCTCTCCCCAG

- **Elongation factor 1 alpha promoter in its short version (EF1α(s)) (SEQ ID NO: 11):**

ATCATGGCTCCGGTGCCCGTCAGTGGGCAGAGCGCACATCGCCCACAGTCCCCGAGA
AGTTGGGGGGGAGGGGTCGGCAATTGAACCGGTGCCTAGAGAAGGTGGCGCGGGGTAA
ACTGGGAAAGTGATGTCGTGTACTGGCTCCGCCTTTTTCCCGAGGGTGGGGGGAGAACC
GTATATAAGTGCAGTAGTCGCCGTGAACGTTCTTTTTCGCAACGGGTTTGCCGCCAGAA
CACAGGTGTCGTGACGCTCG

**Codon optimized version of RPS19 (CoRPS19) (SEQ ID NO: 12):**

ATGCCCGGCGTGACCGTGAAGGACGTGAACCAGCAGGAGTTCGTGAGGGCCCTGGCA
GCCTTTCTGAAGAAGAGCGGCAAGCTGAAGGTGCCCGAGTGGGTGGACACAGTGAAG
CTGGCCAAGCACAAGGAGCTGGCCCCTTACGATGAGAACTGGTTCTATACCAGGGCAG
CCTCCACAGCAAGGCACCTGTACCTGAGGGGAGGAGCAGGAGTGGGCTCTATGACCAA
GATCTATGGCGGCAGGCAGCGCAATGGCGTGATGCCATCTCACTTTAGCCGGGGCAGC
AAGTCCGTGGCAAGGAGAGTGCTGCAGGCCCTGGAGGGCCTGAAGATGGTGGAGAAG
GACCAGGATGGCGGCAGAAAGCTGACACCACAGGGACAGCGGGACCTGGATAGAATC
GCAGGACAGGTGGCAGCAGCCAATAAGAAGCACTGATAG

**Mutated Wpre (WHV (woodchuck hepatitis virus) post-transcriptional regulatory element) (Wpre\*) (SEQ ID NO: 13):** It increases transgene expression in transduced cells due to improvements in polyadenylation, RNA export from the nuclei and protein synthesis. It contains a mutation in gene X frame to increase security.

CGAGCATCTTACCGCCATTTATTCCCATATTTGTTCTGTTTTTCTTGATTTGGGTATACAT

TTAAATGTTAATAAAACAAAATGGTGGGGCAATCATTTACATTTTTAGGGATATGTAATTA

CTAGTTCAGGTGTATTGCCACAAGACAAACATGTTAAGAAACTTTCCCGTTATTTACGCT

CTGTTCCTGTTAATCAACCTCTGGATTACAAAATTTGTGAAAGATTGACTGATATTCTTAA

CTATGTTGCTCCTTTTACGCTGTGTGGATATGCTGCTTTAATGCCTCTGTATCATGCTATT

GCTTCCCGTACGGCTTTCGTTTTCTCCTCCTTGTATAAATCCTGGTTGCTGTCTCTTTAT

GAGGAGTTGTGGCCCGTTGTCCGTCAACGTGGCGTGGTGTGCTCTGTGTTTGCTGACG

CAACCCCCACTGGCTGGGGCATTGCCACCACCTGTCAACTCCTTTCTGGGACTTTCGCT

TTCCCCCTCCCGATCGCCACGGCAGAACTCATCGCCGCCTGCCTTGCCCGCTGCTGGA

CAGGGGCTAGGTTGCTGGGCACTGATAATTCCGTGGTGTTGTCGGGGAAG

**3' LTR: DR3RU5 (SEQ ID NO: 14):** 3' LTR is involved in mRNAs polyadenylation during lentiviral vector production. In this case, 3' LTR is composed of a truncated U3 element and the elements R and U5 from wild-type HIV-1, being a self-inactivating lentiviral vector. The integrated viral genome will contain a deletion in viral promoter region (U3), resulting in the transcriptional inactivation of potentially packable viral genomes in the transduced cells.

TGGAAGGGCTAATTCACTCCCAACGAAGACAAGATCTGCTTTTTGCTTGTACTG**GGTCT**

**CTCTGGTTAGACCAGATCTGAGCCTGGGAGCTCTCTGGCTAACTAGGGAACCCACTG**

**CTTAAGCCTCAATAAAGCTTGCCTTGAGTGCTTC**AAGTAGTGTGTGCCCGTCTGTTGTG

TGACTCTGGTAACTAGAGATCCCTCAGACCCTTTTAGTCAGTGTGGAAAATCTCTAGCA

- **ΔU3 (SEQ ID NO: 15):**
  TGGAAGGGCTAATTCACTCCCAACGAAGACAAGATCTGCTTTTTGCTTGTACTG

- **RU5 (SEQ ID NO: 16):**

**GGTCTCTCTGGTTAGACCAGATCTGAGCCTGGGAGCTCTCTGGCTAACTAGGGAACC**

**CACTGCTTAAGCCTCAATAAAGCTTGCCTTGAGTGCTTC**AAGTAGTGTGTGCCCGTCTG

TTGTGTGACTCTGGTAACTAGAGATCCCTCAGACCCTTTTAGTCAGTGTGGAAAATCTCT

AGCAG

**SV40 Polyadenylation (PolyA) signal sequence (SEQ ID NO: 33):**

AACTTGTTTATTGCAGCTTATAATGGTTACAAATAAAGCAATAGCATCACAAATTTCACAA

ATAAAGCATTTTTTTCACTGCATTCTAGTTGTGGTTTGTCCAAACTCATCAATGTATCTTA

**1.1.3 Whole FASTA vector sequence**

A) *PGK.CoRPS19. Wpre*- LV sequence (SEQ ID NO: 17):

GTGTTTAAACCTAGATATTGATAGTCTGATCGGTCAACGTATAATCGAGTCCTAGCTTTT
GCAAACATCTATCAAGAGACAGGATCAGCAGGAGGCTTTCGCATGATTGAACAAGATGG
ATTGCACGCAGGTTCTCCGGCGGCTTGGGTGGAGAGGCTATTCGGCTATGACTGGGCA
CAACAGACAATCGGCTGCTCTGATGCCGCCGTGTTCCGGCTGTCAGCGCAGGGGCGTC
CGGTTCTTTTTGTCAAGACCGACCTGTCCGGTGCCCTGAATGAACTGCAAGACGAGGCA
GCGCGGCTATCGTGGCTGGCGACGACGGGCGTTCCTTGCGCGGCTGTGCTCGACGTT
GTCACTGAAGCGGGAAGGGACTGGCTGCTATTGGGCGAAGTGCCGGGGCAGGATCTC
CTGTCATCTCACCTTGCTCCTGCCGAGAAAGTATCCATCATGGCTGATGCAATGCGGCG
GCTGCATACGCTTGATCCGGCTACCTGCCCATTCGACCACCAAGCGAAACATCGCATC
GAGCGAGCACGTACTCGGATGGAAGCCGGTCTTGTCGATCAGGATGATCTGGACGAAG
AGCATCAGGGGCTCGCGCCAGCCGAACTGTTCGCCAGGCTCAAGGCGTCTATGCCCG
ACGGCGAGGATCTCGTCGTGACCCACGGCGATGCCTGCTTGCCGAATATCATGGTGGA
AAATGGCCGCTTTTCTGGATTCATCGACTGTGGCCGTCTGGGTGTGGCGGACCGCTAT
CAGGACATAGCGTTGGCTACCCGTGATATTGCTGAAGAGCTTGGCGGCGAATGGGCTG
ACCGCTTCCTTGTGCTTTACGGTATCGCCGCGCCCGATTCGCAGCGCATCGCCTTCTAT
CGCCTTCTTGACGAGTTCTTCTGACCGATTCTAGGTGCATTGGCGCAGAAAAAAATGCC
TGATGCGACGCTGCGCGTCTTATACTCCCACATATGCCAGATTCAGCAACGGATACGGC
TTCCCCAACTTGCCCACTTCCATACGTGTCCTCCTTACCAGAAATTTATCCTTAACGATC
GGACGGGGAGTCAGGCAACTATGGATGAACGAAATAGACAGATCGCTGAGATAGGTGC
CTCACTGATTAAGCATTGGTAACTGTCAGACCAAGTTTACTCATATATACTTTAGATTGAT
TTAAAACTTCATTTTTAATTTAAAAGGATCTAGGTGAAGATCCTTTTTGATAATCTCATGA
CCAAAATCCCTTAACGTGAGTTTTCGTTCCACTGAGCGTCAGACCCCGTAGAAAAGATC
AAAGGATCTTCTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCTTTTTCCGAA
GGTAACTGGCTTCAGCAGAGCGCAGATACCAAATACTGTCCTTCTAGTGTAGCCGTAGT
TAGGCCACCACTTCAAGAACTCTGTAGCACCGCCTACATACCTCGCTCTGCTAATCCTG
TTACCAGTGGCTGCTGCCAGTGGCGATAAGTCGTGTCTTACCGGGTTGGACTCAAGAC
GATAGTTACCGGATAAGGCGCAGCGGTCGGGCTGAACGGGGGGTTCGTGCACACAGC
CCAGCTTGGAGCGAACGACCTACACCGAACTGAGATACCTACAGCGTGAGCTATGAGA
AAGCGCCACGCTTCCCGAAGGGAGAAAGGCGGACAGGTATCCGGTAAGCGGCAGGGT
CGGAACAGGAGAGCGCACGAGGGAGCTTCCAGGGGGAAACGCCTGGTATCTTTATAGT
CCTGTCGGGTTTCGCCACCTCTGACTTGAGCGTCGATTTTTGTGATGCTCGTCAGGGGG
GCGGAGCCTATGGAAAAACGCCAGCAACGCGGCCTTTTTACGGTTCCTGGCCTTTTGCT
GGCCTTTTGCTCACATGTTCTTTCCTGCGTTATCCCCTGATTCTGTGGATAACCGTATTA

CCGCCTTTGAGTGAGCTGATACCGCTCGCCGCAGCCGAACGACCGAGCGCAGCGAGT
CAGTGAGCGAGGAAGCGGAAGAGCGCCCAATACGCAAACCGCCTCTCCCCGCGCGTT
GGCCGATTCATTAATGCAGCTGGCACGACAGGTTTCCCGACTGGAAAGCGGGCAGTGA
GCGCAACGCAATTAATGTGAGTTAGCTCACTCATTAGGCACCCCAGGCTTTACACTTTAT
GCTTCCGGCTCGTATGTTGTGTGGAATTGTGAGCGGATAACAATTTCACACAGGAAACA
GCTATGACCATGATTACGCCAAGCGCGCAATTAACCCTCACTAAAGGGAACAAAAGCTG
GAGCTGCAAGCTTGGCCATTGCATACGTTGTATCCATATCATAATATGTACATTTATATT
GGCTCATGTCCAACATTACCGCCATGTTGACATTGATTATTGACTAGTTATTAATAGTAAT
CAATTACGGGGTCATTAGTTCATAGCCCATATATGGAGTTCCGCGTTACATAACTTACGG
TAAATGGCCCGCCTGGCTGACCGCCCAACGACCCCCGCCCATTGACGTCAATAATGAC
GTATGTTCCCATAGTAACGCCAATAGGGACTTTCCATTGACGTCAATGGGTGGAGTATTT
ACGGTAAACTGCCCACTTGGCAGTACATCAAGTGTATCATATGCCAAGTACGCCCCCTA
TTGACGTCAATGACGGTAAATGGCCCGCCTGGCATTATGCCCAGTACATGACCTTATGG
GACTTTCCTACTTGGCAGTACATCTACGTATTAGTCATCGCTATTACCATGGTGATGCGG
TTTTGGCAGTACATCAATGGGCGTGGATAGCGGTTTGACTCACGGGGATTTCCAAGTCT
CCACCCCATTGACGTCAATGGGAGTTTGTTTTGGCACCAAAATCAACGGGACTTTCCAA
AATGTCGTAACAACTCCGCCCCATTGACGCAAATGGGCGGTAGGCGTGTACGGTGGGA
GGTCTATATAAGCAGAGCTCGTTTAGTGAACCGGGGTCTCTCTGGTTAGACCAGATCTG
AGCCTGGGAGCTCTCTGGCTAACTAGGGAACCCACTGCTTAAGCCTCAATAAAGCTTGC
CTTGAGTGCTTCAAGTAGTGTGTGCCCGTCTGTTGTGTGACTCTGGTAACTAGAGATCC
CTCAGACCCTTTTAGTCAGTGTGGAAAATCTCTAGCAGTGGCGCCCGAACAGGGACTTG
AAAGCGAAAGGGAAACCAGAGGAGCTCTCTCGACGCAGGACTCGGCTTGCTGAAGCGC
GCACGGCAAGAGGCGAGGGGCGGCGACTGGTGAGTACGCCAAAAATTTTGACTAGCG
GAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAA
CATATAGTATGGGCAAGCAGGGAGCTAGAACGATTCGCAGTTAATCCTGGCCTGTTAGA
AACATCAGAAGGCTGTAGACAAATACTGGGACAGCTACAACCATCCCTTCAGACAGGAT
CAGAAGAACTTAGATCATTATATAATACAGTAGCAACCCTCTATTGTGTGCATCAAAGGA
TAGAGATAAAAGACACCAAGGAAGCTTTAGACAAGATAGAGGAAGAGCAAAACAAAAGT
AAGACCACCGCACAGCAAGCGGCCGCTGATCTTCAGACCTGGAGGAGGAGATATGAGG
GACAATTGGAGAAGTGAATTATATAAATATAAAGTAGTAAAAATTGAACCATTAGGAGTA
GCACCCACCAAGGCAAAGAGAAGAGTGGTGCAGAGAGAAAAAAGAGCAGTGGGAATAG
GAGCTTTGTTCCTTGGGTTCTTGGGAGCAGCAGGAAGCACTATGGGCGCAGCGTCAAT
GACGCTGACGGTACAGGCCAGACAATTATTGTCTGGTATAGTGCAGCAGCAGAACAATT
TGCTGAGGGCTATTGAGGCGCAACAGCATCTGTTGCAACTCACAGTCTGGGGCATCAA
GCAGCTCCAGGCAAGAATCCTGGCTGTGGAAAGATACCTAAAGGATCAACAGCTCCTG
GGGATTTGGGGTTGCTCTGGAAAACTCATTTGCACCACTGCTGTGCCTTGGAATGCTAG

TTGGAGTAATAAATCTCTGGAACAGATTTGGAATCACACGACCTGGATGGAGTGGGACA

GAGAAATTAACAATTACACAAGCTTAATACACTCCTTAATTGAAGAATCGCAAAACCAGC

AAGAAAAGAATGAACAAGAATTATTGGAATTAGATAAATGGGCAAGTTTGTGGAATTGGT

TTAACATAACAAATTGGCTGTGGTATATAAAATTATTCATAATGATAGTAGGAGGCTTGGT

AGGTTTAAGAATAGTTTTTGCTGTACTTTCTATAGTGAATAGAGTTAGGCAGGGATATTC

ACCATTATCGTTTCAGACCCACCTCCCAACCCCGAGGGGACCCGACAGGCCCGAAGGA

ATAGAAGAAGAAGGTGGAGAGAGAGACAGAGACAGATCCATTCGATTAGTGAACGGAT

CTCGACGGTATCGGTTAACTTTTAAAAGAAAAGGGGGGGATTGGGGGGTACAGTGCAGG

GGAAAGAATAGTAGACATAATAGCAACAGACATACAAACTAAAGAATTACAAAAACAAAT

TACAAAAATTCAAAATTTTATCGATCACGAGACTAGCCTCGAGAAGCTTGATATCGAATT

CCACGGGGGTTGGGGTTGCGCCTTTTCCAAGGCAGCCCTGGGTTTGCGCAGGGACGCG

GCTGCTCTGGGCGTGGTTCCGGGAAACGCAGCGGCGCCGACCCTGGGTCTCGCACAT

TCTTCACGTCCGTTCGCAGCGTCACCCGGATCTTCGCCGCTACCCTTGTGGGCCCCCC

GGCGACGCTTCCTGCTCCGCCCCTAAGTCGGGAAGGTTCCTTGCGGTTCGCGGCGTG

CCGGACGTGACAAACGGAAGCCGCACGTCTCACTAGTACCCTCGCAGACGGACAGCG

CCAGGGAGCAATGGCAGCGCGCCGACCGCGATGGGCTGTGGCCAATAGCGGCTGCTC

AGCAGGGCGCGCCGAGAGCAGCGGCCGGGAAGGGGCGGTGCGGGAGGCGGGGTGT

GGGGCGGTAGTGTGGGCCCTGTTCCTGCCCGCGCGGTGTTCCGCATTCTGCAAGCCT

CCGGAGCGCACGTCGGCAGTCGGCTCCCTCGTTGACCGAATCACCGACCTCTCTCCCC

AGGGGGATCCGTCGACACACCGGTATGCCCGGCGTGACCGTGAAGGACGTGAACCAG

CAGGAGTTCGTGAGGGCCCTGGCAGCCTTTCTGAAGAAGAGCGGCAAGCTGAAGGTG

CCCGAGTGGGTGGACACAGTGAAGCTGGCCAAGCACAAGGAGCTGGCCCCTTACGAT

GAGAACTGGTTCTATACCAGGGCAGCCTCCACAGCAAGGCACCTGTACCTGAGGGGAG

GAGCAGGAGTGGGCTCTATGACCAAGATCTATGGCGGCAGGCAGCGCAATGGCGTGAT

GCCATCTCACTTTAGCCGGGGCAGCAAGTCCGTGGCAAGGAGAGTGCTGCAGGCCCT

GGAGGGCCTGAAGATGGTGGAGAAGGACCAGGATGGCGGCAGAAAGCTGACACCACA

GGGACAGCGGGACCTGGATAGAATCGCAGGACAGGTGGCAGCAGCCAATAAGAAGCA

CTGATAGGATCCCATCTAGAGGATCCCCCGGGCTGCAGGAATTCGAGCATCTTACCGC

CATTTATTCCCATATTTGTTCTGTTTTTCTTGATTTGGGTATACATTTAAATGTTAATAAAA

CAAAATGGTGGGGCAATCATTTACATTTTTAGGGATATGTAATTACTAGTTCAGGTGTAT

TGCCACAAGACAAACATGTTAAGAAACTTTCCCGTTATTTACGCTCTGTTCCTGTTAATC

AACCTCTGGATTACAAAATTTGTGAAAGATTGACTGATATTCTTAACTATGTTGCTCCTTT

TACGCTGTGTGGATATGCTGCTTTAATGCCTCTGTATCATGCTATTGCTTCCCGTACGGC

TTTCGTTTTCTCCTCCTTGTATAAATCCTGGTTGCTGTCTCTTTATGAGGAGTTGTGGCC

CGTTGTCCGTCAACGTGGCGTGGTGTGCTCTGTGTTTGCTGACGCAACCCCCACTGGC

TGGGGCATTGCCACCACCTGTCAACTCCTTTCTGGGACTTTCGCTTTCCCCCTCCCGAT

CGCCACGGCAGAACTCATCGCCGCCTGCCTTGCCCGCTGCTGGACAGGGGCTAGGTT

GCTGGGCACTGATAATTCCGTGGTGTTGTCGGGGAAGGGCCTGCTGCCGGCTCTGCG
GCCTCTTCCGCGTCTTCGCCTTCGCCCTCAGACGAGTCGGATCTCCCTTTGGGCCGCC
TCCCCGCCTGGAATTCGAGCTCGGTACCTTTAAGACCAATGACTTACAAGGCAGCTGTA
GATCTTAGCCACTTTTTAAAAGAAAAGGGGGGACTGGAAGGGCTAATTCACTCCCAACG
AAGACAAGATCTGCTTTTTGCTTGTACTGGGTCTCTCTGGTTAGACCAGATCTGAGCCT
GGGAGCTCTCTGGCTAACTAGGGAACCCACTGCTTAAGCCTCAATAAAGCTTGCCTTGA
GTGCTTCAAGTAGTGTGTGCCCGTCTGTTGTGTGACTCTGGTAACTAGAGATCCCTCAG
ACCCTTTTAGTCAGTGTGGAAAATCTCTAGCAGTAGTAGTTCATGTCATCTTATTATTCAG
TATTTATAACTTGCAAAGAAATGAATATCAGAGAGTGAGAGGAACTTGTTTATTGCAGCT
TATAATGGTTACAAATAAAGCAATAGCATCACAAATTTCACAAATAAAGCATTTTTTTCAC
TGCATTCTAGTTGTGGTTTGTCCAAACTCATCAATGTATCTTATCATGTCTGGCTCTAGCT
ATCCCGCCCCTAACTCCGCCCATCCCGCCCCTAACTCCGCCCATCCCGCCCCTAACTC
CGCCCAGTTCCGCCCATTCTCCGCCCCATGGCTGACTAATTTTTTTTATTTATGCAGAGG
CCGAGGCCGCCTCGGCCTCTGAGCTATTCCAGAAGTAGTGAGGAGGCTTTTTTGGAGG
CCTAGGGACGTACCCAATTCGCCCTATAGTGAGTCGTATTACGCGCGCTCACTGGCCG
TCGTTTTACAACGTCGTGACTGGGAAAACCCTGGCGTTACCCAACTTAATCGCCTTGCA
GCACATCCCCCTTTCGCCAGCTGGCGTAATAGCGAAGAGGCCCGCACCGATCGCCCTT
CCCAACAGTTGCGCAGCCTGAATGGCGAATGGGACGCGCCCTGTAGCGGCGCATTAAG
CGCGGCGGGTGTGGTGGTTACGCGCAGCGTGACCGCTACACTTGCCAGCGCCCTAGC
GCCCGCTCCTTTCGCTTTCTTCCCTTCCTTTCTCGCCACGTTCGCCGGCTTTCCCCGTC
AAGCTCTAAATCGGGGGCTCCCTTTAGGGTTCCGATTTAGTGCTTTACGGCACCTCGAC
CCCAAAAAACTTGATTAGGGTGATGGTTCACGTAGTGGGCCATCGCCCTGATAGACGGT
TTTTCGCCCTTTGACGTTGGAGTCCACGTTCTTTAATAGTGGACTCTTGTTCCAAACTGG
AACAACACTCAACCCTATCTCGGTCTATTCTTTTGATTTATAAGGGATTTTGCCGATTTCG
GCCTATTGGTTAAAAAATGAGCTGATTTAACAAAAATTTAACGCGAATTTTAACAAAATCG
TTCCCTCAGGACGTC

## B) *EF1α(s).CoRPS19.Wpre\*- LV sequence (SEQ ID NO: 18)*

GTGTTTAAACCTAGATATTGATAGTCTGATCGGTCAACGTATAATCGAGTCCTAGCTTTT
GCAAACATCTATCAAGAGACAGGATCAGCAGGAGGCTTTCGCATGATTGAACAAGATGG
ATTGCACGCAGGTTCTCCGGCGGCTTGGGTGGAGAGGCTATTCGGCTATGACTGGGCA
CAACAGACAATCGGCTGCTCTGATGCCGCCGTGTTCCGGCTGTCAGCGCAGGGGCGTC
CGGTTCTTTTTGTCAAGACCGACCTGTCCGGTGCCCTGAATGAACTGCAAGACGAGGCA
GCGCGGCTATCGTGGCTGGCGACGACGGGCGTTCCTTGCGCGGCTGTGCTCGACGTT
GTCACTGAAGCGGGAAGGGACTGGCTGCTATTGGGCGAAGTGCCGGGGCAGGATCTC
CTGTCATCTCACCTTGCTCCTGCCGAGAAGTATCCATCATGGCTGATGCAATGCGGCG

GCTGCATACGCTTGATCCGGCTACCTGCCCATTCGACCACCAAGCGAAACATCGCATC
GAGCGAGCACGTACTCGGATGGAAGCCGGTCTTGTCGATCAGGATGATCTGGACGAAG
AGCATCAGGGGCTCGCGCCAGCCGAACTGTTCGCCAGGCTCAAGGCGTCTATGCCCG
ACGGCGAGGATCTCGTCGTGACCCACGGCGATGCCTGCTTGCCGAATATCATGGTGGA
AAATGGCCGCTTTTCTGGATTCATCGACTGTGGCCGTCTGGGTGTGGCGGACCGCTAT
CAGGACATAGCGTTGGCTACCCGTGATATTGCTGAAGAGCTTGGCGGCGAATGGGCTG
ACCGCTTCCTTGTGCTTTACGGTATCGCCGCGCCCGATTCGCAGCGCATCGCCTTCTAT
CGCCTTCTTGACGAGTTCTTCTGACCGATTCTAGGTGCATTGGCGCAGAAAAAATGCC
TGATGCGACGCTGCGCGTCTTATACTCCCACATATGCCAGATTCAGCAACGGATACGGC
TTCCCCAACTTGCCCACTTCCATACGTGTCCTCCTTACCAGAAATTTATCCTTAACGATC
GGACGGGGGAGTCAGGCAACTATGGATGAACGAAATAGACAGATCGCTGAGATAGGTGC
CTCACTGATTAAGCATTGGTAACTGTCAGACCAAGTTTACTCATATATACTTTAGATTGAT
TTAAAACTTCATTTTTAATTTAAAAGGATCTAGGTGAAGATCCTTTTTGATAATCTCATGA
CCAAAATCCCTTAACGTGAGTTTTCGTTCCACTGAGCGTCAGACCCCGTAGAAAAGATC
AAAGGATCTTCTTGAGATCCTTTTTTTCTGCGCGTAATCTGCTGCTTGCAAACAAAAAA
CCACCGCTACCAGCGGTGGTTTGTTTGCCGGATCAAGAGCTACCAACTCTTTTTCCGAA
GGTAACTGGCTTCAGCAGAGCGCAGATACCAAATACTGTCCTTCTAGTGTAGCCGTAGT
TAGGCCACCACTTCAAGAACTCTGTAGCACCGCCTACATACCTCGCTCTGCTAATCCTG
TTACCAGTGGCTGCTGCCAGTGGCGATAAGTCGTGTCTTACCGGGTTGGACTCAAGAC
GATAGTTACCGGATAAGGCGCAGCGGTCGGGCTGAACGGGGGGTTCGTGCACACAGC
CCAGCTTGGAGCGAACGACCTACACCGAACTGAGATACCTACAGCGTGAGCTATGAGA
AAGCGCCACGCTTCCCGAAGGGAGAAAGGCGGACAGGTATCCGGTAAGCGGCAGGGT
CGGAACAGGAGAGCGCACGAGGGAGCTTCCAGGGGGAAACGCCTGGTATCTTTATAGT
CCTGTCGGGTTTCGCCACCTCTGACTTGAGCGTCGATTTTTGTGATGCTCGTCAGGGGG
GCGGAGCCTATGGAAAAACGCCAGCAACGCGGCCTTTTTACGGTTCCTGGCCTTTTGCT
GGCCTTTTGCTCACATGTTCTTTCCTGCGTTATCCCCTGATTCTGTGGATAACCGTATTA
CCGCCTTTGAGTGAGCTGATACCGCTCGCCGCAGCCGAACGACCGAGCGCAGCGAGT
CAGTGAGCGAGGAAGCGGAAGAGCGCCCAATACGCAAACCGCCTCTCCCCGCGCGTT
GGCCGATTCATTAATGCAGCTGGCACGACAGGTTTCCCGACTGGAAAGCGGGCAGTGA
GCGCAACGCAATTAATGTGAGTTAGCTCACTCATTAGGCACCCCAGGCTTTACACTTTAT
GCTTCCGGCTCGTATGTTGTGTGGAATTGTGAGCGGATAACAATTTCACACAGGAAACA
GCTATGACCATGATTACGCCAAGCGCGCAATTAACCCTCACTAAAGGGAACAAAAGCTG
GAGCTGCAAGCTTGGCCATTGCATACGTTGTATCCATATCATAATATGTACATTTATATT
GGCTCATGTCCAACATTACCGCCATGTTGACATTGATTATTGACTAGTTATTAATAGTAAT
CAATTACGGGGTCATTAGTTCATAGCCCATATATGGAGTTCCGCGTTACATAACTTACGG
TAAATGGCCCGCCTGGCTGACCGCCCAACGACCCCCGCCCATTGACGTCAATAATGAC
GTATGTTCCCATAGTAACGCCAATAGGGACTTTCCATTGACGTCAATGGGTGGAGTATTT

ACGGTAAACTGCCCACTTGGCAGTACATCAAGTGTATCATATGCCAAGTACGCCCCCTA
TTGACGTCAATGACGGTAAATGGCCCGCCTGGCATTATGCCCAGTACATGACCTTATGG
GACTTTCCTACTTGGCAGTACATCTACGTATTAGTCATCGCTATTACCATGGTGATGCGG
TTTTGGCAGTACATCAATGGGCGTGGATAGCGGTTTGACTCACGGGGATTTCCAAGTCT
CCACCCCATTGACGTCAATGGGAGTTTGTTTTGGCACCAAAATCAACGGGACTTTCCAA
AATGTCGTAACAACTCCGCCCCATTGACGCAAATGGGCGGTAGGCGTGTACGGTGGGA
GGTCTATATAAGCAGAGCTCGTTTAGTGAACCGGGGTCTCTCTGGTTAGACCAGATCTG
AGCCTGGGAGCTCTCTGGCTAACTAGGGAACCCACTGCTTAAGCCTCAATAAAGCTTGC
CTTGAGTGCTTCAAGTAGTGTGTGCCCGTCTGTTGTGTGACTCTGGTAACTAGAGATCC
CTCAGACCCTTTTAGTCAGTGTGGAAAATCTCTAGCAGTGGCGCCCGAACAGGGACTTG
AAAGCGAAAGGGAAACCAGAGGAGCTCTCTCGACGCAGGACTCGGCTTGCTGAAGCGC
GCACGGCAAGAGGCGAGGGGCGGCGACTGGTGAGTACGCCAAAAATTTTGACTAGCG
GAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAA
CATATAGTATGGGCAAGCAGGGAGCTAGAACGATTCGCAGTTAATCCTGGCCTGTTAGA
AACATCAGAAGGCTGTAGACAAATACTGGGACAGCTACAACCATCCCTTCAGACAGGAT
CAGAAGAACTTAGATCATTATATAATACAGTAGCAACCCTCTATTGTGTGCATCAAAGGA
TAGAGATAAAAGACACCAAGGAAGCTTTAGACAAGATAGAGGAAGAGCAAAACAAAAGT
AAGACCACCGCACAGCAAGCGGCCGCTGATCTTCAGACCTGGAGGAGGAGATATGAGG
GACAATTGGAGAAGTGAATTATATAAATATAAAGTAGTAAAAATTGAACCATTAGGAGTA
GCACCCACCAAGGCAAAGAGAAGAGTGGTGCAGAGAGAAAAAAGAGCAGTGGGAATAG
GAGCTTTGTTCCTTGGGTTCTTGGGAGCAGCAGGAAGCACTATGGGCGCAGCGTCAAT
GACGCTGACGGTACAGGCCAGACAATTATTGTCTGGTATAGTGCAGCAGCAGAACAATT
TGCTGAGGGCTATTGAGGCGCAACAGCATCTGTTGCAACTCACAGTCTGGGGCATCAA
GCAGCTCCAGGCAAGAATCCTGGCTGTGGAAAGATACCTAAAGGATCAACAGCTCCTG
GGGATTTGGGGTTGCTCTGGAAAACTCATTTGCACCACTGCTGTGCCTTGGAATGCTAG
TTGGAGTAATAAATCTCTGGAACAGATTTGGAATCACACGACCTGGATGGAGTGGGACA
GAGAAATTAACAATTACACAAGCTTAATACACTCCTTAATTGAAGAATCGCAAAACCAGC
AAGAAAAGAATGAACAAGAATTATTGGAATTAGATAAATGGGCAAGTTTGTGGAATTGGT
TTAACATAACAAATTGGCTGTGGTATATAAAATTATTCATAATGATAGTAGGAGGCTTGGT
AGGTTTAAGAATAGTTTTTGCTGTACTTTCTATAGTGAATAGAGTTAGGCAGGGATATTC
ACCATTATCGTTTCAGACCCACCTCCCAACCCCGAGGGGACCCGACAGGCCCGAAGGA
ATAGAAGAAGAAGGTGGAGAGAGAGACAGAGACAGATCCATTCGATTAGTGAACGGAT
CTCGACGGTATCGGTTAACTTTTAAAAGAAAAGGGGGGATTGGGGGGTACAGTGCAGG
GGAAAGAATAGTAGACATAATAGCAACAGACATACAAACTAAAGAATTACAAAAACAAAT
TACAAAAATTCAAAATTTTATCGATCACGAGACTAGCCTCGAGAAGCTTGATATCATGGC
TCCGGTGCCCGTCAGTGGGCAGAGCGCACATCGCCCACAGTCCCCGAGAAGTTGGGG

GGAGGGGGTCGGCAATTGAACCGGTGCCTAGAGAAGGTGGCGCGGGGTAAACTGGGAA
AGTGATGTCGTGTACTGGCTCCGCCTTTTTCCCGAGGGTGGGGGAGAACCGTATATAA
GTGCAGTAGTCGCCGTGAACGTTCTTTTTCGCAACGGGTTTGCCGCCAGAACACAGGT
GTCGTGACGCTCGTCGACACACCGGTATGCCCGGCGTGACCGTGAAGGACGTGAACC
AGCAGGAGTTCGTGAGGGCCCTGGCAGCCTTTCTGAAGAAGAGCGGCAAGCTGAAGGT
GCCCGAGTGGGTGGACACAGTGAAGCTGGCCAAGCACAAGGAGCTGGCCCCTTACGA
TGAGAACTGGTTCTATACCAGGGCAGCCTCCACAGCAAGGCACCTGTACCTGAGGGGA
GGAGCAGGAGTGGGCTCTATGACCAAGATCTATGGCGGCAGGCAGCGCAATGGCGTG
ATGCCATCTCACTTTAGCCGGGGCAGCAAGTCCGTGGCAAGGAGAGTGCTGCAGGCCC
TGGAGGGCCTGAAGATGGTGGAGAAGGACCAGGATGGCGGCAGAAAGCTGACACCAC
AGGGACAGCGGGACCTGGATAGAATCGCAGGACAGGTGGCAGCAGCCAATAAGAAGC
ACTGATAGGATCCCATCTAGAGGATCCCCGGGCTGCAGGAATTCGAGCATCTTACCG
CCATTTATTCCCATATTTGTTCTGTTTTTCTTGATTTGGGTATACATTTAAATGTTAATAAA
ACAAAATGGTGGGGCAATCATTTACATTTTTAGGGATATGTAATTACTAGTTCAGGTGTA
TTGCCACAAGACAAACATGTTAAGAAACTTTCCCGTTATTTACGCTCTGTTCCTGTTAATC
AACCTCTGGATTACAAAATTTGTGAAAGATTGACTGATATTCTTAACTATGTTGCTCCTTT
TACGCTGTGTGGATATGCTGCTTTAATGCCTCTGTATCATGCTATTGCTTCCCGTACGGC
TTTCGTTTTCTCCTCCTTGTATAAATCCTGGTTGCTGTCTCTTTATGAGGAGTTGTGGCC
CGTTGTCCGTCAACGTGGCGTGGTGTGCTCTGTGTTTGCTGACGCAACCCCCACTGGC
TGGGGCATTGCCACCACCTGTCAACTCCTTTCTGGGACTTTCGCTTTCCCCCTCCCGAT
CGCCACGGCAGAACTCATCGCCGCCTGCCTTGCCCGCTGCTGGACAGGGGCTAGGTT
GCTGGGCACTGATAATTCCGTGGTGTTGTCGGGGAAGGGCCTGCTGCCGGCTCTGCG
GCCTCTTCCGCGTCTTCGCCTTCGCCCTCAGACGAGTCGGATCTCCCTTTGGGCCGCC
TCCCCGCCTGGAATTCGAGCTCGGTACCTTTAAGACCAATGACTTACAAGGCAGCTGTA
GATCTTAGCCACTTTTTAAAAGAAAAGGGGGGACTGGAAGGGCTAATTCACTCCCAACG
AAGACAAGATCTGCTTTTTGCTTGTACTGGGTCTCTCTGGTTAGACCAGATCTGAGCCT
GGGAGCTCTCTGGCTAACTAGGGAACCCACTGCTTAAGCCTCAATAAAGCTTGCCTTGA
GTGCTTCAAGTAGTGTGTGCCCGTCTGTTGTGTGACTCTGGTAACTAGAGATCCCTCAG
ACCCTTTTAGTCAGTGTGGAAAATCTCTAGCAGTAGTAGTTCATGTCATCTTATTATTCAG
TATTTATAACTTGCAAAGAAATGAATATCAGAGAGTGAGAGGAACTTGTTTATTGCAGCT
TATAATGGTTACAAATAAAGCAATAGCATCACAAATTTCACAAATAAAGCATTTTTTTCAC
TGCATTCTAGTTGTGGTTTGTCCAAACTCATCAATGTATCTTATCATGTCTGGCTCTAGCT
ATCCCGCCCCTAACTCCGCCCATCCCGCCCCTAACTCCGCCCATCCCGCCCCTAACTC
CGCCCAGTTCCGCCCATTCTCCGCCCCATGGCTGACTAATTTTTTTTATTTATGCAGAGG
CCGAGGCCGCCTCGGCCTCTGAGCTATTCCAGAAGTAGTGAGGAGGCTTTTTTGGAGG
CCTAGGGACGTACCCAATTCGCCCTATAGTGAGTCGTATTACGCGCGCTCACTGGCCG
TCGTTTTACAACGTCGTGACTGGGAAAACCCTGGCGTTACCCAACTTAATCGCCTTGCA

GCACATCCCCCTTTCGCCAGCTGGCGTAATAGCGAAGAGGCCCGCACCGATCGCCCTT
CCCAACAGTTGCGCAGCCTGAATGGCGAATGGGACGCGCCCTGTAGCGGCGCATTAAG
CGCGGCGGGTGTGGTGGTTACGCGCAGCGTGACCGCTACACTTGCCAGCGCCCTAGC
GCCCGCTCCTTTCGCTTTCTTCCCTTCCTTTCTCGCCACGTTCGCCGGCTTTCCCCGTC
AAGCTCTAAATCGGGGGCTCCCTTTAGGGTTCCGATTTAGTGCTTTACGGCACCTCGAC
CCCAAAAAACTTGATTAGGGTGATGGTTCACGTAGTGGGCCATCGCCCTGATAGACGGT
TTTTCGCCCTTTGACGTTGGAGTCCACGTTCTTTAATAGTGGACTCTTGTTCCAAACTGG
AACAACACTCAACCCTATCTCGGTCTATTCTTTTGATTTATAAGGGATTTTGCCGATTTCG
GCCTATTGGTTAAAAAATGAGCTGATTTAACAAAAATTTAACGCGAATTTTAACAAAATCG
TTCCCTCAGGACGTC

### 2. Lentiviruses production

Lentiviruses were produced using a third generation set up with HEK293T line as packaging cells. Productions were carried out in 150 mm dishes (p150, Corning) with 60-70% of cell confluence transferring the different plasmids required by CaCl2 DNA precipitation method.

Plasmids used were kindly provided by Dr. L. Naldini (HSR-TIGET, Milan, Italy) and produced by Plasmid Factory. Plasmids used were: transfer plasmid (transgene of interest; 37 pg/p150), rev plasmid (pRSV-REV; 9 $\mu$g/p150), gag and pol plasmid (pMDLg/pRRE; 23.5 $\mu$g/p150) and VSV envelope plasmid (pMD2-VSVG; 12.3 $\mu$g/p150). Plasmids were mixed with 457$\mu$l/p150 of CaCl2 2.5 M and 3.2ml/p150 of water. Afterwards, 3.6 ml/p150 of HBS 2x (281 mM NaCl, 100 mM HEPES, 1.5 mM Na2HPO4, pH 7) were added dropwise to the mix to generate Ca2+/DNA-precipitates and this solution was added to the HEK293T cell cultures.

After 5-6 hours, medium was removed and fresh collecting medium was added (IMDM supplemented with 5% HyClone). Supernatant was harvested 48 hours post-transfection, centrifuged (450 g, 7 minutes) and filtered (0.22 $\mu$m, PES, Merck) to eliminate cellular rests. Supernatant was concentrated by ultracentrifugation (70000g, 2 hours, 20°C; Optima L-100 XP Ultracentrifuge, Beckman-Coulter) using Ultra-clear tubes (Beckman-Coulter). Viral pellet was reconstituted on 100-300 $\mu$L saline solution (NaCl 0.9%). Viruses were titered by serial dilution of the vector (from 10-2 to 10-6) on HEK293T cells (150-180,000 cells/well on multiple well plate size 24; FALCON). In the case of GFP labeled vectors, transducing units (TU) were calculated based on flow cytometry measures 48-72 hours after transduction applying the following formula.

### 3. Lentiviral vector titration

PGK-CoRPS19 LV titration was performed in HEK293T. For LV titration, $5 \times 10^4$ cells were seeded in 24 well plates. Twenty-four hours after the seed, cells were transduced with serial dilutions of the LV in culture media and the number of cells at day 0 was determined. 14 days after transduction, cells were collected, and genomic DNA (gDNA) was extracted using NucleoSpin® Tissue Kit (Macherey Nagel®) following manufacturer's instructions.

The number of lentiviral vector genomes (VCN) integrated into transduced cells was determined by qPCR. To analyse the copies of the lentiviral vector genome we used primers ((SEQ ID NO: 19) Fw: 5'-CAGGACTCGGCTTGCTGAAG-3'; (SEQ ID NO: 20) Rv: 5'-TCCCCCGCTTAATACTGACG-3') and a probe ((SEQ ID NO: 21) 5'-CGCACGGCAAGAGGCGAGG-3') (Taqman®, Thermo Fisher Scientific) designed on the $\psi$ sequence of the lentiviral vector. To determine the amount of endogenous DNA we used primers ((SEQ ID NO: 22) Fw: 5'-GCTGTCATCTCTTGTGGGCTG-3'; (SEQ ID NO: 23) Rv: 5'-ACTCATGGGAGCTGCTGGTTC-3') and a probe ((SEQ ID NO: 24) 5'-CCTGTCATGCCCACACAAATCTCTCC-3') (Taqman®, Thermo Fisher Scientific) against the human albumin gene. We also used a standard curve to determine the number of copies of $\psi$ LV specific sequence and the number of diploid genomes per sample, which allows us to calculate the number of LV copies per cell (**VCN = number of copies of $\psi$/number of diploid genomes**). Finally, based on this parameter we calculated LV title, defined as the number of transducing units per millilitre, with the following formula:

$$Title \; (UT/mL) = \frac{number \; of \; cells \; at \; day \; 0 \cdot VCN \; per \; diploid \; genome}{LV \; volume \; (mL)}$$

Standard curve to extrapolate qPCR data was performed using serial dilutions of a DNA fragment which contains ψ and human albumin sequence. All reactions were carried out in duplicate in 7500 Fast Real-Time PCR System (Applied Biosystems®).

### 4. Experimental animals

Human CD34$^+$ cells were transplanted in non-obese diabetic (NOD) immunodeficient Cg-Prkdcscid Il2rgtm1Wjl/SzJ mice (NSG). This strain carries two mutations on the NOD/ShiLtJ genetic background: one causing a severe combined immune deficiency (scid) and another inducing a complete absence of the IL2 receptor common gamma chain (IL2rgnull). The scid mutation takes place in the *Prkdc* gene, encoding for a DNA repair complex and originates a deficiency in B and T cells. The IL2rgnull mutation prevents cytokine signaling through multiple receptors, causing a NK-cell function deficiency. The severe immunodeficiency allows the mice to be humanized by engraftment of human CD34$^+$ cells, patient derived xenografts, or adult stem cells and tissues.

All experimental procedures were conducted according to the European and Spanish regulations in the field: European convention ETS 123, regarding the use and protection of vertebrate mammals used in experimentation and other scientific purposes, Directive 2010/63/UE and Spanish Law 6/2013 and Real Decreto (R.D.) 53/2013 regarding the protection and use of animals in scientific research.

### 5. Animal procedures

Procedures involving Genetically Modified Organisms were conducted according to the proper European and Spanish regulations: Directive 2009/41/CE and, Spanish Law 9/2003 and R.D. 178/2004. Procedures were approved by the CIEMAT Animal Experimentation Ethical Committee according to all external and internal bio-safety and bio-ethics guidelines, and previously authorized by the Spanish Government (Code PROEX #070-15# Cell and Gene Therapy in rare diseases with chromosomal instability).

Mice were housed and bred at the CIEMAT Laboratory Animals Facility (registration number ES280790000183), where they were routinely screened for pathogens in accordance with the Spanish Society for the Laboratory Animal Science (SECAL) and the Federation of European Laboratory Animal Science Associations (FELASA, Tomworth, United Kingdom) recommendations and no pathogens were found.

Mice were provided with food (TEKLAD Global Diet 2918, irradiated with 25 KGy gamma rays) and water (acidified and autoclaved) ad libitum, under controlled environmental conditions. Mice were housed during the experimental protocols in micro insulators individually ventilated cages type 11L with 25 air cage changes per hour. A maximum of 6 mice were housed in each cage. Room lighting was controlled with light/dark cycles of 13/11 hours, and temperature and humidity were regulated at 20 ± 2°C and 55 ± 10%, respectively. HEPA air filters were present in all rooms.

Mice were maintained under standard diet (water and food *ad libitum*) and all protocols were done following the European and Spanish laws and regulations (ETS123 European convention about the use and protection of vertebrate mammals used in experimentation and other scientific purposes and Spanish law RD 53/2013).

### 6. Human samples from healthy donors and DBA patients

The human samples used in the results presented were peripheral blood and bone marrow, both from healthy donors and from patients with DBA, as well as umbilical cord blood from healthy donors collected under informed consent.

All samples have been used in the context of the project "Preclinical studies to demonstrate the efficacy and the safety of an ex vivo gene therapy approach in Diamond Blackfan Anemia with lentiviral vectors", funded by the CIBERER-ISCIII-Centro de Investigation Biomédica en Red of Rare Diseases and that was approved by the ethics committee of the Jiménez Diaz Foundation on May 8, 2018. This evaluation states that:

- The study meets the requirements established in current legislation (Royal Decree 1090/2015 and Decree 39/94 of the CAM).

- Meets the standard ethical standards of the institution in this type of study.

- The ethical precepts formulated in SAS Order 3470/2009 and the Declaration of Helsinki of the World Medical Association are complied with.

Hematology analyzes from samples from patients with DBA and healthy donors were performed on the Sysmex XN-1000 ™ hematology analyzer (Sysmex, Kobe, Japan) from 120 μl of peripheral blood or bone marrow in pre-dilution mode.

### 7. Determination of VCN in LV injected mice

After animals' sacrifice, different tissues were collected (liver, spleen, lungs, bone marrow, lymph nodes, brain, testicles, pancreas, and kidneys) and preserved at -80°C. For the determination of LV genomes integrated into these tissues, genomic DNA was extracted with the NucleoSpin® Tissue Kit (Macherey Nagel) following the manufacturer's instructions.

The number of integrated lentiviral vector genomes (VCN) in the injected mice tissues was determined by qPCR. To analyse the number of of copies of the integrated provirus we used primers ((SEQ ID NO: 25) Fw: 5' CAGGACTCGGCTTGCTGAAG 3'; (SEQ ID NO: 26) Rv: 5' TCCCCCGCTTAATACTGACG 3') and a probe ((SEQ ID NO: 27) 5'-CGCACGGCAAGAGGCGAGG-3') (Taqman®, Thermo Fisher Scientific) designed on the $\psi$ sequence of the lentiviral vector. To determine the amount of endogenous DNA we used primers ((SEQ ID NO: 28) Fw: 5' AAAACGAGCAGTGACGTGAGC 3'; (SEQ ID NO: 29) Rv: 5' TTCAGTCATGCTGCTAGCGC 3') and a probe ((SEQ ID NO: 30) 5'-TGCACGGAAGCGTCTCGTCTCAGTC-3') against the mouse *titin* gene. Once determined the number of LV copies and the number of diploid genomes per sample, VCN was calculated (**VCN = number of copies of $\psi$/number of diploid genomes**).

A standard curve was performed using serial dilutions of a DNA fragment that contains $\psi$ and the *titin* sequences. All reactions were carried out in duplicate in 7500 Fast Real-Time PCR System (Applied Biosystems®).

### 8. Determination of *CoRPS19* expression

The determination of the expression of the CoRPS19 transgene integrated by the therapeutic vectors, as well as its non-optimized physiological version RPS19, was carried out by Q-PCR. RNA was extracted from cells using the RNeasy Tissue kit (Qiagen GMBH) and the reverse transcription process was carried out using the Superscript Vilo kit (Invitrogen Superscript IV VILO Master Mix, ThermoFisher). Once the cDNA was obtained, expression analysis was performed on a 7500 real-time PCR system (ThermoFisher Scientific). To calculate the expression of the endogenous RPS19 gene, as well as the optimized version CoRPS19, a variation of the relative quantification method of Livak ($2\Delta Ct$) 209 was used. For this, the amplification efficiency of the genes of interest (hRPS19 and CoRPS19) and the reference gene hGAPDH was initially calculated. Once 100% efficiency in amplification has been assumed, the relative expression of the gene of interest is normalized with a reference gene, in this case GAPDH using the formula:

$$\underline{\text{Relative expression of } RPS19} = 2^{(CT\ GAPDH)-(CT\ RPS19)}$$

$$\underline{\text{Relative expression of } CoRPS19} = 2^{(CT\ GAPDH)-(CT\ CoRPS19)}$$

**Table 1.** Sequences of the primers and probes used for the detection of the expression of the *CoRPS19* transgene and its wild version RPS19.

| Primer/Probe | | | Sequence | Tm (ºC) |
|---|---|---|---|---|
| Primers | *hGAPDH* Fw | | **GCTCTCTGCTCCTCCTGTTC** | 60 |
| | *hGAPDH* Rv | | **ACGACCAAATCCGTTGACTC** | 60 |
| | *RPS19* Fw | | **AGCCGAGGCTCCAAGAGT** | 57.3 |
| | *RPS19* Rv | | **CCCTGAGGTGTCAGTTTGC** | 59.25 |
| | CoRPS19 Fw | | **GTGAACCAGCAGGAGTTCGT** | 73.9 |
| | CoRPS19 Rv | | **TCAGCTTGCCGCTCTTCT** | 75.7 |
| Probe | | | *SYBER* Green | |

### 9. Northern blotting and primer extension analysis of pre-rRNA processing.

5 $\mu$g total RNA extracted using the TRI reagent (Invitrogen) was either resolved on a denaturing agarose gel and processed for northern blotting. The northern blots were exposed to Fuji imaging plates (Fujifilm) and quantification was performed on a phosphorimager (FLA-7000; Fujifilm) using the MultiGauge software (Fujifilm, v 3.1).

### 10. Flow cytometry

### 10.1 Hematopoietic characterization of patients with DBA

The immunophenotype of patients with DBA and healthy donors was analyzed by flow cytometry using six different panels specified in table 2. Antibodies were added (shown in table 2), incubated for 30 minutes at 4°C and then washed with PBA (PBS with 0.2% sodium azide, Merck, and 1% BSA, Sigma). For panel 1 and 2, the peripheral blood samples were pre-diluted in PBA at ratios of 1:400 and 1:10, respectively. After antibody incubation, a lysis step with ammonium chloride buffer (0.155 M NH4Cl, 0.01 M KHCO3, 0.1 mM EDTA; 10 min incubation at room temperature) was performed on panels 3, 4, 5 and 6 to remove the erythroid fraction. DAPI (4', 6-diamidino-2-phenylindole) was added to a final concentration of 1 μg/ml to identify dead cells. The events were acquired and recorded with a Fortessa LSR (BD Biosciences) and the cytometry data were analyzed by FlowJo software (BD, Becton, Dickinson & Company).

**Table 2.** Panel of antibodies used for the immunophenotype of patients with DBA and healthy donors.

| Panel | Epitope | Fluorochrome | Clone | Supplier | Catalogue | V$_{final}$ (μL) | V$_{ab}$/V$_{final}$ |
|---|---|---|---|---|---|---|---|
| 1: Erythroid progenitos | CD71 | PE | YDJ. 1.2.2 | Beckman Coulter | IM2001U | 50 | 2.5 |
| | CD235a | FITC | 11E4B-7-6 | Beckman Coulter | B49206 | | 5 |
| 2: Megakaryocytes and platelets | CD41a | PE | B61391 | BD Pharimigen | 555472 | 50 | 5 |
| | CD42b | FITC | 4336556 | Beckman Coulter | A07781 | | 5 |
| 3: Hematopoietic progenitors | CD34 | PE | 8G12 | BD Pharimigen | 345802 | 100 | 4 |
| | CD38 | FITC | T16 | Immunotech | IM0775 | | 4 |
| | CD45 | APC | 2D1 | Biolegend | 368512 | | 5 |
| 4: Lymphoid population | CD3 | APC | UCHT1 | BioLegend | 300439 | 100 | 2 |
| | CD4 | PE | 13B8.2 | Beckman Coulter | A07751 | | 2 |
| | CD8 | FITC | B9.11 | Beckman Coulter | A07756 | | 2 |
| 5: Myeloid population | CD3 | APC | UCHT1 | BioLegend | 300439 | 100 | 2 |
| | CD14 | BV711 | M5E2 | Biolegend | 301837 | | 1 |
| | CD15 | PE | VIMC6 | Miltenyi Biotec | 130-091-375 | | 2 |
| | CD19 | PECy7 | SL25C1 | eBioscience | 25-0198-42 | | 1.5 |

### 10.2 Multistem Analysis

The determination of the different hematopoietic progenitors in bone marrow of DBA patients and healthy donors was carried out by flow cytometry using the combination of antibodies reflected in table 3. The labeling protocol was similar to that used in the determination of the immunophenotyping of samples of patients and healthy donors acquired with a Fortessa RSL (BD Biosciences). The minimum number of cells analyzed were $1 \times 10^6$ for the correct evaluation of the different analysis. The cytometry results were analyzed by FlowJo software (BD, Becton, Dickinson & Company).

Table 3. Panel of antibodies used for the determination of hematopoietic progenitors in bone marrow.

| | | Antibodies | | | | | | | | | | |
|---|---|---|---|---|---|---|---|---|---|---|---|---|
| | TUBE # | CD15S BV421 (h203) | CD49f BV510 (h207) | HECA452 BV605 (h204) | CD135 BV711 (h205) | CD10 FITC (h18) | PI (20µg/ml) | CD38 PECy5.5 (h32) | CD7 PE (h14) | CD34 PECY7 (h45) | CD90 APC (h164) | CD45RA APCe fluor780 (h194) |
| COMPENSATION CONTROLS (200,000 cells negative fraction) | 1: CD155 | 2 µl | --- | ---- | ---- | ---- | --- | ---- | ---- | ---- | ---- | ---- |
| | 2: CD49f | ---- | 2 µl | ---- | ---- | --- | ---- | ---- | ---- | ---- | ---- | ---- |
| | 3: HECA452 | ---- | --- | 2 µl | ---- | ---- | ---- | ---- | ---- | ---- | ---- | ---- |
| | 4; CD135 | ---- | ---- | ---- | 2 µl | ---- | ---- | ---- | ---- | ---- | ---- | ---- |
| | 5: CD10 | ---- | ---- | ---- | ---- | 2 µl | ---- | ---- | ---- | ---- | ---- | ---- |
| | 6: PI | ---- | ---- | ---- | ---- | ---- | PI | ---- | ---- | ---- | ---- | ---- |
| | 7: CD38 | ---- | ---- | ---- | ---- | ---- | ---- | 1.5 µl | ---- | ---- | ---- | ---- |
| | 8: CD7 | ---- | ---- | ---- | ---- | ---- | ---- | ---- | 2 µl | ---- | ---- | ---- |
| | 9: CD34 | ---- | ---- | ---- | ---- | ---- | ---- | ---- | ---- | 1.5 µl | ---- | ---- |
| | 10: CD90 | ---- | ---- | ---- | ---- | ---- | ---- | ---- | ---- | ---- | 1.5 µl | --- |
| | 11: CD45RA | ---- | ---- | ---- | ---- | ---- | ---- | ---- | ---- | ---- | ---- | 1.5 µl |

| | TUBE # | CD15S BV421 (h203) | CD49f BV510 (h207) | HECA452 BV605 (h204) | CD135 BV711 (h205) | CD10 FITC (h18) | PI (20μg/ml) | CD38 PECy5.5 (h32) | CD7 PE (h14) | CD34 PECY7 (h45) | CD90 APC (h164) | CD45RA APCe fluor780 (h194) |
|---|---|---|---|---|---|---|---|---|---|---|---|---|
| | | | | | | | Antibodies | | | | | |
| FMOs (20,000 CD34+ cells or 200,000 cells negative fraction or 200,000 total BM) | 12: CD15S | ---- | 2 μl | 2 μl | 2 μl | 2 μl | PI | 1,5 μl | 2 μl | 1.5 μl | 1.5 μl | 1.5 μl |
| | 13: CD49f | 2 μl | ---- | 2 μl | 2 μl | 2 μl | PI | 1,5 μl | 2 μl | 1.5 μl | 1.5 μl | 1.5 μl |
| | 14: HECA452 | 2 μl | 2 μl | ---- | 2 μl | 2 μl | PI | 1,5 μl | 2 μl | 1.5 μl | 1.5 μl | 1.5 μl |
| | 15: CD135 | 2 μl | 2 μl | 2 μl | ---- | 2 μl | PI | 1,5 μl | 2 μl | 1.5 μl | 1.5 μl | 1.5 μl |
| | 16: CD10 | 2 μl | 2 μl | 2 μl | 2 μl | ---- | PI | 1,5 μl | 2 μl | 1.5 μl | 1.5 μl | 1.5 μl |
| | 17: PI | 2 μl | 2 μl | 2 μl | 2 μl | 2 μl | ---- | 1,5 μl | 2 μl | 1.5 μl | 1.5 μl | 1.5 μl |
| | 18: CD38 | 2 μl | 2 μl | 2 μl | 2 μl | 2 μl | PI | ---- | 2 μl | 1.5 μl | 1.5 μl | 1.5 μl |
| | 19: CD7 | 2 μl | 2 μl | 2 μl | 2 μl | 2 μl | PI | 1,5 μl | --- | 1.5 μl | 1.5 μl | 1.5 μl |
| | 20: CD34 | 2 μl | 2 μl | 2 μl | 2 μl | 2 μl | PI | 1,5 μl | 2 μl | ---- | 1.5 μl | 1.5 μl |
| | 21: CD90 | 2 μl | 2 μl | 2 μl | 2 μl | 2 μl | PI | 1,5 μl | 2 μl | 1.5 μl | ---- | 1.5 μl |
| | 22: CD45RA | 2 μl | 2 μl | 2 μl | 2 μl | 2 μl | PI | 1,5 μl | 2 μl | 1.5 μl | 1.5 μl | --- |
| PURIFIED SAMPLE (50,000 CD34+ cells) | 23A: Sample | 2 μl | 2 μl | 2 μl | 2 μl | 2 μl | PI | 1,5 μl | 2 μl | 1.5 μl | 1.5 μl | 1.5 μl |
| MUESTRA TOTAL (BM, mPB) (≥2*10^6 células totales) | 23B: Sample | 3 μl | 3 μl | 3 μl | 3 μl | 3 μl | PI | 2.5 μl | 3 μl | 2.5 μl | 2.5 μl | 2.5 μl |

EP 4 339 291 A1

38

*10.3 Immunophenotype of erythroid differentiation*

The immunophenotype of hematopoietic progenitors differentiated towards the erythroid lineage was analyzed by flow cytometry using the combination of antibodies specified in Table 4.

| Panel | Epitope | Fluorochrome | Clone | Supplier | Catalogue | V.A./100µl (µL) |
|---|---|---|---|---|---|---|
| 1 | CD36 | PE | CB38(NL 07) | BD Pharmingen | 555455 | 1 |
| | CD45 | APCCy7 | HI30 | BioLegend | 304014 | 1 |
| | CD71 | PECy5 | M-A712 | BD Pharmingen | 551143 | 1 |
| | CD235a | PECy7 | HI264 | BioLegend | 349111 | 1 |

**Table 4.** Panel of antibodies used for the immunophenotype of cells differentiated towards the erythroid lineage.

Cells were diluted to a final volume of 100 µl, labeled for 30 minutes at 4° C, and washed with PBA. DAPI (1 µg / ml final concentration) was added and events were recorded with a Fortessa LSR (BD Biosciences). The results were analyzed by FlowJo software (BD Biosciences).

## 11. Cell line cultures

The K562 cell line (chronic myelogenous leukemia; ATCC: CCL-243) was grown in Iscove's modified Dulbecco's medium (IMDM; Gibco), HyClone (10%; GE Healthcare) and penicillin / streptomycin (1%; Gibco). The cells were maintained at a concentration of $1\times10^5$-$1\times10^6$ cells / ml.

The HEK293T cell line (human embryonic kidney cell line competent to replicate vectors bearing the SV40 T antigen; ATCC: CRL-3219) was grown in Iscove's modified Dulbecco's medium (IMDM; Gibco), HyClone (10%; GE Healthcare) and penicillin / streptomycin (1%; Gibco). The cells were maintained at a concentration of $5\times10^5$ cells / ml.

The incubation conditions were the same for all cell lines used: 37° C, 5% CO2 and 95% relative humidity.

## 12. Hematopoietic progenitor and hematopoietic stem cell functional assays

CD34+ cells were obtained from peripheral blood and bone marrow samples from patients with DBA and healthy donors, for characterization and lentiviral correction studies, and from umbilical cord blood from healthy donors for safety studies. First, either the whole blood or the bone marrow was subjected to a density gradient using Ficoll-Paque PLUS (GE Healthcare) according to the manufacturer's instructions. The mono-nucleated cell band was then collected and washed with PBS (Dulbecco's Phosphate Buffered Saline, Sigma), herein referred to as PBE; Hyclone 2%, GE Healthcare; 2 mM EDTA. Once the mononucleated cell band was obtained, the CD34+ cell fraction was separated using the commercial CD34+ Microbeads kit (MACS, Miltenyi Biotec, Bergisch Gladbach, Germany), together with the columns and the QuadroMACS and OctoMACS magnetic separator (Miltenyi Biotech). Once the CD34+ cells were obtained, flow cytometric analysis was carried out to determine the purity of the sample. For this, an aliquot was collected and labeled with an anti-CD34 PE antibody for 30 minutes at 4°C, washed with PBA and the percentage of CD34+ cells was analyzed with a Fortessa LSR (BD Biosciences) and the cytometry data was analyzed by FlowJo software (BD, Becton, Dickinson & Company).

The cells were cultured in StemSpam medium (StemCell Technologies) or X-VIVO 20 medium (Lonza, Basel, Switzerland) supplemented with 1% GlutaMAX ™ (Gibco), 1% P/S (Gibco), 100 ng/ml hSCF and hFlt3 , 20 ng/ml hTPO and hIL3 (all EuroBioSciences GmbH) under hypoxic conditions (37°C, 5% O2, 5% CO2 and 95% relative humidity).

Human hematopoietic progenitors differentiated towards the erythroid lineage using three different means (Figure 11). The first medium (day 1-7) was based on StemSpan SFEM I (Stem Cell Technologies) supplemented with hSCF (50 ng/ml; EuroBioSciences), hFlt3-ligand (16.7 ng/ml; EuroBioSciences), bone morphogenetic protein 4 (BMP-4, 6.7 ng/ml; Peprotech), human interleukin 3 (hIL-3, 6.7 ng/ml; EuroBioSciences), human interleukin 11 (hIL-11, 6.7 ng/ml; EuroBioSciences) and human erythropoietin (h-EPO, 1.3 U/ml; Amgen). On day 7 of expansion, the cells were transferred to the second medium composed of IMDM (Iscove's modified Dulbecco's medium) supplemented with Glutamine (supplemented with IMDM GlutaMAX; Gibco) and enriched with BSA (1%; Sigma), insulin (0.01 mg/ml; Sigma), human transferrin (0.2 mg/ml; Sigma), β-mercaptoethanol (91 µM; Gibco), penicillin/streptomycin (1%; Gibco), lipid mix 1 (1x; Sigma), ethanolamine (0.004%, Sigma), hSCF (5 ng/ml; EuroBioSciences), hIL-3 (6.7 ng/ml; EuroBio-Sciences), hIL-11 (6.7 ng/ml; EuroBioSciences), hEPO (1, 3 U/ml; Amgen), insulin-as growth factor-1 (IGF-1, 20 ng/ml; PeproTech) and hydrocortisone (1 $\mu$M; Sigma) and were cultured until day 14. After day 14 and for 2 days, the cells were transferred to the third medium which was also based on IMDM GlutaMAX medium (Gibco) supplemented with BSA (1%; Sigma), insulin (0.01 mg/mL; Sigma), transferrin human (0.2 mg/mL; Sigma), $\beta$-mercaptoethanol (91 $\mu$M; Gibco), penicillin/streptomycin (1%; Gibco), lipid mix 1 (1x; Sigma), ethanolamine (0.004%; Sigma) and hEPO (10 U/ml Amgen).

All media were filtered through 0.22 $\mu$m filters before use. Counting and flow cytometric analysis of cells were performed on day 3, 5, 7, 10 and 14. During erythroid differentiation, cells were maintained at a concentration between $4\times10^5$ and $4\times10^6$ cells/ml. The incubation conditions were also 37°C, 5% $CO_2$ and 95% relative humidity.

The number of hematopoietic progenitors in peripheral blood and bone marrow in patients with DBA was evaluated according to the following protocol: mononuclear hematopoietic cells and / or CD34$^+$ cells were resuspended in X-vivo medium and seeded in semi-solid methylcellulose medium (StemMACS ™ HSC-CFU supplemented with 30% fetal calf serum, 1% BSA, 2 mM Glutamine, 2-Mercaptoethanol 0 , 1 nM, SCF 50 ng/ml, GM-CSF 20 ng/ml, G-CSF 20 ng/ml, IL-3 20 ng/ml, IL-6 20 ng/ml, EPO 3 U/ml; Miltenyi) at a concentration of $3\times10^5$ nucleated cells per ml for peripheral blood and $5\times10^4$ nucleated cells / ml for bone marrow in 25 mm2 culture plates. Triplicates were made for each sample using 1 ml for each replica. After 14 days of incubation in hypoxia (37°C, 5% $O_2$ and 5% $CO_2$, 98% relative humidity) the colonies formed were counted using a phase contrast Nikon ELWD 0.3 inverted microscope. The results were expressed as the average of the total number of colonies for every $10^5$ cells seeded.

### 13. Transplantation of human hematopoietic cells in NSG mice

The mononuclear cell fraction was obtained from the bone marrow of DBA patients and healthy donors and separated by density gradient using Ficoll-Paque PLUS (GE Healthcare) according to the manufacturer's instructions. In the case of safety trials, MNCs were obtained from umbilical cord blood using the same process. The CD34$^+$ fraction was obtained by immunomagnetic separation using the commercial CD34$^+$ Microbeads kit (MACS, Miltenyi Biotec, Bergisch Gladbach, Germany), together with the columns and the QuadroMACS and OctoMACS magnetic separator (Miltenyi Biotech). Prior to engraftment, NSG mice were irradiated with a submyeloablative dose (1.5 Gy) and transplanted with $2\times10^5$ mouse purified CD34$^+$ cells. We analyzed the hematopoietic graft in the NSG mice in the peripheral blood and bone marrow at day 30, 60 and 90 and in the case of the safety tests up to day 120 post infusion by flow cytometryTo assess the level of human hematopoietic graft, cells were harvested by femoral bone marrow aspiration at 4, 8 and 12 weeks after transplantation and labeled with the hCD45 APC-Cy7 antibody (eBioscience) according to with the manufacturer's instructions. Multilineage graft analyzes were performed using CD45 APC-Cy7 (BioLegend), CD34 APC (BD Biosciences), CD33 PE (BD Biosciences), CD19 PE-Cy7, and CD3 FITC (BioLegend), according to the manufacturer's instructions. Paired fluorochrome isotypes were used as controls. Positive 4'6-diamidino-2-phenylindole (DAPI) cells were excluded from the analysis. All flow cytometry analyzes were performed on the Fortessa LSR (BD Biosciences) and analyzed with FlowJo v7.6.5 software. To carry out the secondary transplantation, the primary NSG recipients were anesthetized and bled to obtain the hematological results. Once anesthetized, they were sacrificed by cervical dislocation and the femurs and tibiae were removed from the hind legs of each mouse. The bone marrow of the femurs and tibiae were obtained by perfusing each bone. Once the bone marrow cells were obtained, the human CD45 + cells were selected by cell separation by flow cytometry. Once purified, the cells were transplanted back into immunodeficient 1.5 Gy irradiated NSG mice. The analyzes were carried out at 30, 60, 90 and 120 days post transplantation.

### 14. Integration site analysis (ISA)

The analysis of these integrations was carried out both in the cells expanded in vitro for 14 days, as well as the cells grafted in primary and secondary NSG recipients of both experiment. The analyses we conducted by Genewerk. The technique used by Genewerk is based on S-EPTS / LM-PCR (shearing extension first tag selection ligation-mediated PCR), which amplifies and sequences unknown regions that flank the integrated vector DNA. DNA samples are fragmented by sonication into 500 base pair fragments, then amplified by PCR using biotinylated primers specific for the LTR sequences of the integrated vector. After amplification, the biotinylated products are purified, the products are ligated into cassettes that include molecular labels (Bar code) and are subjected to a second step of PCR amplification with primers that allow sequencing using MiSeq technology (Illumina, Thermofisher). For the IS analysis, the 10 most frequent integration sites were used.

### 15. Statistical analysis

Statistical analysis was performed with GraphPad Prism, version 7.0 for Windows. Previous statistical studies were carried out to check if the data followed a normal distribution using the statistical analysis of columns and applying the D'Agostino & Pearson normality test, Shapiro-Wilk normality test and KS normality test. Once the distribution in the different analyzes had been studied, the most appropriate test was applied being the Mann-Whitney statistical test, the Student's t-test and the ANOVA test. Differences were considered significant when $P < 0.05$.

The Results are described in Examples 2-4 below.

**EXAMPLE 2: Characterization of the populations of bone marrow CMHs from patients with bone marrow DBA**

Because it was still uncertain to what extent the number of HSCs in DBA may limit the collection of these cells, as already observed in a number of Fanconi anemia (FA) patients, we have first characterized bone marrow (BM) samples from DBA patients comparing them with healthy donors and with FA patients.

2.1 Determination of the populations of bone marrow HSCs from patients with DBA

The results obtained in this study showed that the levels of CD34$^+$ cells, as well as the more primitive population of CD34$^+$/CD38$^-$ progenitors, were not decreased in bone marrow samples from patients with DBA, when we compared with samples from healthy donors. These results (See **Figure 2**) show that, contrary to what occurs in other diseases, such as in FA, where patients present a marked drop in the number of CD34$^+$ cells, obtaining these cells in patients with DBA does not constitute a limitation for the collection of clinically relevant numbers of HSCs in DBA patients to be used in a gene therapy trial.

2.2 Analisis of multistem CD34$^+$ hematopoietic progenitors in the bone marrow of DBA patients

Although the MHC population did not appear to be affected, several studies have described a reduced number of erythroid progenitors in patients with DBA, and even in some cases neutropenia and thrombocytopenia during the course of the disease (Giri et al. 2000; Santucci et al. 1999; Tsai, Arkin, and Lipton 1989) To further study the hematopoietic progenitors in patients with DBA, the populations of intermediate hematopoietic precursors (Doulatov et al. 2012) were analyzed.Our results showed that there were no significant reductions in any of these populations (See **Figure 3: a)** HSC (hematopoietic stem cell: Lin-CD34+CD38-CD90+CD45RA-), **b)** MPP (multipotent progenitors: CD34$^+$CD38-Thy-1$^-$CD45RA$^-$Flt3$^+$CD7$^-$CD10$^-$), **c)** MLP (multi-lymphoid progenitors: CD34$^+$CD38$^-$Thy1$^{low}$CD45RA$^-$Flt3$^+$CD7$^{-/+}$CD10$^-$), **d)** CMP (common myeloid progenitors: CD34$^+$CD38$^+$Thy-1$^-$CD45RA$^-$Flt3$^+$CD7$^-$CD10$^-$), **e)** MEP (erythroid and megakaryocytic progenitors: CD34$^+$CD38$^+$Thy-1$^-$CD45RA$^-$Flt3$^-$CD7$^-$CD10$^-$) and f) GMP (granulocytic-monocytic progenitors: CD34$^+$CD38$^+$Thy-1$^-$CD45RA$^+$Flt3$^+$CD7$^-$CD10$^-$)).

2.3 Analysis of committed hematopoietic progenitors in the bone marrow of DBA patients

Once it was confirmed that the number of CD34$^+$ cells was not significantly affected in the bone marrow of patients with DBA, the next point to assess was its functionality by *in vitro* clonogenic assays. Various studies have observed that the ability of CD34$^+$ cells from patients with DBA to generate erythroid progenitors is decreased (Hamaguchi et al. 2003; Iskander et al. 2015; Ruggero and Shimamura 2014). Our results were consistent with this observation (**Figure 4**), since there is a very significant decrease in the erythroid progenitor count (BFU-Es, burst-forming unit-erythroid progenitors) (**Figure 4b**). Regarding the progenitors of the myeloid lineage (CFU-GMs, granulocyte-macrophage progenitor colony forming units) (**Figure 4a**), the results showed a decrease compared to healthy donors, although much less marked than that observed in BFU-Es.

2.4 Analysis of the engraftment and differentiation potential of HSCs from patients with DBA

It is important to consider that a successful gene therapy protocol to treat DBA patients requires the engraftment of corrected cells. Therefore, we have first evaluated the populating potential of these cells in immunodeficient NSG mice, as detailed in the Material and Methods session. Our results showed that, although there were no significant differences during the first 60 days after transplantation between cells derived from bone marrow from patients with DBA and from healthy donors, when we reached the end of the experiment at day 90, a decrease was observed significant at the level of engraftment of the cells of DBA patients (**Figure 5a**), which could indicate a lower long-term repopulation capacity of the MHCs of these patients. Although the repopulation potential showed a drop at 90 days post-transplantation, the same did not happen with the differentiation potential. Figure 5b shows the proportion of the different lineages generated within human CD45$^+$ cells, and it is shown that there are no significant differences between cells from patients with DBA and from healthy donors. These results indicated that the differentiation potential of the CD34$^+$ population of transplanted DBA patients remained similar to that observed in healthy donors.

In conclusion, the results obtained in this section allow us to predict that the collection of HSCs should not constitute a significant limitation in patients with DBA; In principle, in the samples studied there is no reduction in the content of CD34[+] CD38[-] cells or in the content of immature progenitors committed to certain lineages. However, despite the absence of a decrease in the number of hematopoietic progenitors, the lower clonogenic potential, mainly at the level of CFU-GM and BFU-Es, allows us to glimpse a lower erythropoietic potential of this population. Along with this, it should be noted that cells from patients with untransduced DBA maintain a high repopulation potential in immunodeficient mice, unlike cells from patients with FA, where a highly defective repopulation potential has been observed (Rio et al. 2019).

**EXAMPLE 3: Generation of a therapeutic clinically applicable lentiviral vector (LV): Efficacy studies in the K562 cell line and in RPS19-haploinsuficient CD34[+] cells from DBA patients**

Supported by the real feasibility of collecting functional HSC from patients with DBA, which could be corrected using a gene therapy protocol, we decided to develop two clinically applicable lentiviral vectors (LV) using a codon-optimized version of the *RPS19* gene, under the control of would be that of the phosphoglycerate kinase promoter (PGK) or the elongation *factor alpha in its short version (EF1α(s)).*

3.1 Analysis of the functionality of the therapeutic vectors in the K562 cell line interfered for RPS19

First, the ability to express the transgene of interest by the therapeutic vectors was checked, which consisted of the optimized codon version of the *RPS19* gene (*CoRPS19;* optimized codon *RPS19*). To do this, K562 cells were initially transduced with two lentiviral vectors carrying two specific shRNA sequences to interfere with RPS19 expression (*LV-THM.shRPS19 and LV-MISSION® pLKO.1-pure TurboGFP ™ TurboGFP ™ shRPS19*), generating in this way a DBA model; subsequently these cells in which the expression of RPS19 was affected, were corrected with the therapeutic vectors.
Interference with the *LV-THM.shRPS19* vector showed that it was able to mute the mRNA levels of the RPS19 gene at levels of 50-60% as shown in **Figure 6a.** Together with this result, the expression of therapeutic vectors (transgene CoRPS19) was confirmed in the interfering cells, both in those control conditions without interfering but transduced with therapeutic vectors, and in the conditions interfered and subsequently corrected with the therapeutic vectors (**Figure 6a**).
This same study was conducted for the second vector LV-*MISSION® pLKO.1-pure TurboGFP™ shRPS19,* which confirmed a higher interference capacity showing an mRNA silencing of 95% of the *RPS19* gene (**Figure 6b**). However, even with this ability to interfere with the gene of interest, the analysis of the optimized version *CoRPS19* showed the same results as in the previous experiment, observing only the expression of the *CoRPS19* sequence under the conditions transduced with therapeutic vectors (**Figure 6b**).

*3.1.1 Analysis of the ribosomal biogeneis process in the K562 line interfered with in the RPS19 gene and corrected with the therapeutic vectors*

Since the cells of patients with Blackfan-Diamond anemia are characterized by a defect in the ribosomal biogenesis process, in the first place, the degree of involvement of this process in K562 cells in which the expression of *RPS19* had been interfered, was studied. For this, we proceeded to study K562 cells transduced with the two interference vectors generated. In parallel, the possible reversal of the effect was analysed when correcting the K562 interfered cells with the therapeutic vectors *PGK.CoRPS19.Wpre** and *EF1α(s).CoRPS19.Wpre*.*
Analysis of these samples revealed that there was an accumulation of 21S pre-rRNA in the interfered-with cells. The interference vector LV-THM.shRPS19 generates an accumulation of 21S pre-rRNA significantly greater than that observed in control K562 cells without transducing or interfered with a nonspecific hairpin (scramble). When the K562 cells, interfered or not, were transduced with either of the two therapeutic vectors, the levels of pre-21S rRNA showed levels similar to those observed in the control conditions (**Figure 7a**).
When cells were interfered with the *LV-MISSION® pLKO.1-pure TurboGFP ™ shRPS19* interference vector, the accumulation of 21S / 21C pre-rRNA in the interfered cells was more than two times higher than that observed when the cells interfered with the vector *LV-THM.shRPS19* It is noteworthy that even under these conditions, when the cells were transduced with the therapeutic vectors *PGK.CoRPS19.Wpre** and *EF1α(s).CoRPS19.Wpre*,* the 21S pre-rRNA levels showed values similar to the conditions control (**Figure 7b**), the vector *PGKCoRPS19.Wpre** always showing a more significant recovery than the vector *EF1a(s).CoRPS19. Wpre*.*

3.2 Efficacy studies of the lentiviral gene therapy in cells of patients with DBA

Once the functionality of PGK therapeutic vectors had been determined *PGK.CoRPS19.Wpre** and *EF1α(s).*

*CoRPS19.Wpre** in cell lines, the next step was to continue these studies in primary cells of DBA patients. These studies were conducted on bone marrow cells in different DBA patients. The objective of these experiments was to: 1) evaluate the possibility of transducing the hematopoietic progenitors of patients with DBA, 2) confirm whether the therapeutic vectors had toxicity in the hematopoietic progenitors, 3) evaluate the degree of reversal of the phenotype of these cells, and 4) assess whether the corrected cells were able to graft into an in vivo xenogeneic transplant model.

*3.2.1 Analysis of the clonogenic potential (CFCs) of the hematopoietic progenitors of patients with DBA transduced with therapeutic vectors*

In order to assess the effect of transduction on hematopoietic progenitors of DBA patients, BM CD34$^+$ cells from DBA patients were transduced with the therapeutic vectors *PGK.CoRPS19.Wpre** and *EF1α(s). CoRPS19.Wpre** and with a control vector *PGK.EGFP.Wpre*.* Clonogenic assays were then conducted to check whether transduction with different therapeutic vectors modified the clonogenic potential of CD34$^+$ cells. The fact that the number of colonies generated by CD34$^+$ cells of DBA patients did not decrease when translating with the therapeutic vectors *PGK.CoRPS19.Wpre** and *EF1α(s).CoRPS19.Wpre** indicated the absence of toxicity mediated by these vectors (**Figure 8**).The determination of the total number of colonies formed revealed that transduction with the therapeutic vectors *PGK.CoRPS19.Wpre** and *EF1α(s).CoRPS19.Wpre** increased the number of colonies compared to cells transduced with the control vector *PGK.EGFP.Wpre*,* being significant the difference in the number of BFU-Es colonies in samples transduced with vector *EF1α(s). CoRPS19.Wpre** (**Figure 8b**).

*3.2.2 Determination of the number of copies of the provirus (VCN) and the percentage of transduction in hematopoietic progenitors of DBA patients after transduction with the therapeutic vectors*

Our laboratory's experience in the transduction of primary CD34$^+$ and in particular of cells of patients with bone marrow failures, enabled the use of an optimized protocol of transduction of these cells from patients with DBA. To determine the efficiency of this protocol in hematopoietic progenitors of DBA patients, the number of copies of the integrated vector (VCN) in the transduced cells was determined by detecting the Q-PCR provirus packaging signal ($\Psi$). These analyses were performed, both in the set of CD34$^+$ transduced$^+$ expanded cells in liquid culture for 14 days, as well as in the individual colonies obtained in clonogenic assays. From these colonies the percentage of transduction was determined, quantifying the number of colonies positive to provirus versus totals.

The determination of transduction efficiency revealed that 77.93% of CFCs had been transduced with the *PGK vector. CoRPS19.Wpre** and 57.79% with *vector EF1α(s). CoRPS 19. Wpre*,* 48.85% for the PGK vector *control. EGFP.Wpre** (**Figure 9a**). Detection of the number of copies of the lentiviral vectors, both in liquid culture (**Figure 9b**) and in the collected colonies (**Figure 9c**) showed the presence of approximately two copies of the provirus per cell under all conditions. Individual CD34$^+$ cell transduction results of each patient are listed in **Table 5**.

**Table 5.** Results obtained after transduction of CD34$^+$ cells from patients with DBA with the therapeutic vectors *PGK.CoRPS19.Wpre** and *EF1α(s).CoRPS19.Wpre** and the control vector *PGK.EGFP.Wpre*.*

| Patient | Vector | CD34$^+$ Cells (%) | Transduction (%) | MOI | VCN/Cell in CFCs | VCN/Cell in Liquid Culture |
|---|---|---|---|---|---|---|
| DBA-31 | *PGK.EGFP.Wpre** | ND | 12.5 | 300 | 0.10 | 1.80 |
| | *PGK.CoRPS19.Wpre** | | 69.2 | | 2.20 | 2.20 |
| 25 | *PGK.EGFP.Wpre** | 92.6 | DBA-33.3 | 200 | 0.10 | 2.10 |
| | *PGK.CoRPS19.Wpre** | | 66.7 | | 0.50 | 1.90 |
| | *EF1α (s).CoRPS19.Wpre** | | 36.4 | | 0.50 | 1.50 |
| DBA-36 | *PGK.EGFP.Wpre** | 70 | 66.6 | 200 | 2.30 | 2.10 |
| | *PGK.CoRPS19.Wpre** | | 66.6 | | 2.40 | 2.00 |
| | *EF1α(s) CoRPS19.Wpre** | | 41.2 | | 2.60 | 1.60 |

(continued)

| Patient | Vector | CD34+ Cells (%) | Transduction (%) | MOI | VCN/Cell in CFCs | VCN/Cell in Liquid Culture |
|---|---|---|---|---|---|---|
| DBA-5 | PGK.CoRPS19.Wpre* | ND | 100.0 | 100 | 2.43 | 4.23 |
| | EF1α(s) CoRPS19.Wpre* | | 83.3 | | 1.86 | 3.30 |
| DBA-13 | PGK.EGFP.Wpre* | ND | 16.7 | 100 | 0.40 | 1.90 |
| | PGK.CoRPS19.Wpre* | | ND | | 5.80 | 1.40 |

*3.2.3 Analysis of the effect of transduction on the proliferation of hematopoietic progenitors in patients with DBA*

These experiments analyzed whether complementation with therapeutic vectors generated an *in vitro* proliferative advantage of corrected haematopoietic progenitors over uncorrected ones. Once the CD34+ cells have been with both PGK therapeutic vectors *PGK.CoRPS19.Wpre** and *EF1α(s).CoRPS19.Wpre** and with the vector control *PGK.EGFP.Wpre*,* were kept in liquid culture for 14 days. The growth curve analysis revealed no significant differences in cell growth of hematopoietic progenitors transduced with PGK therapeutic vectors *PGK.CoRPS19.Wpre** and *EF1α(s).CoRPS19.Wpre** and with the vector control *PGK.EGFP.Wpre** over the time **(Figure 10)**.

*3.2.4 Analysis of erythroid differentiation in hematopoietic progenitors of DBA patients corrected with therapeutic vectors*

The process of erythroid differentiation of cells from patients with DBA shows a blockage in the maturation phases of erythroid progenitors generating fewer erythrocytes. Therefore, it was analyzed whether therapeutic vectors were able to reverse the blockage of erythroid differentiation compared to uncorrected cells.

To determine this process, marrow-derived CD34+ cells from DBA patients were transduced with the therapeutic vectors *PGK.CoRPS19.Wpre** and *EF1α(s).CoRPS19.Wpre** and with the control vector *PGK.EGFP.Wpre*.* After transduction, the cells were grown in a medium that promotes erythroid differentiation and the different stages of the erythroid differentiation process were analyzed using flow cytometry using the markers previously described CD45, CD36, CD71 (transferrin receptor) and CD235a (glycophorine A; GPA) characteristic of the erythroid differentiation process.

The cytometry analysis was carried out using the following strategy: in the first selection C36+/CD45- which are those cells that are differentiating towards the erythroid lineage. Within the cell group C36+/CD45- select two windows, in which the first one collects the events CD71+/CD235a+ corresponding to the erythroid progenitors, and the second window collects the events CD71-/CD235a+ corresponding to reticulocytes and mature erythrocytes (**Figure 11).**

The determination of the erythroid differentiation process in hematopoietic progenitors of DBA patients revealed two observations. Samples transduced with therapeutic vectors *PGK.CoRPS19.Wpre** and *EF1α(s).CoRPS19.Wpre** have a higher percentage of mature erythrocytes (window CD71-/CD235a+) compared to the conditions transduced with the control vector *PGK.EGFP.Wpre** **(Figure 11).** This fact suggests that the blockage in the maturation of erythroid progenitors between phases CD71+/CD235+ and CD71-/CD235+ a is reversed by transduction with therapeutic vectors.

3.3. Analysis of the hematological phenotype of the hematopoietic progenitors of patients with DBA transduced with the therapeutic vectors and transplanted into immunodeficient NSG mice.

In order to study whether the process of transduction with therapeutic vectors *PGK.CoRPS19.Wpre** or *EF1α(s).CoRPS19.Wpre** affected the repopulation capacity of CMHs in patients with haploinsufficiency for *RPS19,* transduced cell transplants were performed in NSG immunodeficient mice.

*3.3.1 Analysis of the potential for reconstitution of CD34+ bone marrow cells of patients with DBA transduced with therapeutic vectors*

For these experiments, CD34+ Cells from DBA patients were transduced with the therapeutic vectors *PGK.CoRPS19.Wpre** and *EF1α(s).CoRPS19.Wpre** and control vector *PGK. EGFP.Wpre*,* and transplanted into NSG immunodeficient mice. At 30 and 60, bone marrow samples were obtained by femur puncture and on day 90 the mice were slaughtered.

The study of the percentage+ of human CD45+ cells in the bone marrow of transplanted mice revealed the repopulation capacity of these corrected cells, with no significant differences observed between samples supplemented by therapeutic vectors and those transduced with *PGK.EGFP.Wpre** to day 90 post transplantation (see **Figure 12**). The ability of hematopoietic progenitors corrected with therapeutic vectors was also investigated *PGK.CoRPS19.Wpre** and *EF1α(s).CoRPS19.Wpre** and with the vector control *PGK.EGFP.Wpre** to generate different lineages. **Figure 12** shows that the pluripotent potential of CD34$^+$ as well as the ability to generate myeloid cells (CD33$^+$) or b lymphoid (CD19$^+$), was similar in all groups of transduced cells.

*3.3.2 Vector copy number analysis in transduced and grafted DBA patient cells in NSG mice*

Confirmation that the graft obtained in the previous section corresponded to our therapeutically transduced cells was obtained by the following process. Once the mice were sacrificed at day 90 posttransplant, the human bone marrow CD45$^+$ population of the transplanted mice were selected by sorting (selection of the population of interest by flow cytometry). Once the human cells that had been grafted were obtained, VCN was determined by Q-PCR (**Figure 13**). The determination of the VCN in cells of patients transduced with the therapeutic vectors and grafted in NSG mice revealed that, on day 90 posttransplant, integration of the therapeutic vectors is observed at levels of approximately 2 copies per cell on average for the vector *PGK.CoRPS19.Wpre** and one copy per cell for the vector *EF1α(s).CoRPS19.Wpre**.

**EXAMPLE 4: Safety studies associated with HCM gene therapy in patients with DBA**

4.1 *In vitro* safety studies

In order to carry out studies that would allow us to evaluate the safety of the transduction of MHCs from patients with DBA with the lentiviral vectors *PGK.CoRPS19.Wpre** and *EF1α(s).CoRPS19.Wpre**, experiments were carried out first. of transduction of CD34$^+$ cells from umbilical cord blood from healthy donors.

*4.1.1 Clonogenic analysis of hematopoietic progenitors of healthy and transduced donors with therapeutic vectors*

In order to analyze whether the transduction with both therapeutic vectors showed toxic effects on the hematopoietic progenitors of healthy donors, CD34$^+$ cells were transduced with the two therapeutic vectors *PGK.CoRPS19.Wpre** or *EF1α(s).CoRPS19.Wpre** and the control vector *PGK.EGFP.Wpre**.
Determination of the number of colonies generated from the transduced cells showed that the number of CFU-GMs (**Figure 14a**) was similar with all vectors. However, a significant decrease of 20.32% in the number of BFU-Es was observed in cells transduced with the vector *EF1α(s).CoRPS19.Wpre**, compared to cells transduced with the vector *PGK.EGFP.Wpre** (**Figure 14b**).

*4.1.2. Analysis of the number of copies of the proviruses and the levels of expression of CoRPS19 in colonies and post-transduction liquid culture of CD34$^+$ cells from healthy donors.*

In order to verify that the transduction levels were comparable between the different conditions, the vector per cell copy number was analyzed in colonies and in liquid culture. The determination of the VCN per cell made from the individual colonies collected from the previous experiments showed that, taking as a positive colony the one with a value greater than 0.3 copies percel, the transduction efficiency of the colonies with the vector *PGK.EGFP.Wpre** was 85.9%, and the efficacy for the vector *PGK.CoRPS19.Wpre** was 77.03%. Regarding the vector *EF1α(s).CoRPS19.Wpre**, its transduction efficiency was 67.83%, this being significantly lower than those shown by the control vector *PGK.EGFP.Wpre** and the therapeutic vector *PGK.CoRPS19.Wpre** (**Figure 15a**).
The mean number of copies observed in the analysis of the liquid culture was $3.40 \pm 0.91$ copies per cell for the condition *PGK.EGFP.Wpre**, $5.80 \pm 1.80$ copies for the condition *PGK.CoRPS19.Wpre** and $2.00 \pm 0.49$ for the condition *EFα(s).CoRPS19.Wpre**, this being significantly lower than those shown in the two previous vectors (**Figure 15b**).
In the following three experiments, the expression of the CoRPS19 transgene was determined based on transduction with the therapeutic vectors *PGK.CoRPS19.Wpre** and *EF1α(s).CoRPS19.Wpre**, taking as reference the control *PGK.EGFP.Wpre**. As can be seen in **Figure 15c,** expression of the mRNA corresponding to the optimized version of the RPS19 gene is only observed in cells transduced with therapeutic vectors. In addition, the expression of the CoRPS19 transgene of each vector was determined as a function of the number of integrated copies. **Figure 15c** shows that the CoRPS19 expression levels induced by the therapeutic vectors were similar.

*4.1.3. Analysis of the cell growth of CD34 $^+$ cells from healthy donors transduced with the therapeutic vectors and the control vector*

In these experiments we investigated the growth of CD34$^+$ cells after transduction with therapeutic vectors. For this, the transduced CD34$^+$ cells were cultured and the number of cells was quantified in liquid culture once a week for three weeks.

As shown in **Figure 16,** the growth of the cells transduced with the therapeutic vectors *PGK.CoRPS19.Wpre** and *EF1α(s).CoRPS19.Wpre** and the control *PGK.EGFP.Wpre*,* showed no significant differences.

4.2 *In vivo* safety studies

*4.2.1. Analysis of the repopulation potential of CD34$^+$ cells from healthy donors transduced with therapeutic vectors*

To analyze the repopulation potential of transduced healthy donor MHCs, these cells were transplanted into immunodeficient NSG mice. At 30, 60, 90 and 120 days post-transplantation, the proportion of human CD45$^+$ cells that had grafted onto the primary recipients was determined. Likewise, bone marrow cells from these primary recipients were transplanted back into secondary recipients to study the long-term repopulation potential (30 and 120 days post secondary transplantation).

As shown in **Figure 17a,** no significant differences were observed in the graft level of the primary recipients between the different experimental groups. Secondary receptor analysis revealed a significantly lower level of engraftment at day 120 post secondary transplantation in cells transduced with the control vector *PGK.EGFP.Wpre*,* compared to cells transduced with the vector *PGK.CoRPS19.Wpre*.* Presumably, the significant difference observed is due to the homogeneity of the results in the group of cells transduced with the vector *PGK.CoRPS19.Wpre*,* compared to the high heterogeneity obtained with the vector *PGK.EGFP.Wpre*.*

Together with the previous analysis, the quantification of the different lineages generated by the grafted cells was carried out, not observing significant differences in the differentiation potential, neither in the primary nor in the secondary receptors (**Figure 17b**).

*4.2.2. Provirus copy number analysis in NSG mice transplanted with CD34$^+$ cells from healthy donors transduced with therapeutic vectors*

After the sacrifice of the NSG mice from the previous experiment, the bone marrow was obtained and the VCN per cell was determined to confirm that the grafted cells carried the proviral sequence of the therapeutic vectors *PGK.CoRPS19.Wpre** and *EF1α (s).CoRPS19. Wpre*.*

As can be seen in figure 18, the determination of the VCNVCN / cell showed significant differences between the number of copies of provirus detected in the cells transduced with the control vector *PGK.EGFP.Wpre** (2.6 $\pm$ 1.87 copies) and the transduced with the vector *PGK.CoRPS19.Wpre** (5.9 $\pm$ 1.8 copies), these differences not being observed with the vector *EF1α(s).CoRPS19.Wpre** (5.65 $\pm$ 3.67) (**Figure 18**).

*4.2.3. Analysis of the variation of body weight and hematologies in NSG mice transplanted with CD34$^+$ cells derived from the umbilical cord of healthy donors and transduced with therapeutic vectors.*

In order to assess the health status of the transplanted animals, the body weight and hematology of the transplanted NSG mice were evaluated every 30 days until the day of sacrifice in the primary recipients and at day 30 and 120 in the secondary recipients.

The determination of the body weight of the NSG mice transplanted with the transduced cells revealed that there were no significant differences between the different conditions, as shown in **Figure 19.**

The hematological parameters determined in the primary and secondary NSG receptors showed that there were no significant differences in the primary receptors in the erythrocytes, platelets, leukocytes or neutrophils (**Figure 20**). However, the analysis of the secondary receptors showed a slight increase that was significant in the number of erythrocytes and in the hemoglobin concentration between the control group *PGK.EGFP.Wpre** and the group corresponding to the vector *PGK.CoRPS19.Wpre** Despite this, none of the other parameters corresponding to the erythroid lineage (HCT, MCV) showed significant differences between groups.

4.3 Genotoxicity studies: analysis of the integration of the provirus in the genome of hematopoietic cells from healthy donors transduced with the vector *PGK.CoRPS19.Wpre*.*

Analysis of vector integration sites was carried out in CD CD34$^+$ cells from healthy donors transduced with the therapeutic vector *PGK.CoRPS19.Wpre** and transplanted into the NSG mice shown in the previous section. Genewerk conducted analysis of the most common integration sites to evaluate possible oncogenesis or clonal-dominant events. The study was conducted in two sample types: cells kept in pre-transplant liquid culture and in human CD45⁺ cells present in transplanted mice. For the analysis, reference was made to the 10 most common integration sites for each sample (list of 114 genes used to determine the top 10 of each).

The first of the integration site analyses study was performed with liquid cultured samples from pre-transplant safety experiments in NSG mice. In these liquid culture samples, the highest contribution of the integration sites corresponded to 4.37% as a top 1, showing no signs of clonal dominance. The second analysis was carried out from human+ cells present in transplanted primary and secondary recipients. For primary recipients, integration site analysis revealed that the highest contribution accounted for 5.009% of the total. For secondary recipients, the highest contribution represented 32.33%.

Analysis of common integration sites (CIS) revealed a diverse composition in terms of the 10 main cis regions, none of which were associated with adverse events such as clonal growth or oncogenic processes. Along with this, there was also no specific integration into nearby genes or involved in adverse effects described in other gene therapy trials (CCND2, LMO2, MDS1/EVI1 (MECOM), MN1).

In short, the samples transduced with *PGK.CoRPS19.Wpre**, both those kept in liquid culture and those transplanted in NSG mice, showed a polyclonal integration pattern, with a stronger clonal growth observed in two of the samples transplanted into secondary recipients of safety experiment 3, where the relative contribution of IS top 1 reached 32,336%.

**Claims**

1. A lentiviral vector, wherein the lentiviral vector comprises a polynucleotide, wherein the polynucleotide is **characterized in that** it comprises from 5' to 3' the following nucleotides:

    a) a 5' long terminal repeat (5' LTR) which in turn comprises a chimeric cytomegalovirus promoter,
    b) a primer binding site (PBS),
    c) a psi packaging signal,
    d) a Rev responsive element (RRE),
    e) a DNA flap central polypurine tract (cPPT),
    f) a human phosphoglycerate kinase (PGK) promoter,
    g) a nucleotide sequence encoding for the RPS19 protein,
    h) a mutated optimized woodchuck hepatitis virus posttranscriptional regulatory element (Wpre), and
    i) a 3' long terminal repeat (3' LTR),

    wherein 5' and 3' LTR regions have been rendered substantially transcriptionally inactive by virtue of total or partial deletion in the U3 region of the LTR, and wherein f), and h) are operably linked to and regulate the expression of g).

2. The lentiviral vector of claim 1, wherein the human PGK promoter has at least 95%, preferably 98%, sequence identity over the full length of SEQ ID NO: 11.

3. The lentiviral vector according to any of claims 1 and 2, wherein the nucleotide sequence encoding for the RPS19 protein is a codon-optimized nucleotide sequence with at least 95%, preferably 98%, sequence identity over the full length with SEQ ID NO: 12.

4. The lentiviral vector according to any of claims 1 to 3, wherein the mutated optimized woodchuck hepatitis virus posttranscriptional regulatory element (Wpre) has at least 95%, preferably 98%, sequence identity over the full length with SEQ ID NO: 13.

5. The lentiviral vector according to any of claims 1 to 4, wherein the polynucleotide comprised in the lentiviral vector further comprises polyadenylation (polyA) signal sequence located after the 3' LTR.

6. The lentiviral vector according to any of claims 1 to 5, wherein the full-length polynucleotide of said lentiviral vector has at least 95%, preferably 98% sequence identity with SEQ ID NO: 17.

7. The lentiviral vector according to claim 6, wherein the sequence identity with SEQ ID NO: 17 is 100%.

8. A population of cells transduced with the lentiviral vector as defined in any of claims 1 to 7.

9. The population of cells according to claim 8, wherein the cells are enriched in CD34$^+$ hematopoietic stem and progenitor cells.

10. The population of cells according to any of claims 8 and 9, wherein the cells are autologous to the subject.

11. A pharmaceutical composition comprising the lentiviral vector as defined in any of claims 1 to 7, or the population of cells as defined in any of claims 8 to 10, and a pharmaceutically acceptable carrier or diluent.

12. The lentiviral vector according to any of claims 1 to 7, the population of cells according to any of claims 8 to 10, or the pharmaceutical composition of claim 11, for use as a medicament.

13. The lentiviral vector according to any of claims 1 to 7, the population of cells according to any of claims 8 to 10, or the pharmaceutical composition of claim 11, for use in the treatment of Diamond-Blackfan anemia in a subject in need thereof.

14. The lentiviral vector according to any of claims 6 and 7, for use in the treatment of Diamond-Blackfan anemia in a subject in need thereof.

15. The lentiviral vector for use according to any of claims 13 and 14, wherein the subject in need thereof is a human being.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

a)

Primary receptors

Secondary receptors

% of hCD45 cells in bone marrow

D30  D60  D90  D120

% of hCD45 cells in bone marrow

D30  D120

b)

Lineage distribution in primary receptors

Lineage distribution in secondary receptors

% of Lineages within hCD45 analysis

CD34  CD33  CD19

% of Lineages within hCD45 analysis

CD34  CD33  CD19

○ PGK-EGFP
● PGK-CoRPS19
▲ EF-CoRPS19

EP 4 339 291 A1

65

Figure 18

Figure 19

Figure 20

## INTERNATIONAL SEARCH REPORT

| International application No |
| --- |
| **PCT/ES2021/070343** |

**A. CLASSIFICATION OF SUBJECT MATTER**
**INV. C12N15/867    C12N5/10     A61K48/00    A61P7/00**
**ADD.**

According to International Patent Classification (IPC) or to both national classification and IPC

**B. FIELDS SEARCHED**

Minimum documentation searched (classification system followed by classification symbols)
**C12N   A61K**

Documentation searched other than minimum documentation to the extent that such documents are included in the fields searched

Electronic data base consulted during the international search (name of data base and, where practicable, search terms used)

**EPO-Internal, BIOSIS, WPI Data**

**C. DOCUMENTS CONSIDERED TO BE RELEVANT**

| Category* | Citation of document, with indication, where appropriate, of the relevant passages | Relevant to claim No. |
| --- | --- | --- |
| Y | **GIMENEZ YARI ET AL:  "Preclinical Gene Therapy in Hematopoietic Stem and Progenitor Cells from RPS19-Deficient Diamond-Blackfan Anemia Patients",** **MOLECULAR THERAPY,** **vol. 29, no. 4, Suppl. 1,** **27 April 2021 (2021-04-27), pages 335-336,** **XP055879948,** **abstract** _____ -/-- | **1,2,4-6, 8-15** |

[X] Further documents are listed in the continuation of Box C.　　　　[X] See patent family annex.

* Special categories of cited documents :

"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance;; the claimed invention cannot be considered novel or cannot be considered to involve an inventive step when the document is taken alone
"Y" document of particular relevance;; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **19 January 2022** | **04/02/2022** |

| Name and mailing address of the ISA/ | Authorized officer |
| --- | --- |
| European Patent Office, P.B. 5818 Patentlaan 2 NL - 2280 HV Rijswijk Tel. (+31-70) 340-2040, Fax: (+31-70) 340-3016 | **Brenz Verca, Stefano** |

Form PCT/ISA/210 (second sheet) (April 2005)

**page 1 of 3**

**INTERNATIONAL SEARCH REPORT**

International application No.

PCT/ES2021/070343

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.    [X]    forming part of the international application as filed:

          [X]    in the form of an Annex C/ST.25 text file.

          [X]    on paper or in the form of an image file.

    b.    [ ]    furnished together with the international application under PCT Rule 13ter.1(a) for the purposes of international search only in the form of an Annex C/ST.25 text file.

    c.    [ ]    furnished subsequent to the international filing date for the purposes of international search only:

          [ ]    in the form of an Annex C/ST.25 text file (Rule 13ter.1(a)).

          [ ]    on paper or in the form of an image file (Rule 13ter.1(b) and Administrative Instructions, Section 713).

2.    [ ]    In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.    Additional comments:

Form PCT/ISA/210 (continuation of first sheet (1)) (January 2015)

## INTERNATIONAL SEARCH REPORT

| | International application No |
|---|---|
| | **PCT/ES2021/070343** |

**C(Continuation).** DOCUMENTS CONSIDERED TO BE RELEVANT

| Category* | Citation of document, with indication, where appropriate, of the relevant passages | Relevant to claim No. |
|---|---|---|
| Y | GIMENEZ YARI ET AL: "Lentiviral-Mediated Preclinical Studies for the Gene Therapy of RPS19-Deficient Diamond-Blackfan Anemia Patients", MOLECULAR THERAPY, vol. 28, no. 4, Suppl.1, 28 April 2020 (2020-04-28), page 352, XP055879939, abstract ----- | 1,2,4-6, 8-15 |
| Y | GIMENEZ MARTINEZ Y ET AL: "Lentiviral-mediated Phenotypic Correction of CD34+Cells from RPS-19-deficient Diamond-Blackfan Anemia Patients", HUMAN GENE THERAPY, vol. 30, no. 11, 1 November 2019 (2019-11-01), page A10, XP055879933, abstract ----- | 1,2,4-6, 8-15 |
| Y | WO 2019/079338 A1 (CENTRO DE INVESTIG ENERGETICAS MEDIOAMBIENTALES Y TECNOLOGICAS O A M P) 25 April 2019 (2019-04-25) figures 1,2A,21-23; sequence 25 ----- | 1,2,4-6, 8-15 |
| A | LIU YANG ET AL: "Successful gene therapy of Diamond-Blackfan anemia in a mouse model and human CD34+ cord blood hematopoietic stem cells using a clinically applicable lentiviral vector", HAEMATOLOGICA, 14 January 2021 (2021-01-14), XP055880146, IT ISSN: 0390-6078, DOI: 10.3324/haematol.2020.269142 Retrieved from the Internet: URL:https://haematologica.org/article/down load/haematol.2020.269142/72783> the whole document ----- -/-- | 1-15 |

Form PCT/ISA/210 (continuation of second sheet) (April 2005)

**page 2 of 3**

## INTERNATIONAL SEARCH REPORT

| International application No |
|---|
| **PCT/ES2021/070343** |

| C(Continuation). | DOCUMENTS CONSIDERED TO BE RELEVANT | |
|---|---|---|
| Category* | Citation of document, with indication, where appropriate, of the relevant passages | Relevant to claim No. |
| A | DEBNATH SHUBHRANSHU ET AL: "Lentiviral Vectors with Cellular Promoters Correct Anemia and Lethal Bone Marrow Failure in a Mouse Model for Diamond-Blackfan Anemia", MOLECULAR THERAPY, vol. 25, no. 8, 1 August 2017 (2017-08-01) , pages 1805-1814, XP055879911, US ISSN: 1525-0016, DOI: 10.1016/j.ymthe.2017.04.002 Retrieved from the Internet: URL:https://www.cell.com/molecular-therapy -family/molecular-therapy/pdfExtended/S152 5-0016(17)30161-2> the whole document ----- | 1-15 |
| A | JAAKO P. ET AL: "Gene therapy cures the anemia and lethal bone marrow failure in a mouse model of RPS19-deficient Diamond-Blackfan anemia", HAEMATOLOGICA, vol. 99, no. 12, 12 September 2014 (2014-09-12), pages 1792-1798, XP055880151, IT ISSN: 0390-6078, DOI: 10.3324/haematol.2014.111195 the whole document ----- | 1-15 |

Form PCT/ISA/210 (continuation of second sheet) (April 2005)

page 3 of 3

## INTERNATIONAL SEARCH REPORT
Information on patent family members

| International application No |
|---|
| **PCT/ES2021/070343** |

| Patent document cited in search report | Publication date | Patent family member(s) | Publication date |
|---|---|---|---|
| WO 2019079338 A1 | 25-04-2019 | AU 2018352583 A1 | 09-04-2020 |
| | | BR 112020007444 A2 | 20-10-2020 |
| | | CA 3076253 A1 | 25-04-2019 |
| | | CN 111163810 A | 15-05-2020 |
| | | EP 3697452 A1 | 26-08-2020 |
| | | JP 2021500920 A | 14-01-2021 |
| | | KR 20200116076 A | 08-10-2020 |
| | | SG 11202002263T A | 29-04-2020 |
| | | US 2021187126 A1 | 24-06-2021 |
| | | WO 2019079338 A1 | 25-04-2019 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 280790000183

**Non-patent literature cited in the description**

- **SHUBHRANSHU et al.** Lentiviral Vectors with Cellular Promoters Correct Anemia and Lethal Bone Marrow Failure in a Mouse Model for Diamond-Blackfan Anemia. *Molecular Therapy,* 2017, vol. 25 (8), ISSN 1525-0016, 1805-1814, https://doi.org/10.1016/j.ymthe.2017.04.002

- *Jiménez Diaz Foundation,* 08 May 2018